# EUROPEAN PATENT APPLICATION

(11) **EP 2 324 837 A1**
(43) Date of publication of application: **25.05.2011**
(21) Application number: 11000435.5
(22) Date of filing: 21.12.2007
(51) Int. Cl.: A61K 31/70, A61P 35/00, A61P 3/10

(54) **Synthetic lipophilic inositol glycans for treatment of glucose-metabolism disorders**

(30) Priority: 21.12.2006 US 876277 P; 22.12.2006 US 876779 P
(62) Divisional of application: 07865960.4
(71) Applicant: Trustees Of Tufts College, Medford, MA 02155 (US); Tohoku University, Aoba-ku, Sendai-shi Miyagi (JP)
(72) Inventor: D'Alarcao, Marc, San Jose, CA 95123 (US); Azev, Viatcheslav, Khimki, 141400 Moscow Region (RU); Suzuki, Susumu, 981-0933 Aoba-ku (JP)
(74) Representative: Wibbelmann, Jobst

(57) **Abstract**

The present intervention related to materials and methods for the synthesis of lipophilic inositol glycan compounds, and salts, solvates and physiological functional derivatives thereof, and their use for the treatment of cancer and glucose-metabolism disorders, such as, for example, diabetes, obesity and metabolic syndrome.

## Description

This is a divisional application on the basis of the parent application European Patent Application 07 865 960.4 (PCT/US2007/088562) the content of which is incorporated herein by reference.

### FIELD OF THE INVENTION

The present intervention related to materials and methods for the synthesis of lipophilic inositol glycan compounds and their use for the treatment of cancer and glucose-metabolism disorders, such as, for example, diabetes, obesity and metabolic syndrome.

### BACKGROUND OF THE INVENTION

Cancer is the second leading cause of death in the United States. In the year 2000, malignant tumors were responsible for 12 per cent of the nearly 56 million deaths worldwide from all causes. Global cancer rates could increase by 50% to 15 million by 2020. Although advances have been made in detection and therapy of cancer, no vaccine or other universally successful method for prevention or treatment is currently available.

Breast cancer is the most common cancer for women, followed by lung cancer and colorectal cancer. Management of the disease currently relies on a combination of early diagnosis and aggressive treatment, which may include one or more of a variety of treatments such as surgery, radiotherapy, chemotherapy and hormone therapy.

Prostate cancer is the most common form of cancer among man, with an estimated incidence of 30% in men over the age of 50. Human prostate cancer has the propensity to metastasize to bone. Treatment is commonly based on surgery and/or radiation therapy, but these methods are ineffective in a significant percentage of cases. Accordingly, there remains a great need in the art for new anti-cancer compounds and therapies for a broad spectrum of human cancers.

The prevalence of diabetes for all age-groups worldwide was estimated to be 2.8% in 2000 and is expected to reach 4.4% in 2030. The total number of people with diabetes is projected to rise from 171 million in 2000 to 366 million in 2030. This increase in the prevalence of type 2 diabetes is closely linked to the upsurge in obesity, since about 90% of type 2 diabetes can be attributed to excess weight. Furthermore, approximately 197 million people worldwide have impaired glucose tolerance, most commonly because of obesity and the associated metabolic syndrome. This number is expected to increase to approximately 420 million by 2025.

Metabolic syndrome is central obesity with a collection of heart disease risk factors that increase the chance of developing heart disease, stroke, and arteriosclerosis obliterans. According to national health surveys, more than one in five Americans has metabolic syndrome. Since physical inactivity and excess weight are the main underlying contributors to the development metabolic syndrome and diabetes, life style modification, including healthy diets, more exercise and losing body weight can help reduce or prevent the complications associated with metabolic syndrome, and control diabetes.

However, life style modification is difficult for many subjects affected with diabetes and metabolic syndrome resulting in limited compliance with traditional treatments. Current anti-diabetic medications, including oral hypoglycemic agents and insulin, do not completely control the disease nor completely prevent diabetic complications. Thus, new approaches are needed for the treatment of diabetes and metabolic syndrome.

### SUMMARY OF THE INVENTION

The present invention relates, in part, to a new class of compounds for the treatment of cancer, diabetes, obesity and metabolic syndrome. In particular, the present invention provides lipophilic inositol glycan compounds as anti-cancer agents, alone, or in combination with other anti-cancer agents or therapies. The present invention also provides lipophilic inositol glycan compounds as anti-diabetic agents, alone, or in combination with other anti-diabetic agents or therapies. The present invention also provides lipophilic inositol glycan compounds for treating metabolic syndrome, alone, or in combination with other agents or therapies for reducing obesity or treating metabolic syndrome.

The lipophilic inositol glycan (IG) compound can be selected from one or more compounds of formula I: A-O₁-B (I), wherein A is a hexosamine selected from the group consisting of glucosamine and galactosamine; O₁ is alpha 1, 6; alpha 1, 3; or alpha 1, 2; and B is selected from the group consisting of inositol, myo-inositol, chiro-inositol, scyllo-inositol, epi-inositol, cis-inositol, neo-inositol, muco-inositol, and allo-inositol. The B moiety is optionally mono- or di-substituted, independently of one another, by phosphate, thiophosphate, alkyl group (C₁₋₂₂) or acyl residues O=C-C₀₋₂₁). In some embodiments, lipophilic inositol glycan compounds of the present invention includes one or more compounds, or functional analogs or isomers thereof, depicted in formula II: 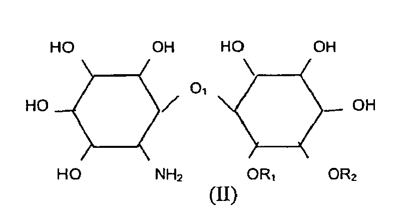 wherein O₁ depicts the alpha 1,6 or alpha 1,3 linkage; R₁ is phosphate, alkyl group (C₁₋₂₂) or acyl residues (O=C-C₀₋₂₁); R₂ is alkyl group (C₁₋₂₂) or acyl residues (O=C-C₀₋₂₁). Therapeutically and/or functionally active compounds of this invention include those of formulas I and II, and isomers, salts, esters and hydrates thereof.

In some embodiments of the invention, the lipophilic inositol glycan compounds of formula (I) and (II), and salts, solvates and physiological functional derivatives thereof, are co-administered with other agents or therapies. In another aspect, the lipophilic inositol glycan compounds of formula (I) and (II), and salts, solvates and physiological functional derivatives thereof, can be co-administered with on or more alkyl, acyl-phosphatidic acid (AAPA) compounds. Alkyl, acyl-phosphatidic acid (AAPA) is a putative product from glycosylphospatidylinositol (GPI) precursor of IG-1 by the hydrolysis with GPI-specific phospholipase D (GPI-PLD). In preferred embodiments, AAPA compounds are selected from the compounds of formula III:

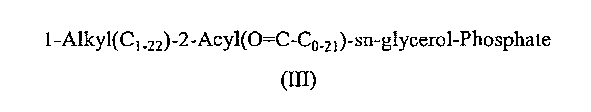

AAPA compounds also include and salts, solvates and physiological functional derivatives thereof. Non-limiting examples of alkyl, acyl-phosphatidic acid (AAPA) compounds include AAPA1 (1-0-alkyl (18:0)-2-0-acyl-(18:0)-sn-glycerol-phosphate), AAPA2 (1-0-alkyl (18:0)-2-0-acyl-(20:4)-sn-glycerol-phosphate) and AAPA3 (1-0-alkyl (18:0)-2-0-acyl-(22:4)-sn-glycerol-phosphate). In preferred embodiments, co-administration results in synergistic effects.

Aspects of the invention include administering to a patient, compositions providing one or more lipophilic inositol glycan compounds and/or one or more alkyl, acyl-phosphatidic acid (AAPA) compounds to a patient. Embodiments of the invention include methods of administering a combination of one or more lipophilic inositol glycan compounds and one or more AAPA compounds that provides a synergistic therapeutic effect with respect to treating cancer cells. "Synergistic" indicates that the therapeutic effect is greater than would have been expected based on adding the effects of each agent applied as a monotherapy.

Another aspect of the invention comprises methods of synthesis of compounds of formulas I, II, and III.

In another aspect, the invention provides a method of reducing cellular proliferation comprising the step of exposing said cells to an effective amount of a lipophilic inositol glycan compound, or functional analogs or isomers thereof. The cellular proliferation can be associated with cancer. In a preferred embodiment, the cells are located *in vivo* in a subject. In other aspects, the one or more compounds of the present invention can be used to slow or amerliorate abnormal cellular proliferation of cancer cells.

Another aspect of the invention comprises treating, slowing, and/or ameliorating abnormal cell growth by administering a therapeutically effective amount of a compound of the invention to a subject in need thereof. The abnormal cell growth can be a benign growth or a malignant growth. In particular, the abnormal cell growth can be a carcinoma, sarcoma, lymphoma, or leukemia. In one embodiment of this method, the abnormal cell growth is a cancer, including, but not limited to, lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, pituitary adenoma, or a combination of one or more of the foregoing cancers.

The methods of the invention also comprise treating a patient having cancer wherein the cancer is selected from the group consisting of small cell lung carcinoma, non-small cell lung carcinoma, esophageal cancer, kidney cancer, pancreatic cancer, melanoma, bladder cancer, breast cancer, colon cancer, liver cancer, lung cancer, sarcoma, stomach cancer, cholangiocarcinoma, mesothelioma, or prostate cancer. In another embodiment of said method, said abnormal cell growth is a benign proliferative disease, including, but not limited to, psoriasis, benign prostatic hypertrophy or restenosis. Exemplary cancers that are suitable for treatment with the methods and compositions of the present invention include prostate, breast, pancreatic, skin, colon, prostate cancer and neuroblastoma.

In another aspect, the present invention provides a method for reducing cellular proliferation comprising the step of exposing at least one lipophilic inositol glycan compound of formula (I) and (II), or salts, solvates and physiological functional derivatives thereof, to cells. In some embodiments, the cellular proliferation is associated with cancer. The cells can be located *in vivo* in a subject, preferably a mammal. The cancer can be, for example, pancreatic cancer, breast cancer, colon cancer, skin cancer or prostate cancer. In some embodiments, the cells are further exposed, simultaneously or sequentially, to a second compound having anti-proliferative properties. Non-limiting examples of compounds having anti-proliferative properties include, for example, anti-neoplastic agents, cancer chemotherapeutic agents and anti-proliferation agent, such as resveratrol or butyrate.

The present invention further provides a therapeutic composition comprising a lipophilic inositol glycan compound of formula (I) and (II), or salts, solvates and physiological functional derivatives thereof, configured for administration to a subject having or suspected of having cancer or diseases characterized by aberrant angiogenesis. In preferred embodiments, the lipophilic inositol glycan is formulated as a pharmaceutical composition. The composition can further comprise a second anti-proliferative compound.

The ability to induce apoptosis of cancer cells is a desireable feature of anticancer agents. The ability to induce apoptosis in cancer cells, without affecting healthy cells, as well as minimizing side-effects are major goals for the development of new anticancer agents. Experiments conducted during the course of development of the present invention indicate that lipophilic inositol glycan reduces cell proliferation, and induces rapid cell death in cancer cells, such as, for example, human pancreatic cancer cells, human hepatoma cells, human gastric cancer cells, human colon cancer cells, and human mammary cancer cells. In contrast, the lipophilic inositol glycans do not affect normal cells.

In another embodiment, the invention encompasses a method for treating or preventing cancer in a patient in need thereof, comprising administering to the patient a therapeutically or prophylactically effective amount of a compound according to formula I or II and a pharmaceutically acceptable excipient, carrier, or vehicle.

In another aspect, the invention encompasses a method for treating or preventing cancer in a patient in need thereof, comprising administering to the patient a therapeutically or prophylactically effective amount of a formula I or II compound, or salts, solvates and physiological functional derivatives thereof, and at least one additional therapeutic agent. In preferred embodiments, a therapeutically or prophylactically effective amount of a formula I or II compound, or salts, solvates and physiological functional derivatives thereof, is co-administered with one or more AAPA compounds of formula (III), or salts, solvates and physiological functional derivatives thereof. This co-administration can produce a synergistic effect. In other embodiments, at least one additional therapeutic agent can be combined with the co-administration.

Methods of the invention specifically include methods for treating cancer in a patient comprising providing to the patient one or more lipophilic inositol glycan compounds in combination with one or more AAPA compounds. The amount provided may be considered an "effective amount" in certain embodiments. It will be understood that "an effective amount" means that the patient is provided with both 1) lipophilic inositol glycan compounds and 2) AAPA compounds in an amount or amounts that leads to a therapeutic benefit. It will be understood that the patient is given an amount of lipophilic inositol glycan and an amount of AAPA, both in amounts that are believed to contribute to the therapeutic benefit.

In another aspect, the lipophilic inositol glycan compounds of formula (I) and (II), and salts, solvates and physiological functional derivatives thereof, can be used in the treatment of glucose-metabolism disorders. Glucose-metabolism disorders include, but are not limited to, obesity, diabetes, metabolic syndrome, insulin resistance, glucose intolerance, hyerinsulinemia, Syndrome X, hypercholesterolemia, hyperlipoproteinemia, hypertriglyceridemia, atherosclerosis, and diabetic renal disease. For example, the lipophilic inositol glycan compounds are useful in the treatment of type 2 diabetes, a discase which is associated with insulin resistance in peripheral tissues and impaired glucose-stimulated insulin secretion from pancreatic beta-cells and elevated hepatic gluconeogenesis. The lipophilic inositol glycan compounds have been shown in the Examples to increase lipogenesis in adipocytes, and reduce plasma glucose concentrations in mice with high fat diets, as well as STZ-diabetic mice. Furthermore, the lipophilic inositol glycan compounds can be used to control glucose homoeostasis. As shown in the Examples, treatment with lipophilic inositol glycan compounds for 28 days significantly reduced daily food intake, body weight, and fasting plasma concentrations of insulin, triglyceride, free fatty acids and leptin in ob/ob obese mice and the C57B/6 mice on high-fat diets.

### BRIEF DESCRIPTION OF THE FIGURES

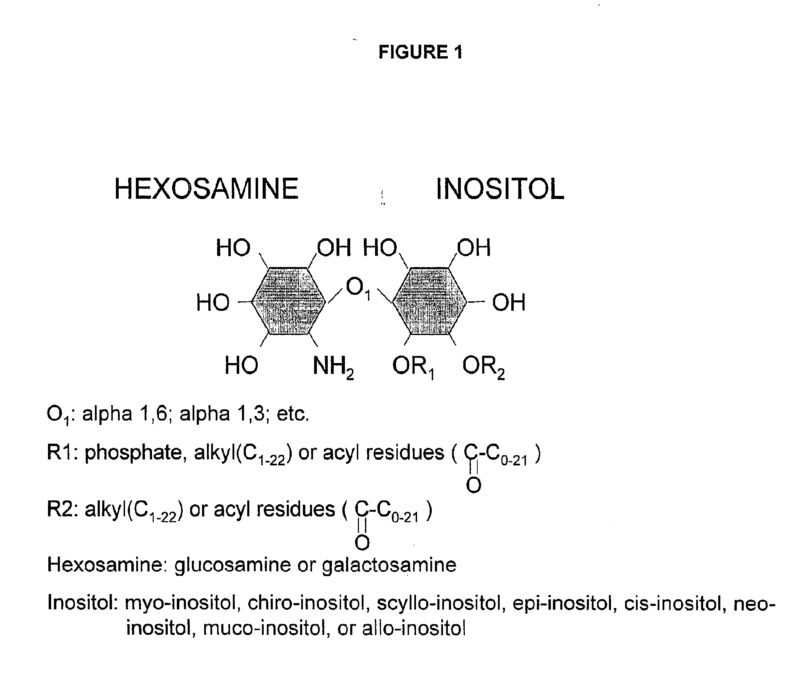

Fig. 1 depicts the general formula for the lipophilic inositol glycan compounds of the present invention;

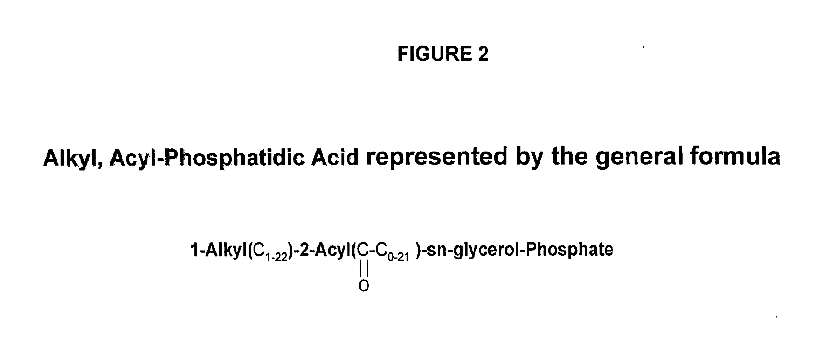

Fig.2 depicts the general formula for the alkyl, acyl-phosphatidic acid compounds of the present invention;
Fig.3A is a bar graph showing that IG-1 (2-amino-2-deoxy-α-D-glucopyranosyl-(1->6)-2-palmitoyl-D-*myo*-inositol) inhibits lactate production in Panc-1 cells following 1 hour incubation in serum free media;
Fig. 3B is a bar graph showing that IG-1 does not affect ATP contents in Panc-1 cells (human pancreatic cancer cells) following 1 hour incubation in serum free media;
Fig. 4A is a bar graph comparing the effects of IG-1 to resveratrol, and butyrate on Panc-1 cells (human pancreatic cancer cells), and demonstrating that IG-1 induced rapid cell death in Panc-1 cells after 1 hour;
Fig. 4B is a bar graph comparing the effects of IG-1 to resveratrol, and butyrate on Panc-1 cells (human pancreatic cancer cells), and demonstrating that IG-1 induced rapid cell death in Panc-1 cells after 24 hours;
Fig. 4C is a bar graph comparing the effects of IG-1 to resveratrol, and butyrate on Panc-1 cells (human pancreatic cancer cells), and demonstrating that IG-1 induced rapid cell death in Panc-1 cells after 3 days;
Fig. 5 is a bar graph comparing the effects of IG-2 (2-amino-2-deoxy-α-D-glucopyranosyl-(1->6)-2-palmitoyl-D-*myo*-inositol-1-phosphate) to resveratrol, and butyrate on PC3 cells (human prostate cancer cells), and demonstrating that IG-2 induced rapid cell death in PC3 cells after 24 hours;
Fig. 6 is a bar graph comparing the effects of IG-13 (2-amino-2-deoxy-α-D-glucopyranosyl-(1->6)-1-palmitoyl-D-*myo*-inositol), IG-14 (2-amino-2-deoxy-α-D-glucopyranosyl-(1->3)-2-palmitoyl-D-*myo*-inositol), IG-15 (2-amino-2-deoxy-α-D-glueopyranosyl-(1->3)-1-palmitoyl-D-*myo*-inositol), resveratrol, and butyrate on human pancreatic cancer (Panc-1) cell death, after treatment for 3 days;
Fig.7 shows a Kaplan-Meier survival curve of the ddY mice bearing Ehrlich ascites cancer cells after daily administration of IG1 and alkyl-acyl-phosphatidic acid (AAPA2) for 7 days;
Fig. 8 shows inhibitory effects of AAPA2 on the early induction of cell death of human umbilical vein endothelial cells by IG-1 in 1 hour;
Fig. 9A shows stimulation of glycogen synthesis in 3T3-L1 adipocytes after incubation with IG1;
Fig. 9B shows stimulation of lipogenesis in 3T3-L1 adipocytes after incubation with IG1;
Fig. 10 shows the percent stimulation of lipogenesis in rat isolated adipocytes versus concentration of IG-1 and IG-2;
Fig. 11 shows stimulation of lipogenesis in native rat adipocytes following incubation of 40µM with IG-1, IG-2, IG-13, IG-14 and IG15;
   Fig. 12A shows stimulation of glycogen synthesis in 3T3-L1 adipocytes after incubation with IG15;
Fig. 12B shows stimulation of lipogenesis in 3T3-L1 adipocytes after incubation with IG15;
Fig. 13 depicts time courses showing reduction of plasma glucose concentrations in the C57B/6 mice on high-fat diets after the intravenous administration of IG1;
Fig. 14 depicts time courses showing reduction of plasma glucose concentrations in the streptozotocin (STZ) diabetic mice after the intravenous administration of IG1;
Fig. 15 shows improved glucose tolerance in the C57B/6 mice on high-fat diets after daily intraperitoneal administration of IG1 for 7 days;
Fig.16 shows improved fasting plasma glucose concentrations in the STZ-diabetic mice after daily intraperitoneal administration of IG1 for 7 days.

### DETAILED DESCRIPTION

So that the invention may more readily be understood, certain terms are first defined:
As used herein, the term "subject suspected of having cancer" refers to a subject that presents one or more symptoms indicative of a cancer (e.g., a noticeable lump or mass) or is being screened for a cancer (e.g., during a routine physical). A subject suspected of having cancer may also have one or more risk factors. A subject suspected of having cancer has generally not been tested for cancer. However, a "subject suspected of having cancer" encompasses an individual who has received a preliminary diagnosis (e.g., a CT scan showing a mass) but for whom a confirmatory test (e.g., biopsy and/or histology) has not been done or for whom the stage of cancer is not known. The term further includes people who once had cancer (e.g., an individual in remission). A "subject suspected of having cancer" is sometimes diagnosed with cancer and is sometimes found to not have cancer.

As used herein, the term "subject diagnosed with a cancer" refers to a subject who has been tested and found to have cancerous cells. The cancer may be diagnosed using any suitable method, including but not limited to, biopsy, x-ray, blood test, and the diagnostic methods of the present invention. A "preliminary diagnosis" is one based only on visual (e.g., CT scan or the presence of a lump) and antigen tests.

The term "subject" as used herein is intended to include living organisms in which an immune response is elicited. Preferred subjects are mammals. Examples of subjects include but are not limited to, humans, monkeys, dogs, cats, mice, rats, cows, horses, pigs, goats and sheep.

The term "lipophilic inositol glycan" as used herein is intended to include compounds, and salts, solvates, functional analogs, and physiological functional derivatives thereof, of the formula I: A-O₁-B (I), wherein A is a hexosamine selected from the group consisting of glucosamine and galactosamine; O₁ is alpha 1,6; alpha 1,3; or alpha 1, 2; and B is selected from the group consisting of inositol, myo-inositol, chiro-inositol, scyllo-inositol, epi-inositol, cis-inositol, neo-inositol, muco-inositol, and allo-inositol, wherein the B moiety is optionally mono- or disubstituted, independently of one another, by phosphate, thiophosphate, alkyl group (C₁₋₂₂) or acyl residues ( O=C-C₀₋₂₁). In some embodiments, lipophilic inositol glycan compounds of the present invention includes one or more compounds, or functional analogs, salts, solvates and physiological functional derivatives, depicted in formula II: 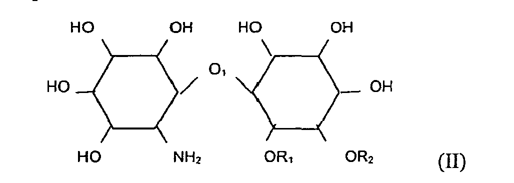 wherein O₁ depicts the alpha 1,6 or alpha 1,3 linkage; R₁ is phosphate, alkyl group (C₁₋₂₂) or acyl residues (O=C-C₀₋₂₁); R₂ is alkyl group (C₁₋₂₂) or acyl residues (O=C-C₀₋₂₁). Exemplary lipophilic inositol glycan compounds of the present invention are described below in Table 1.

**Table 1. Exemplary Lipophilic Inositol Glycan Compounds.**

| **Compound Name** | **IG number** | **O₁, R₁, and R₂** | **Compound number** |
|---|---|---|---|
| 2-amino-2-deoxy-α-D-glucopyranosyl-(1->6)-2-palmitoyl-D-*myo*-inositol | IG1 | O₁=alpha 1,6; R₁ = OH; R₂ = C16:0 | **13** |
| 2-amino-2-deoxy-α-D-glucopyranosyl-(1->6)-2-palmitoyl-D-*myo*-inositol-1-phosphate | IG2 | O₁=alpha 1,6; R₁ = phosphate; R₂ = C16:0 | **14** |
| 2-amino-2-deoxy-α-D-glucopyranosyl-(1->6)-1-palmitoyl-D-*myo*-inositol | IG13 | O₁=alpha 1,6; R₁= C16:0; R₂=OH | **91** |
| 2-amino-2-deoxy-α-D-glucopyranosyl-(1->3)-2-palmitoyl-D-*myo*-inositol | IG14 | O₁=alpha 1,6; R₁=C16:0; R₂=OH | **92** |
| 2-amino-2-deoxy-α-D-glucopyranosyl-(1->3)-1-palmitoyl-D-*myo*-inositol | IG15 | O₁=alpha 1,6; R₁=OH; R2=C16:0 | **93** |

The term "alkyl" as used herein refers to an aliphatic hydrocarbon group, which may be straight or branched-chain, having about 1 to about 22 carbon atoms in the chain. Preferred alkyl groups have 1 to about 14 carbon atoms in the chain. Branched means that one or more lower alkyl groups such as methyl, ethyl or propyl are attached to a linear alkyl chain. "Lower alkyl" means 1 to about 6 carbon atoms in the chain, which may be straight or branched. The alkyl may be substituted with one or more "alkyl group substituents" which may be the same or different, and include halo, cycloalkyl, hydroxy, alkoxy, amino, carbamoyl, acylamino, aroylamino, carboxy, alkoxycarbonyl, aralkyloxycarbonyl, or heteroaralkyloxycarbonyl. Representative alkyl groups include methyl, trifluoromethyl, cyclopropylmethyl, cyclopentylmethyl, ethyl, n-propyl, i-propyl, n-butyl, 1-butyl, n-pentyl, 3-pentyl, methoxyethyl, carboxymethyl, methoxycarbonylethyl, benzyloxycarbonylmethyl, and pyridylmethyloxycarbonylmethyl.

The term "acyl" as used herein refers to an H-CO- or alkyl-CO- group. Preferred acyl groups include palmitoyl, lower alkyl, formyl, acetyl, propanoyl, 2-methylpropanoyl, and butanoyl.

As used herein, the term "optionally" means that the subsequently described event(s) may or may not occur, and includes both event(s), which occur, and events that do not occur.

As used herein, the term "physiologically functional derivative" refers to any pharmaceutically acceptable derivative of a compound of the present invention, for example, an ester or an amide, which upon administration to a mammal is capable of providing (directly or indirectly) a compound of the present invention or an active metabolite thereof. Such derivatives are clear to those skilled in the art, without undue experimentation, and with reference to the teaching of Burger's Medicinal Chemistry and Drug Discovery, 5th Edition, Vol 1: Principles and Practice, which is incorporated herein by reference.

As used herein, the term "solvate" refers to a complex of variable stoichiometry formed by a solute (in this invention, a compound of formula (I) or (II) or a salt or physiologically functional derivative thereof) and a solvent. Such solvents for the purpose of the invention may not interfere with the biological activity of the solute. Examples of suitable solvents include, but are not limited to, water, methanol, ethanol and acetic acid. Preferably the solvent used is a pharmaceutically acceptable solvent. Examples of suitable pharmaceutically acceptable solvents include water, ethanol and acetic acid. Most preferably the solvent used is water.

The compounds of formulae (I) or (II) may have the ability to crystallize in more than one form, a characteristic, which is known as polymorphism, and it is understood that such polymorphic forms ("polymorphs") are within the scope of formulae (I), (II), (III), and (IV). Polymorphism generally can occur as a response to changes in temperature or pressure or both and can also result from variations in the crystallization process. Polymorphs can be distinguished by various physical characteristics known in the art such as x-ray diffraction patterns, solubility, and melting point.

As used herein, the term "substituted" refers to substitution with the named substituent or substituents, multiple degrees of substitution being allowed unless otherwise stated.

The term "glucose-metabolism disorder" as used herein, is intended to refer to any disorder relating to glucose uptake or release, as well as, insulin expression, production, secretion, or usage. The glucose-metabolism disorder can be selected from, but not limited to, the group consisting of obesity, diabetes, metabolic syndrome, insulin resistance, glucose intolerance, hyerinsulinemia, Syndrome X, hypercholesterolemia, hyperlipoproteinemia, hypertriglyceridemia, atherosclerosis, and diabetic renal disease.

The term "analog" refers to a chemical compound that is structurally similar to a parent compound and has chemical properties or pharmaceutical activity in common with the parent compound. Analogs include, but are not limited to, homologs, i.e., where the analog differs from the parent compound by one or more carbon atoms in series; positional isomers; compounds that differ by interchange of one or more atoms by a different atom, for example, replacement of a carbon atom with an oxygen, sulfur, or nitrogen atom; and compounds that differ in the identity one or more functional groups, for example, the parent compound differs from its analog by the presence or absence of one or more suitable substituents.

As used herein, the term "pharmaceutically acceptable salt" refers to any salt (e.g., obtained by reaction with an acid or a base) of a compound of the present invention that is physiologically tolerated in the target subject (e.g., a mammalian subject, and/or in vivo or ex vivo, cells, tissues, or organs). "Salts" of the compounds of the present invention may be derived from inorganic or organic acids and bases. Examples of acids include, but are not limited to, hydrochloric, hydrobromic, sulfuric, nitric, perchloric, fumaric, maleic, phosphoric, glycolic, lactic, salicylic, succinic, toluene-p-sulfonic, tartaric, acetic, citric, methanesulfonic, ethanesulfonic, formic, benzoic, malonic, sulfonic, naphthalene-2-sulfonic, benzenesulfonic acid, and the like. Other acids, such as oxalic, while not in themselves pharmaceutically acceptable, may be employed in the preparation of salts useful as intermediates in obtaining the compounds of the invention and their pharmaceutically acceptable acid addition salts.

Examples of salts include, but are not limited to: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, flucoheptanoate, glycerophosphate, hemisulfate, heptanoate, hexanoate, chloride, bromide, iodide, 2-hydroxyethanesulfonate, lactate, maleate, methanesulfonate, 2-naphthalenesulfonate, nicotinate, oxalate, palmoate, pectinate, persulfate, phenylpropionate, picrate, pivalate, propionate, succinate, tartrate, thiocyanate, tosylate, undecanoate, and the like. For therapeutic use, salts of the compounds of the present invention are contemplated as being pharmaceutically acceptable. However, salts of acids and bases that are non-pharmaceutically acceptable may also find use, for example, in the preparation or purification of a pharmaceutically acceptable compound.

As used herein, the term "effective amount" means that amount of a drug or pharmaceutical agent that will elicit the biological or medical response of a tissue, system, animal or human that is being sought, for instance, by a researcher or clinician. For example, an amount sufficient to effect beneficial or desired results. Furthermore, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function. An effective amount can be administered in one or more administrations.

As used herein, a "prophylactically effective amount" refers to an amount of a compound of the invention or other active ingredient sufficient to result in the prevention of a condition or disease such as cancer, or recurrence or metastasis of cancer. A prophylactically effective amount may refer to an amount sufficient to prevent initial disease or the recurrence or spread of the disease. The term preferably encompasses a non-toxic amount that improves overall prophylaxis or enhances the prophylactic efficacy of or synergies with another prophylactic or therapeutic agent.

As used herein, the term "administration" refers to the act of giving a drug, prodrug, or other agent, or therapeutic treatment (e.g., radiation therapy) to a physiological system (e.g., a subject or in vivo, in vitro, or ex vivo cells, tissues, and organs). Exemplary routes of administration to the human body can be through the eyes (opthalmic), mouth (oral), skin (transdermal), nose (nasal), lungs (inhalant), oral mucosa (buccal), ear, by injection (e.g., intravenously, subcutaneously, intratumorally, intraperitoneally, etc.) and the like.

"Co-administration" refers to administration of more than one chemical agent or therapeutic treatment to a physiological system (e.g., a subject or in vivo, in vitro, or ex vivo cells, tissues, and organs). "Coadministration" of the respective chemical agents and therapeutic treatments may be concurrent, or in any temporal order or physical combination. The agents may be selected and administered in such a manner that their respective effects are additive or synergistic.

### Synthesis of Lipophilic Inositol Glycans

*Lipophilic IGs:* Glycosylphosphatidylinositol (GPI)-anchors with an inositol residue acylated with fatty acid are found in animals. These anchors as well as inositol-acylated free GPIs serve as precursors of acylated IGs (inositol glycanes lacking phosphate residue). GPI-PLC can not cleave GPIs with acylated inositol ring, however GPI-PLD can. Moreover, Glycosylphosphatidylinositol-specific phospholipase D (GPI-PLD) can convert free GPIs into IG's with similar biological activity to IPGs generated by GPI- PLC (Phospholipase C): for example they are able to inhibit PKA (protein kinase A) *in vitro.* However, the structure (e.g., whether they contain an acylated inositol ring) of the IGs or GPI was not known prior to this invention.

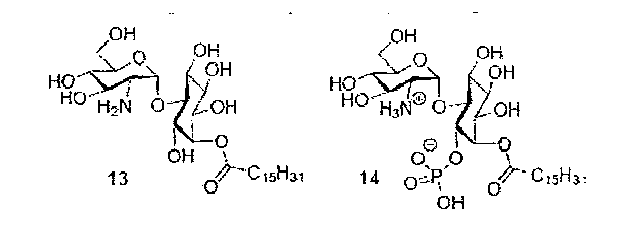

Exemplary acylated IG compounds, such as **13,** as well as acylated IPG compound **14** were synthesized and their biological activities were evaluated as shown in the figures and examples.

### Retrosynthetic analysis of targets 13 and 14

Exemplary IG compounds **13** and **14** can be obtained from the fully protected compounds **63** and **68.** The benzyl ether serves as the protective group to protect alcohols in carbohydrate chemistry. Azide serves as the protective group for an amine. Phosphate **68** in turn could be obtained from **63.**

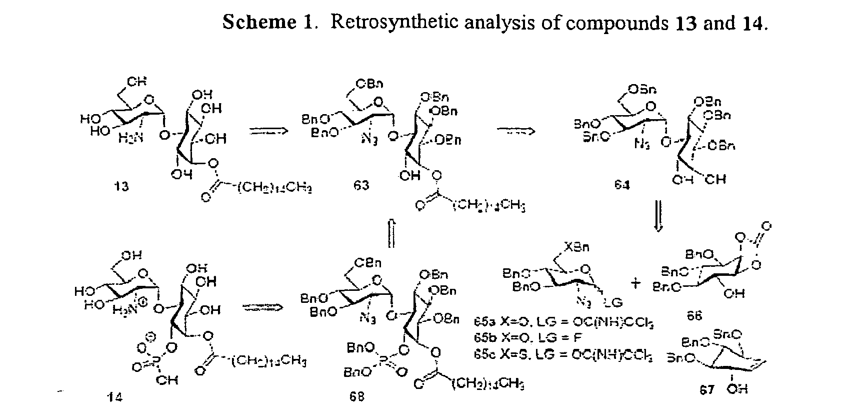

While the synthesis of palmitate **63** has been reported, the present invention discloses a different approach involving manipulations of protective groups in order to install the palmitoyl group. Selective palmitoylation of axial hydroxy-group in diol **64** was proposed in our analysis. Diol **64** is also a known compound. Compound **64** was synthesized from glycosyl fluoride **65b** and inositol residue donor, cyclic carbonate 66 (40% yield in coupling reaction, 87% yield in diole deprotcction steps). It takes 11 steps to prepare the carbonate from readily available D-xylose **69,** however it requires only 4 chromatographic purifications, the total yield is 16% and all the reactions could be scaled up to gram quantities.

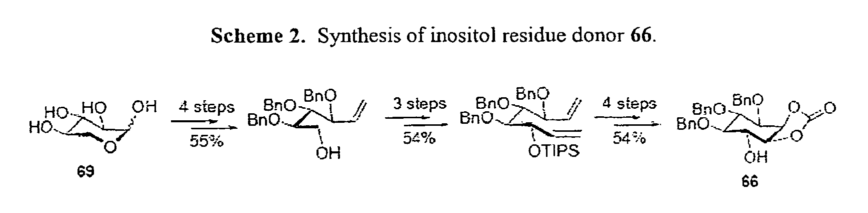

Known synthesis of diol **64** that have been reported, but contain many drawbacks.

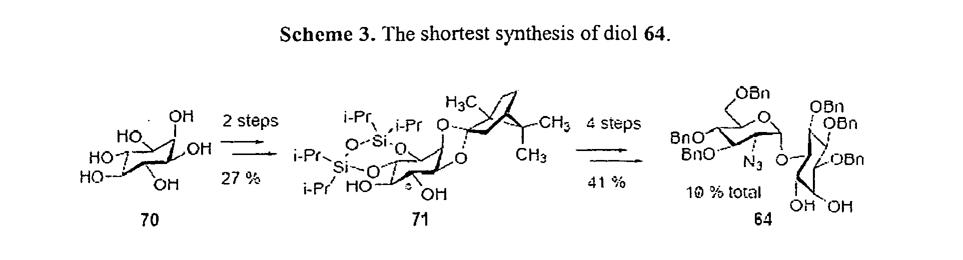

For example, the Martin-Lomas synthesis involves a tedious purification of chiral ketal of inositol with L-camphor that is the first intermediate (not shown on the scheme) (Dietrich, H.; Espinosa, J. F.; Chiara, J. L.; Jimenez-Barbero, J.; Leon, Y.; Varela-Nieto, I.; Mato, J.-M.; Cano, F. H.; Foces-Foces, C.; Martin-Lomas, M. Chem. Eur. J. 1999, 320-336.). Other reported syntheses are even less favorable: the inositol donor is prepared via a procedure that requires manipulation with several protective groups. (Cottaz, S.; Brimacombe, J. S.; Ferguson, M. A. J. Carbohydr. Res. 1995, 271, 85-91.)

It was planned to use cyclic carbonate **66** and trichloroacetylimidate **65a**. Inositol donor 66 was coupled with several glycosylfluorides only providing coupling products (*a* and, β anomers) in 50-60% combined yield. One trichloroacetimidate donor that had 6-S-benzyl-6-thio-6-deoxy substituent 65c was utilized in the synthesis of fluorescent IPG analog providing coupling product. Accordingly, we expanded the scope of trichloroacetimidate donors used in the coupling with cyclic carbonate acceptor and determined the reactivity of donor **65a**.

Yet another approach to diol **64** would involve coupling donor **65a** with acceptor **67** readily available from diene **72**. The double bond in the coupling product 73 could be dihydroxylated. It is thought that cis-alkenes are the not ideal substrates for asymmetric 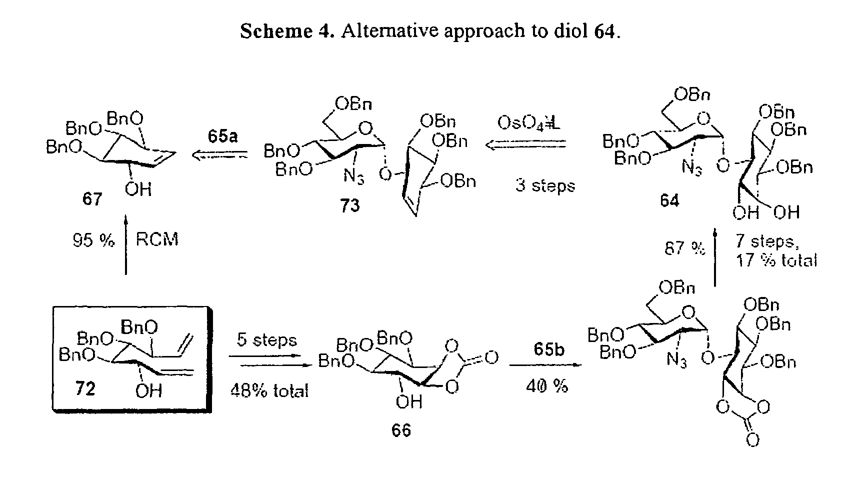 dihydroxylation reaction. However, it was proposed that some level of diastereoselectivity could be obtained because of the presence of a chiral substituents present in the alkene. The advantage of such approach is fewer reaction steps.

The success of the proposed synthesis of inositol glycans **13** and **14** depends on selective palmitoylation of the axial hydroxyl group in diol **64.**

### Methods of selective monoacylation of vicinal diols

A summary of methods aiming the preparation of mono O-acylated diols **74** is presented on Scheme 5. Unlike three previous methods, methods **D** and **E** have been used for esterification of axial hydroxy group in non-symmetric diols. In fact, an acylated inositol building block for the synthesis of palmitoylated GPI-anchor has been prepared using method **D.** Synthesis involved the protection (alkylation) of equatorial hydroxyl-group (intermediate similar to 75), esterefication of remaining alcohol and deprotection. Procedure E involves direct acylation of intermediate O-stannylene 76 (instead of alkylation to prepare 75). However, esterification of axial hydroxyl-group has been observed in a few cases only and not with inositols.

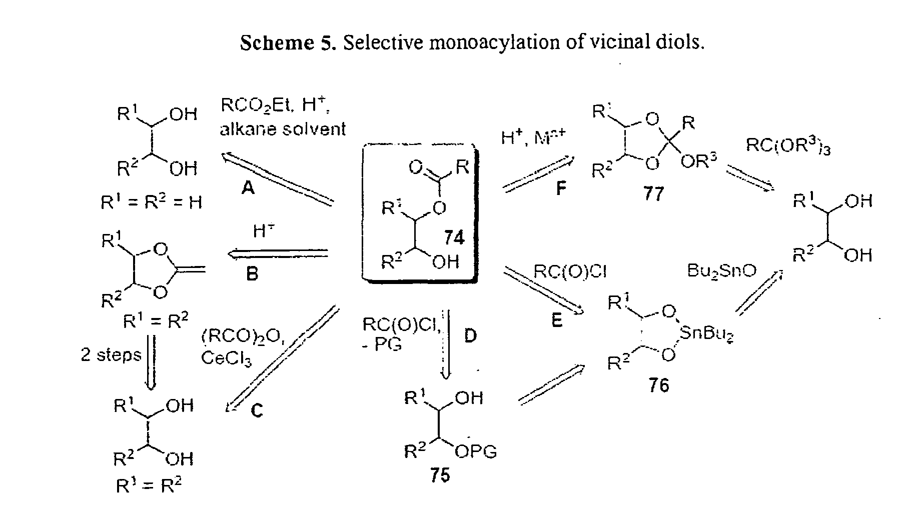

Method **E** is based on an observation of high regioselectivity of hydrolysis of ortho esters (like 77) fused with 6-membered ring. Moreover, that method seems to be applicable for regioselective monoacylation of acyclic diols. Thus, the cyclic ortho ester method was chosen for palmitoylation of **64.** However, those skilled in the are can select other methods for preparation of mono O-acylated diols

### Synthesis of Inositol Glycan 13 and Inositol Phosphate Glycan 14

Trichloacetimidate donor **65a** was coupled with cyclic carbonate **66.** Triflic acid was chosen as a promoter because TMSOTf is known to silylate acceptors during coupling reactions, however at the same time, there was a precedent of high α selectivity in a coupling reaction when TfOH was used with a more disarmed (i. e. having more electron withdrawing groups) donor.

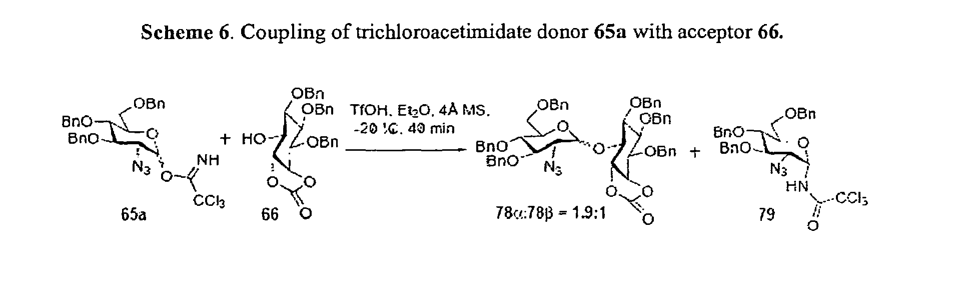

Disaccharides **78** were obtained in combined 62% yield and in modest 2:1 ratio of α to β anomers. The yield was higher as compared to the case when **65c** was used. However utilization of trichloroacetimidate instead of fluoride 65b did not increase the yield of coupling products. It was concluded that the sulfur atom probably had caused side reactions when **65c** had been used. Interestingly, a compound with presumable structure **79** was isolated from the reaction mixture. It should be pointed out, that the separation of the anomers **78** turned out to be quite difficult. However, the corresponding dioles 64α (to be referred to as 64 further in the text) and 64β obtained after cyclic carbonate hydrolysis (**Scheme 7**) are better separated. Hydrolysis can be performed without separation of the anomers. Model compound **17** was subjected to reaction with trimethyl orthopalmitate **80** before attempting to esterify quite precious **64.** Diol 17 was 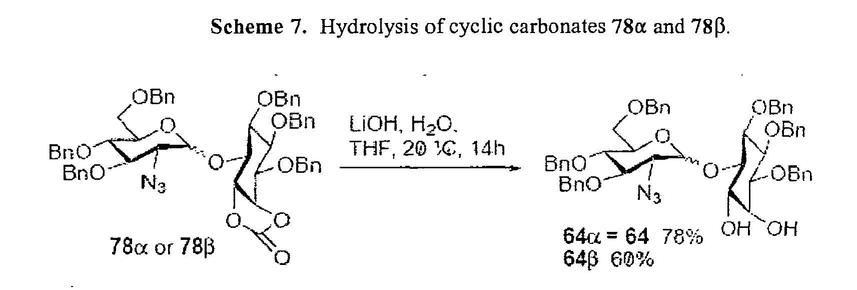 converted into a mixture of **81** and **82** in nearly quantitative yield and a 6:1 ratio (method A on **Scheme 8**)**.** An effect of (+)-Camphorsulphonic acid alone (in MeCN-DCM mixture) in the hydrolysis of intermediate cyclic ortho esters was investigated too (method **B**).

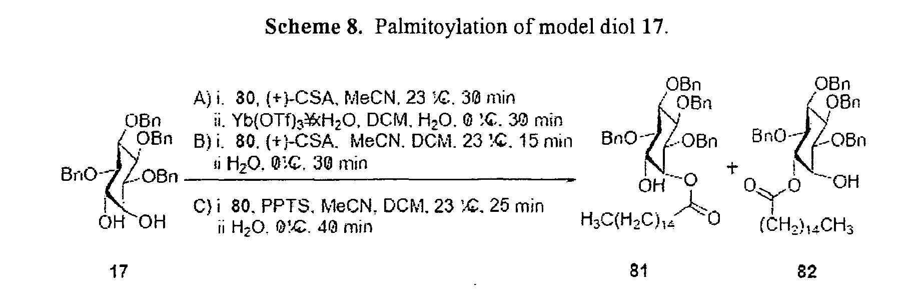

These conditions resulted in 93% yield of regioisomeric palmitates in erroded 3.6:1 ratio. Moreover, it turned out to be problematic to separate major isomer (**81**) from a side product that turned out to be methyl camphorsulfate. When another catalyst, pyridinium p-toluenesulfonate was used (method C) 94% combined yield of **81** and **82** was obtained with the highest ratio of axial to equatorial palmitates (8.2:1).

PPTS catalyzed palmitoylation was tested on diol **64**: conversion of starting material was extremely slow. The reaction can be accelerated with (+)-CSA. In a separate experiment, camphorsulfonic acid was used as the only catalyst. That reaction provided a mixture of **63** and **83** in 3.6:1 ratio and 92% combined yield. It seems that the glucosamine residue had no effect on the regioselectivity, since exactly the same ratio was obtained when model diole **17** was used as the starting material. Regioselectivity can be explored though the hydrolysis promoted with various 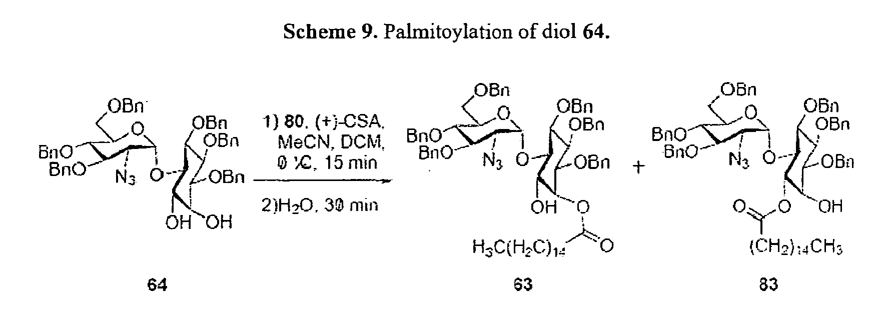 acid salts of azole derivatives. This would be analogous to the study of the effect of acid/azole complexes on the efficiency of condensation of phosphoroamidites with alcohols.

Compound **81** served as a model substance in the phosphorylation reaction with *O*, *O-*dibenzyl-*N*,*N*-diisopropylaminophosphoroamidite (and subsequent oxidation of a phosphite formed into the phosphate using the phosphoroamidite method). The success of the reaction was strongly dependent on the reaction media. Typically acetonitrile solution of tetrazole is 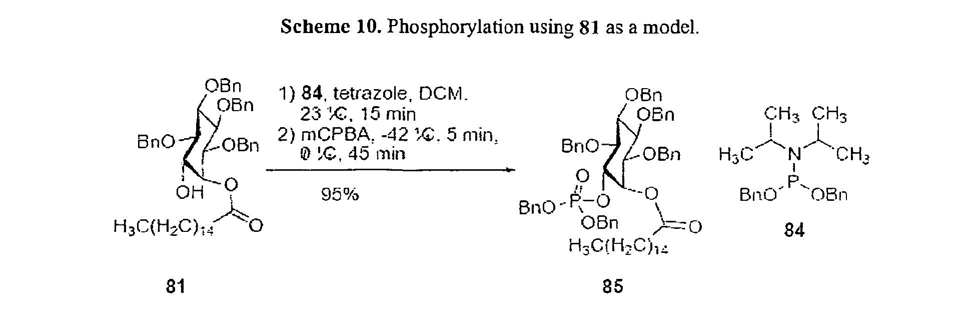 used in olygosaccharide synthesis to catalyze phosphite formation. However, when phosphorylation of **81** was promoted either by commercially available acetonitrile tetrazole solution or by a home made acetonitrile solution of tetrazole, the conversion of alcohol to phosphite was modest. When acetonitrile was completely excluded from the reaction mixture, complete conversion was achieved. Commercially available MeCN tetrazole solution has residual water. The acetonitrile used to prepare tetrazole solution in the lab was dried by passing through activated Al₂O₃ column.

Alcohol **63** was subjected to the same reaction conditions. Unexpectedly, the yield of the desired phosphate **68** was not that excellent (65%). It is possible that once formed, the phosphite formed underwent intramolecular cyclysation with azide. When the reaction mixture was accidentally allowed to sit at 23°C for 5 h after phosphite formation, the cyclization 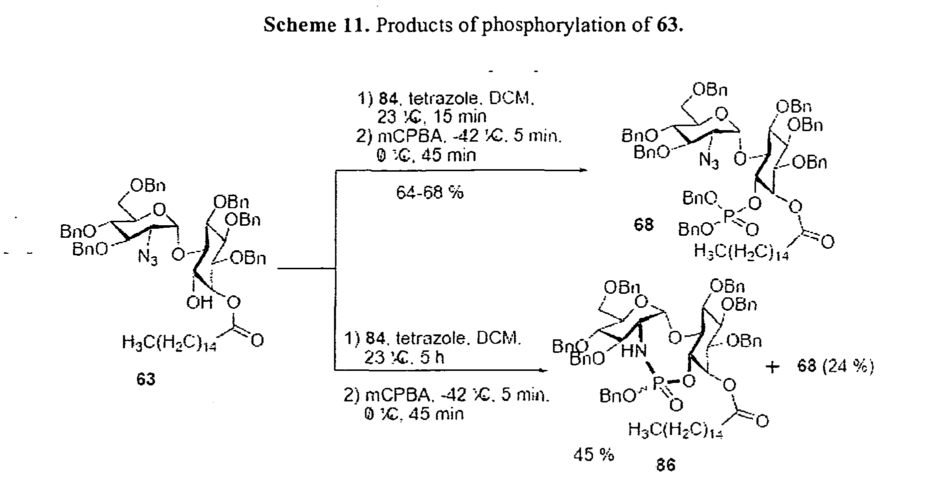 product (one of diastereomers) was indeed isolated in 45% yield together with **68** (24%). It means that the cyclisation rate is quite low and another explanation could be found accounting for the modest yield of **68**. It is possible that azido group in 63 reacts with phosphoroamidate **84** at almost the same rate that activated **84** reacts with OH group of inositol ring.

Final global removal of the benzyl groups in **63** turned out to be difficult. After several unsuccessful attempts, where benzene ring hydrogenation was observed or a fair amount of N-methylation products were detected (data is not shown), appropriate conditions were found. Compound **63** was cleanly converted into **13** at high (70 bar) hydrogen pressure in chloroform-methanol over Pd/C. Solvent proportion as well as solvent/substrate ratio were found to be critical for a sufficient rate of debenzylation. Fortunately, compound **68** was cleanly deprotected under newly found conditions to produce pure **14.**

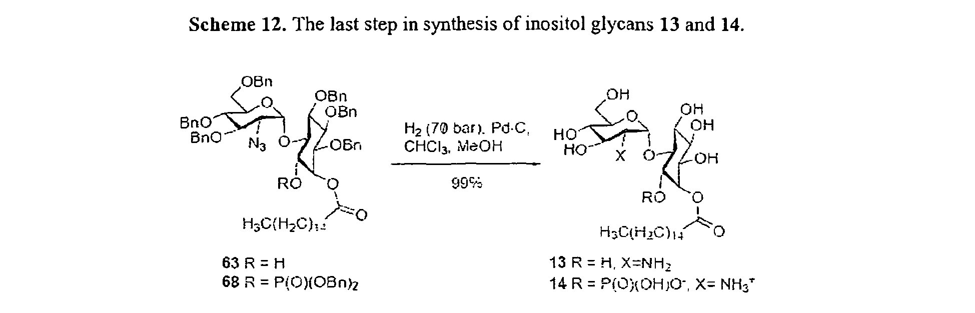

### Synthesis of 63: Dihydrxylation Approach

According to the plan presented on the **Scheme 4** coupling of allylic alcohol **67** with trichloroacetimidate 65a was investigated. There are at least two ways to control the selectivity of a newly forming anomeric bond using the trichloroacetimidate method (besides neighboring group participation).

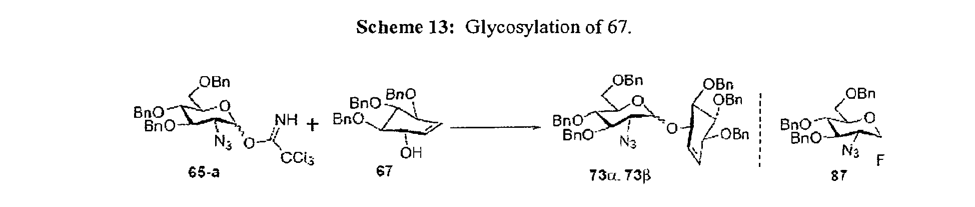

| N° | 65a α : 3 | Promoter | Conditions | Yield 73¹. % | Yield 73.1. % |
|---|---|---|---|---|---|
| 1 | 1:3 | TMSOTf | Er₂O.-78 °C | 43 | < 25² |
| 2 | 1:2.6 | BF₃ . Et₂O | CH₂Cl₂. -20°C | 17 | 34³ |
| 3 | 1:1 | TfOH | Et₂O. 23 °C | 45 | < 15 |
| 4 | 1:1 | TfOH | Et₂O. -5 °C | 58 | 21 |
| 5 | 1:1 | TfOH | Et₂O. -20 °C | 50 | 12 |

| | | | | | |
|---|---|---|---|---|---|
| ¹ Note: 73 = 73.² number corresponds to the wright of crule product. ³24% of compound 87 - | | | | | |

It has been observed generally that α-anomer is selectively formed when a coupling reaction is run in Et₂0 as a solvent with TMSOTf as a catalyst. Indeed, the result shown in entry *N°* 1 is in agreement with this observation.

Another method of stereocontrol is applied particularly to α-trichloroacetimidates. The reaction with alcohols is catalyzed by BF₃ Et₂0 in non-polar solvents, and proceeds primarily via S_{N}2-like pathway, thus giving high β selectivity. Prior to this invention, there were no examples where β-trichloroacetimidates had been utilized for the purpose of selective formation of α-glycosidic bond. Entry *Nº* 2 illustrates the result of an attempt to this transformation. The reaction conditions turned out to favor the formation of β isomer. It is interesting to note, that amount of 73β produced (in moles) is slightly higher then the amount of β-imidate (27.7 molar %) used in the reaction. This probably means that most of α-imidate has been converted to 73β and the excess of β product could come from the isomeric imidate. At the same time, the amount of **73** produced is much lower then the amount of β imidate that was used in the reaction (17 vs 72.3 molar %). Moreover, a significant amount of fluoride **87** was produced. Little is known about the mechanism of this reaction, however it was proposed that fluoride ion source is not the HF impurity in BF₃ . Et₂0. Taken together these results imply that it is not favorable to use β - **65a**) in S_{N}2-like glycosilations with BF₃ . Et₂O as a catalyst.

Entries N° 3-5 represent the results of replacement of TMSOTf for TfOH. That resulted in a slightly increased yield. The increased yield may be the result of heating, since lowering temperature resulted in lower yield of **73** (entry N° 5).

After optimizing the yield in glycosylation step, dihydroxylation of compound **73** was attempted (**Scheme 14**). Initially the dihydroxylation did not proceed under catalytic conditions (entrees N° 1-3). It was found that addition of OsO₄ amine (amine = pyridine and (DHQ)₂PHAL) complexes to alkene 73 procedes quite readily. However, hydrolysis of the osmate ester formed was extremely slow under standard conditions employed in a procedure for asymmetric dihydroxylation (K₂CO₃, ^{t}BuOH/H₂O). It is likely that the cause of such low reactivity is limited solubility of starting material in ^{t}BuOH. That fact became apparent when the reaction was

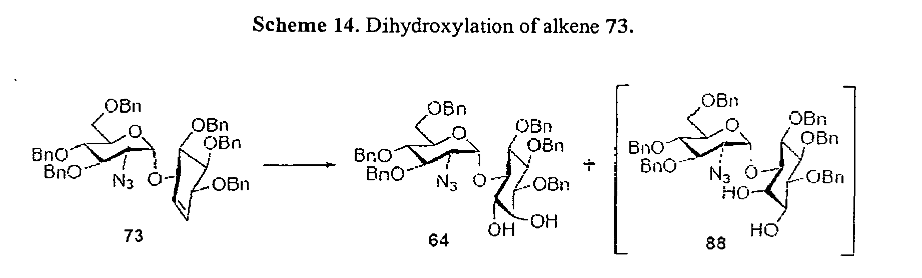

| *Nº* | Conditions | Yield 73 and 88. % |
|---|---|---|
| 1 | OsO₄ (5 mol %). Py, NMO | NR² |
| 2 | AD-mix-α (5 fold excess). MeSO₂NH₂ (1 equiv). 'BuOH/H₂O | NR |
| 3 | (DHQ)₂PHAL (10 mol %). OsO₄ (5 mol %). K₂CO₃ (38 equiv). K₃[Fc(CN)₆] (3 equiv). MeSO₂NH₂ (1 equiv). ^{t}BuOH/H₂O/THF | NR |
| 4 | 1) OsO₄ (1 equiv), Py (5 equiv); 2) NaHSO₃/Py/H₂O (twice): 3) Na₂SO₃, K₂CO₃, ^{t}BuOH/H₂O | 95 |
| 5 | 1)(DHQ)₂PHAL (1.2 equiv), OsO₄ (1 equiv), 'BuOH/H₂O/ THF; 2) NaHSO₃/Py/H₂O | 38 |
| 6 | 1)(DHQ)₂PHAL (1.2 equiv), OsO₄ (1 equiv), 'BuOH/H₂O/ THF: 2) NaHSO₃ | 70 |
| 7 | 1)(DHQ)₂PHAL (1.2 equiv), OsO₄ (1 equiv). 'BuOH/H₂O/ THF; 2) LiOH/MeOH/THF | 29 |
| 8 | 1) OsO₄ (1 equiv), Py (24 equiv); 2) NaHSO₃/Py/H₂O. 3) Na₂SO₃, K₂CO₃, ^{t}BuOH/H₂O/dioxane/toluene. DABCO | 90 |
| 9 | 1) DABCO (10 mol %), OsO₄ (10 mol %), K₂CO₃ (3 equiv), K₃[Fe(CN)₄] (3 equiv). MeSO₂NH₂ (2 equiv), ^{t}BuOH/H₂O/THF : 2) NaOH (1 equiv)3 | 95 |
| 10 | 1) See exper *N*º3. except only 3 equiv, of K₂CO₃ were used. 2) NaOH (1 equiv)³ | 64 |

| | | |
|---|---|---|
| ¹ 88 has newer been isolated in pure form and characterised. ² no reaction, ³ added at 60 ; conversion | | |

attempted to run on ca. 30 mg scale (entry N° 2). THF was added to the reaction mixture as a co-solvent in a different experiment (entry N° 3). The effect was the same. The dihydroxylation stoichiometrically reaction was run with 1 equivalent offs04 and a small excess of the amine ligand and to search for the good hydrolytic conditions for the osmate formed. Mixture NaHSO₃/Py/H₂O are efficient in the hydrolysis of osmate esters (complexed with pyridine) of very lipophilic diols. Indeed, it was efficient in the hydrolysis of osmate ester complexed with (DHQ)₂PHAL and pyridine, but the yield of diastereomeric diols was variable (entrees N° 4 and 5). It is likely that azide could be partially reduced. Sodium bisulfite itself was quite efficient (entree N° 6). Hydrolysis under more harsh basic conditions (LiOH/MeOH/THF with myo-inositol additive) was quite fast, but the yield of diols was less then modest (entree N° 7). DABCO can be more favorable then quinuclidine (a parent of (DHQ)₂PHAL) in accelerating catalytic dihydroxylation of sterically hindered alkenes. Entry N° 8 shows that addition of DABCO to the base promoted hydrolysis accelerated the reaction.

One more detail was taken into account. In the conditions of experiment N° 3 an excess of K₂CO₃ was used since it was predicted that the rate of dihydroxylation decreases proportionally to a decrease in pH of the reaction mixture. However, only 3 equivalents of K₂CO₃ were employed in the experiment N° 9. The dihydroxylation under these conditions was the most efficient. A control experiment with conditions similar to instance N° 3 was performed to check if addition of only 3 equivalents of potassium carbonate would change the outcome of the reaction. After about 20 h ca. 60 % conversion was observed. 1 equiv of NaOH was added at this point. Some further progress was observed by TLC, however the reaction never reached the point of complete conversion of starting materials, despite extra amine, osmium teraoxide and NaOH added. Thus it seems that the right combination of solvents as well as an ionic strength of the reaction media are critical for the catalytic dihydroxylation.

The diols produced were difficult to separate from each other. Thus, a mixture of **64** and **88** was palmitoylated under the conditions that had been used for the esterification of pure **64.** The separation of four palmitates turned out to be quite tedious. While moderately pure **63** (sometimes contaminated with a methyl ester of camphorsulfonic acid) and **89** could 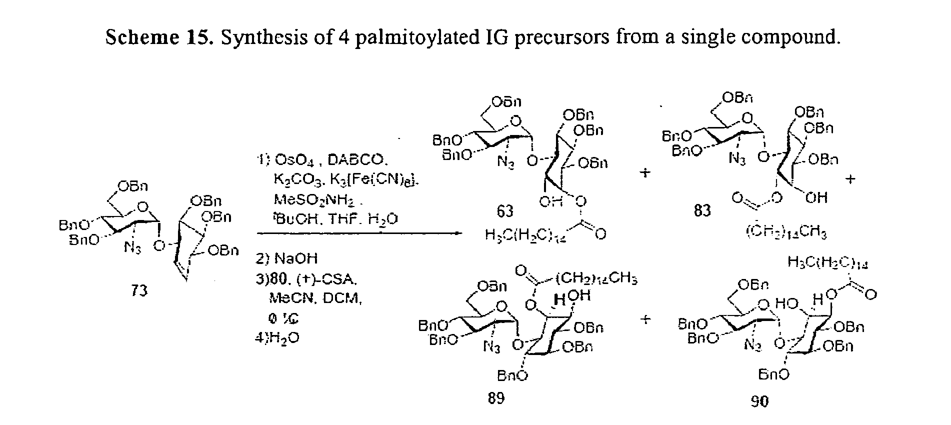 be separated on one flash chromatography column, additional purification step for two minor isomers **83** and **90** was required. Since **63** and **83** were synthesized independently, they were easily identified in the products. Compounds **89** and **90** were distinguished from each other based on the signals of a diagnostic hydrogen atom in inositol ring in ¹H NMR spectrum. In compound **89,** the hydroxyl group that is geminal to equatorial hydrogen atom (H-2, shown in bold on **Scheme 15)** is esterified. As a result, the signal of the H-2 is shifted upfield (8=5.72) because of that. Unlike in **90** the signal of H-2 is at 4.24 ppm.

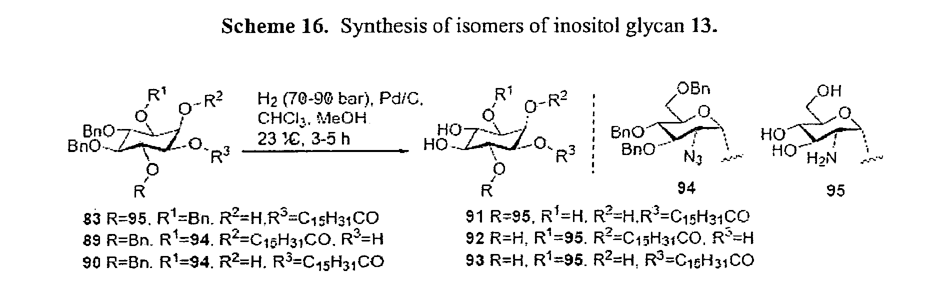

The separation of all the palmitates enabled unambiguous determination of the diastereoselectivity in dihydroxylation of **73.** The ratio of the **63** and **83** to **89** and **90** was very close to 1. Tthe chiral centers in **73** did not induce any diastereoselectivity.

Palmitates **83, 89** and **90** were successfully debenzylated under the conditions developed for **63** providing access to isomers of compound **63 (Scheme 16).**

The synthesis of exemplary lipophilic inositol glycan compounds was accomplished. The presence of the palmitate residue in **63** and **68** had special requirements for debenzylation conditions: high pressure, proper choice of solvent and substrate concentration.

PPTS has been never employed for regioselective cleavage of cyclic ortho-esters (like 7 7 on Scheme 5). The highest selectivity was achieved in a reaction where **17** was used as the starting material and PPTS was a catalyst. However, the same catalyst was not as effective in the reaction of disaccharide **64.**

A new route to the IG precursor **63** was developed. Two critical steps, the glycosylation and the dihydroxylation reactions were optimized. While the dihydroxylation is not essentially diastereoselective in favor to the desired diol **64** at the moment, the yield of **6 4** from the common 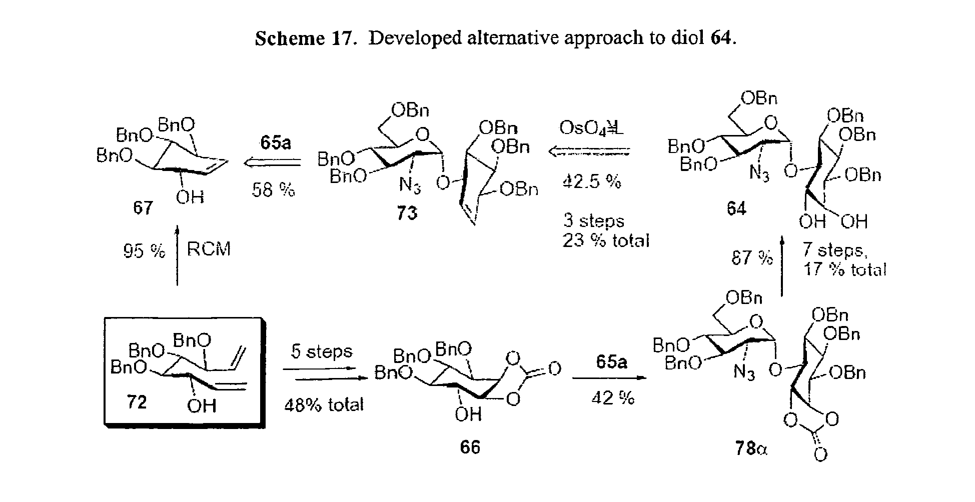 precursor 72 is slightly higher when compared to the target-oriented synthesis developed before (23% vs. 17%). Nevertheless, the possibility of improving the diastereoselectivity still exist. For example, it is desirable to run the dihydroxilation with other chiral amines, in particular with DHQ-IND or DHQD-IND. These amines were found to induce the highest ee with cis-alkenes when compared with other ligands. Since DABCO itself was very efficient in promoting dihydroxylation of 73. The enantioselectivities obtained with that amine were very modest (ca. 25%).

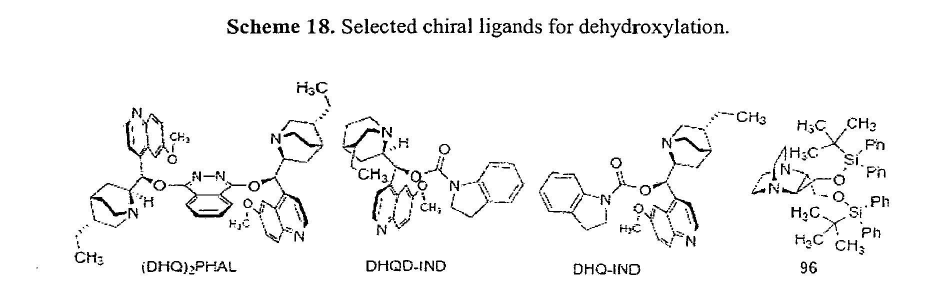

Alkene 73 can serve as a precursor of inositol glycans incorporating *chiro*inositol. The chemistry disclosed in this invention can be used for the synthesis of palmitoylated oligasaccharides having a glucosamine substituted with one or several mannoses. For example, palmitoylated disaccharids having β(1-46) glycosidic bond orientation (that is, the ,β isomer of 13, for example) can be synthesized using the methods disclosed herein.

### Pharmaceutical Compositions

The lipophilic inositol glycan compounds of formula (I) and (II), and salts, solvates and physiological functional derivatives thereof, of the present invention can be administered by virtually any mode and can be administered simultaneously or serially. In another embodiment, the lipophilic inositol glycan compounds can be administered transdermally via a transdermal patch.

The lipophilic inositol glycan compounds of formula (I) and (II), and salts, solvates and physiological functional derivatives thereof, can be administered therapeutically to treat, prevent, or slow the rate of cell proliferation associated with cancer, or prophylactically to either protect against cell proliferation associated with cancer or to avoid or forestall the onset of cell proliferation associated with other disorders. For example, the lipophilic inositol glycan compositions can be administered prophylactically to slow or halt the progression of cancer in a patient who has be diagnosed or is at risk of developing cancer.

The lipophilic inositol glycan compounds of formula (I) and (II), and salts, solvates and physiological functional derivatives thereof, can be administered to a subject, using a wide variety of routes or modes of administration. Suitable routes of administration include, but are not limited to, oral inhalation; nasal inhalation; transdermal; oral; rectal; transmucosal; intestinal; and parenteral administration, including intramuscular, subcutaneous, and intravenous injections. The lipophilic inositol glycan compounds of formula (I) and (II), and salts, solvates and physiological functional derivatives thereof, can be administered via the same or via a different mode of administration. For example, a lipophilic inositol glycan compound with a pharmaceutically acceptable salt or hydrate can be administered orally or can be administered via a transdermal patch, an aerolized formulation, by nasal inhalation, or via nano- or micro- encapsulated formulations. The lipophilic inositol glycan compounds can be administered by intrathecal and intraventricular modes of administration.

Various combinations of the lipophilic inositol glycan compounds of formula (I) and (II), and salts, solvates and physiological functional derivatives thereof, can be administered. For example IG1 and its, analogs, IG2 and its analogs, or a combination of IG1 and IG2 or their various analogs. In addition, the compounds can be administered in a combination with other therapeutic agents. Of course, the choice of therapeutic agents that can be co-administered with the composition of the invention will depend, in part, on the condition being treated. For example, the compounds of the invention can be administered in cocktails comprising other agents used to cancer and other symptoms and side effects commonly associated with cancer.

The lipophilic inositol glycan compounds of formula (I) and (II), and salts, solvates and physiological functional derivatives thereof, can be formulated either as single compound per se or as mixtures of compounds of the same type (e.g., two different analogs or isomers), as well as mixtures of different lipophilic inositol glycan compounds (e.g. one or more of IG1, IG2, IG 13, IG 14, IG 15). Such compositions will generally comprise a lipophilic inositol glycan compound formulated as a pharmaceutically acceptable salt or hydrate.

Pharmaceutical compositions for use in accordance with the present invention can be formulated in conventional manner using one or more physiologically acceptable carriers, excipients, diluents or auxiliaries that further facilitate processing of the lipophilic inositol glycan compounds of formula (I) and (II), and salts, solvates and physiological functional derivatives thereof. The choice of formulation is dependent upon the selected administration route.

The formulations can be administered by implantation or transcutaneous delivery (for example subcutaneously or intramuscularly), intramuscular injection, or transdermally. Thus, for example, the lipophilic inositol glycan compounds of formula (I) and (II), and salts, solvates and physiological functional derivatives thereof, can be formulated with suitable polymeric or hydrophobic materials (such as an emulsion in an acceptable oil) or ion exchange resins.

Formulations suitable for transdermal administration of compounds are described in U.S. Pat. Nos. 5,725,876; 5,716,635; 5,633,008; 5,603,947; 5,411,739; 5,364,630; 5,230,896; 5,004,610; 4,943,435; 4,908,213; and 4,839,174, which patents are hereby incorporated herein by reference. As lipophilic inositol glycan compounds, pharmaceutically acceptable salts or hydrates are readily absorbed and cross cell membranes and the blood-brain barrier. Any of these formulations can be routinely adapted for transdermal administration.

For injection, the lipophilic inositol glycan compounds of formula (I) and (II), and salts, solvates and physiological functional derivatives thereof, can be formulated in physiologically compatible aqueous solutions, such as Hanks's solution, Ringer's solution, or physiological saline buffer. For transmucosal administration, penetrants appropriate to the barrier to be permeated are used in the formulation. Such penetrants are generally known in the art.

For oral administration, the lipophilic inositol glycan compounds of formula (I) and (II), and salts, solvates and physiological functional derivatives thereof, can be formulated with pharmaceutically acceptable carriers well known in the art. Such carriers enable the compounds of the invention to be formulated for oral administration as tablets, pills, gums dragees, capsules, liquids, gels, syrups, slurries, suspensions and the like. Alternatively, the compounds can be formulated into candies, cookies, or other edible foodstuffs. Pharmaceutical preparations for oral use can be obtained by mixing the compounds of the invention with a solid excipient, optionally grinding the resulting mixture, and processing the mixture of granules, after adding suitable auxiliaries, if desired, to obtain tablets or dragee cores. Suitable excipients include, but are not limited to, fillers, such as sugars, including lactose, sucrose, mannitol, or sorbitol; cellulose preparations, such as maize starch, wheat starch, rice starch, potato starch, gelatin, gum tragacanth, methylcellulose, hydroxypropylmethyl-cellulose, sodium carboxymethylcellulose, and polyvinylpyrrolidone (PVP). If desired, disintegrating agents can be added, such as cross-linked polyvinyl pyrrolidone, agar, or alginic acid or a salt thereof, such as sodium alginate.

Concentrated sugar solutions can be used that can optionally contain gum arabic, talc, polyvinyl pyrrolidone, carbopol gel, polyethylene glycol, or titanium dioxide, lacquer solutions, and suitable organic solvents or solvent mixtures. Dyestuffs or pigments can be added to the tablets or coatings for identification or to characterize different combinations of active compound doses.

Pharmaceutical preparations that can be used orally include push-fit capsules made of gelatin, as well as soft, sealed capsules made of gelatin and a plasticizer, such as glycerol or sorbitol. The push-fit capsules can contain the compounds of the invention in an admixture with filler, such as lactose; binders, such as starches; or lubricants, such as talc or magnesium stearate; or stabilizers. In soft capsules, the compounds of the invention can be dissolved or suspended in suitable liquids, such as fatty oils, liquid paraffin, or liquid polyethylene glycols. In addition, stabilizers can be added to the soft-capsule formulation. All formulations for oral administration should be in dosages suitable for such administration.

For buccal administration, the compositions can take the form of oral sprays, tablets, gums, or lozenges formulated by well-known methods. A candy formulation suitable for oral or buccal administration of therapeutic compounds, pharmaceutically acceptable salts and hydrates is described in U. S. Pat. No. 6,083,962, which is hereby incorporated herein by reference. Additional formulations suitable for oral or buccal administration of therapeutic compounds, are described in U.S. Pat. Nos. 5,939,100; 5,799,633; 5,662,920; 5,603,947; 5,549,906; D358,683; 5,326,563; 5,293,883; 5,147,654; 5,035,252; 4,967,773; 4,907,606; 4,848,376; and 4,776,353, which are hereby incorporated herein by reference. All of these formulations can be routinely adapted for administration of lipophilic inositol glycan compounds, pharmaceutically acceptable salts and hydrates.

Compositions for inhalation or insufflation include solutions and suspensions in pharmaceutically acceptable, aqueous or organic solvents, or mixtures thereof, and powders. Preferably the compositions are administered by the oral or nasal respiratory route for local or systemic effect. For administration by oral or nasal inhalation, the compounds of the invention are conveniently delivered in the form of an aerosol spray delivered via pressurized packs or a nebulizer, with a suitable propellant, e.g., carbon dioxide or other suitable gas. In the case of a pressurized aerosol, the dosage unit can be controlled by a dose-metered valve. Capsules and cartridges, e.g. gelatin, for use in an inhaler or insufflator can be formulated as a powder mix of the compounds if the invention and a suitable powder base, such as lactose or starch. Formulations suitable for nasal inhalation are well known in the art. For example, a nasal aerosol spray may contain lipophilic inositol glycan compounds of formula (I) and (II), and salts, solvates and physiological functional derivatives thereof, a water-soluble diluent such as an organic acid, and a thickening agent such as a natural or synthetic polymer or an oil substance comprising the oil phase of an emulsion. The compounds of the invention can also be administered in a vaporizer that delivers a volume of vapor containing lipophilic inositol glycan compounds of formula (I) and (II), and salts, solvates and physiological functional derivatives thereof. The vaporizer can be battery operated and designed to deliver a dosage of lipophilic inositol glycan compound effective to inhibit cell proliferation (e.g., lung cells). The compounds of the invention, in a sterile pharmaceutically acceptable solvent, may be nebulized by use of inert gases. Nebulized solutions may be breathed directly from the nebulizing device or the nebulizing device may be attached to a face mask, tent or intermittent positive pressure breathing machine.

For administration by injection, the compounds of the invention can be formulated with a surface-active agent (or wetting agent or surfactant) or in the form of an emulsion (as a water-in-oil or oil-in-water, emulsion). Suitable surface-active agents include, but are not limited to, non-ionic agents, such as polyoxyethylenesorbitans (e.g. Tween^{™} 20, 40, 60, 80 or 85) and other sorbitans (e.g. Span 20, 40, 60, 80 or 85). Compositions with a surface-active agent may comprise between 0.05 and 5% surface-active agent, and preferably between 0.1 and 2.5%. It will be appreciated that other ingredients may be added, for example mannitol or other pharmaceutically acceptable vehicles, if necessary.

Suitable emulsions may be prepared using commercially available fat emulsions, such as Intralipid, Liposyn, Infonutrol, Lipofundin and Lipiphysan. The active ingredient may be either dissolved in a pre-mixed emulsion composition or alternatively it may be dissolved in an oil (e.g. soybean oil, safflower oil, cottonseed oil, sesame oil. corn oil or almond oil) and an emulsion formed upon mixing with a phospholipid (e.g. egg phospholipids, soybean phospholipids or soybean lecithin) and water. It will be appreciated that other ingredients may be added, for example gylcerol or glucose, to adjust the tonicity of the emulsion. Suitable emulsions will typically contain up to 20% oil, for example, between 5 and 20%. The fat emulsion will preferably comprise fat droplets between 0.1 and 1.0 µm, particularly 0.1 and 0.5 µm, and have a pH in the range of 5.5 to 8.0.

The lipophilic inositol glycan compounds of formula (I) and (II), and salts, solvates and physiological functional derivatives thereof, can be formulated for parenteral administration by injection, e.g., by bolus injection or continuous infusion. Formulations for injection can be presented in unit-dosage form, e.g., in ampules or in multi-dose containers, optionally with an added preservative. The compositions can take such forms as suspensions, solutions, or emulsions in oily or aqueous vehicles, and can contain formulatory agents, such as suspending, stabilizing, or dispersing agents.

Pharmaceutical formulations for parenteral administration include aqueous solutions of the compounds of the invention in water-soluble form. Additionally, suspensions of the compounds of the invention can be prepared as appropriate oily-injection suspensions. Suitable lipophilic solvents or vehicles include fatty oils, such as sesame oil, or synthetic-fatty-acid esters, such as ethyl oleate or triglycerides, or liposomes. Aqueous-injection suspensions can contain substances that increase the viscosity of the suspension, such as sodium carboxymethyl cellulose, sorbitol, or dextran. Optionally, the suspension can contain suitable stabilizers or agents that increase the solubility of the compounds of the invention to allow for the preparation of highly concentrated solutions.

Where appropriate, dosage unit formulations for oral administration can be microencapsulated. The formulation can also be prepared to prolong or sustain the release as for example by coating or embedding particulate material in polymers, wax or the like.

Alternatively, the lipophilic inositol glycan,compounds of formula (I) and (II), and salts, solvates and physiological functional derivatives thereof, can be in powder form for constitution with a suitable vehicle, e.g., sterile pyrogen-free water, before use.

The lipophilic inositol glycan compounds of formula (I) and (II), and salts, solvates and physiological functional derivatives thereof, can also be formulated in rectal compositions, such as suppositories or retention enemas, e.g., containing conventional suppository bases, such as cocoa butter or other glycerides.

The pharmaceutical compositions also can comprise suitable solid- or gel-phase carriers or excipients. Examples of such carriers or excipients include, but are not limited to, calcium carbonate, calcium phosphate, various sugars, starches, cellulose derivatives, gelatin, and polymers, such as polyethylene glycols.

### Effective Dosages

Pharmaceutical preparations suitable for use with the present invention include compositions wherein the lipophilic inositol glycan compounds of formula (I) and (II), and salts, solvates and physiological functional derivatives thereof, are present in effective amounts, i.e., in amounts effective to achieve the intended purpose, for example, treating, preventing, or reducing cell proliferation, or cancer. A therapeutically effective amount of a compound of the present invention will depend upon a number of factors including, for example, the age and weight of the animal, the precise condition requiring treatment and its severity, the nature of the formulation, and the route of administration, and will ultimately be at the discretion of the attendant physician or veterinarian. Determination of effective amounts is well within the capabilities of those skilled in the art. However, an effective amount of a compound of formula (I) for the treatment of neoplastic growth, for example colon or breast carcinoma, will generally be in the range of 0.1 to 100 mg/kg body weight of recipient (mammal) per day and more usually in the range of 1 to 10 mg/kg body weight per day. Thus, for a 70 kg adult mammal, the actual amount per day would usually be from 70 to 700 mg and this amount may be given in a single dose per day or more usually in a number (such as two, three, four, five or six) of sub-doses per day such that the total daily dose is the same. An effective amount of a salt or solvate, or physiologically functional derivative thereof, may be determined as a proportion of the effective amount of the compound of formula (I) per se. It is envisaged that similar dosages would be appropriate for treatment of the other conditions referred to above.

The compounds of the present invention and their salts and solvates, and physiologically functional derivatives thereof, may be employed alone or in combination with other therapeutic agents for the treatment of the above-mentioned conditions. In particular, in anti-cancer therapy, combination with other chemotherapeutic, hormonal or antibody agents is envisaged as well as combination with surgical therapy and radiotherapy. Combination therapies according to the present invention thus comprise the administration of at least one compound of formula (I) or a pharmaceutically acceptable salt or solvate thereof, or a physiologically functional derivative thereof, and the use of at least one other cancer treatment method. Preferably, combination therapies according to the present invention comprise the administration of at least one compound of formula (I) or a pharmaceutically acceptable salt or solvate thereof, or a physiologically functional derivative thereof, and at least one other pharmaceutically active agent, preferably an anti-neoplastic agent. The compound(s) of formula (I) and the other pharmaceutically active agent(s) may be administered together or separately and, when administered separately this may occur simultaneously or sequentially in any order. The amounts of the compound(s) of formula (I) and the other pharmaceutically active agent(s) and the relative timings of administration will be selected in order to achieve the desired combined therapeutic effect.

In some embodiments, the lipophilic inositol glycan compounds of formula (I) and (II), and salts, solvates and physiological functional derivatives thereof, can be co-administered with one or more alkyl, acyl-phosphatidic acid (AAPA) compounds of formula (III). Non-limiting examples of alkyl, acyl-phosphatidic Acid (AAPA) compounds include AAPA1 (1-0-alkyl (18:0)-2-0-acyl-(18:0)-sn-glycerol-phosphate), AAPA2 (1-0-akyl (18:0)-2-0-acyl-(20:4)-sn-glycerol-phosphate) and AAPA3 (1-0-alkyl (18:0)-2-0-acyl-(22:4)-sn-glycerol-phosphate). In preferred embodiments, co-administration results in synergistic effects. Aspects of the invention include administering to a patient, compositions providing one or more lipophilic inositol glycan compounds and/or one or more alkyl, acyl-phosphatidic acid (AAPA) compounds to a patient.

The compounds of the formula (I) and/or (II) or salts, solvates, or physiologically functional derivatives thereof and at least one additional cancer treatment therapy may be employed in combination concomitantly or sequentially in any therapeutically appropriate combination with such other anti-cancer therapies. In some embodiments, the compounds of the formula (I) or salts, solvates, or physiologically functional derivatives thereof, can be co-administered with both at least one AAPA compound and at least one additional cancer treatment therapy may be employed. In some embodiments, the other anti-cancer therapy is at -least one additional chemotherapeutic therapy including administration of at least one anti-neoplastic agent. The administration in combination of a compound of formula (I) or salts, solvates, or physiologically functional derivatives thereof with other anti-neoplastic agents may be in combination in accordance with the invention by administration concomitantly in (1) a unitary pharmaceutical composition including both compounds or (2) separate pharmaceutical compositions each including one of the compounds. Alternatively, the combination may be administered separately in a sequential manner wherein one anti-neoplastic agent is administered first and the other second or vice versa. Such sequential administration may be close in time or remote in time.

Anti-neoplastic agents may induce anti-neoplastic effects in a cell-cycle specific manner, i.e., are phase specific and act at a specific phase of the cell cycle, or bind DNA and act in a non cell-cycle specific manner, i.e., are non-cell cycle specific and operate by other mechanisms.

Non-limiting anti-neoplastic agents useful in combination with the compounds and salts, solvates or physiologically functional derivatives thereof of formula I include the following:
(1) cell cycle specific anti-neoplastic agents including, but not limited to, diterpenoids such as paclitaxel and its analog docetaxel; vinca alkaloids such as vinblastine, vincristine, vindesine, and vinorelbine; epipodophyllotoxins such as etoposide and teniposide; fluoropyrimidines such as 5-fluorouracil and fluorodeoxyuridine; antimetabolites such as allopurinol, fludurabine, methotrexate, cladrabine, cytarabine, mercaptopurine and thioguanine; and camptothecins such as 9-amino camptothecin, irinotecan, CPT-11 and the various optical forms of 7-(4-methylpiperazino-methylene)-10,11-ethylenedioxy-20-camptothecin;
(2) cytotoxic chemotherapeutic agents including, but not limited to, alkylating agents such as melphalan, chlorambucil, cyclophosphamide, mechlorethamine, hexamethylmelamine, busulfan, carmustine, lomustine, and dacarbazine; anti-tumour antibiotics such as doxorubicin, daunomycin, epirubicin, idarubicin, mitomycin-C, dacttinomycin and mithramycin; and platinum coordination complexes such as cisplatin, carboplatin, and oxaliplatin; and
(3) other chemotherapeutic agents including, but not limited to, anti-estrogens such as tamoxifen, toremifene, raloxifene, droloxifene and iodoxyfene; progestrogens such as megestrol acetate; aromatase inhibitors such as anastrozole, letrazole, vorazole, and exemestane; antiandrogens such as flutamide, nilutamide, bicalutamide, and cyproterone acetate; LHRH agonists and antagagonists such as goserelin acetate and luprolide, testosterone 5.alpha.-dihydroreductase inhibitors such as finasteride; metalloproteinase inhibitors such as marimastat; antiprogestogens; urokinase plasminogen activator receptor function inhibitors; cyclooxygenase type 2 (COX-2) inhibitors such as celecoxib; other angiogenic inhibiting agents such as VEGFR inhibitors other than those described herein and TIE-2 inhibitors; growth factor function inhibitors such as inhibitors of the functions of hepatocyte growth factor; erb-B2, erb-B4, epidermal growth factor receptor (EGFr), platelet derived growth factor receptor (PDGFr), vascular endothelial growth factor receptor (VEGFR) other than those described in the present invention, and TIE-2; and other tyrosine kinase inhibitors such as cyclin dependent inhibitors such as CDK2 and CDK4 inhibitors.

In particular, the compounds of the present invention can also be used in the treatment of certain forms of cancer. Furthermore, the compounds of the present invention can be used to provide additive or synergistic effects with certain existing cancer chemotherapies and radiation, and/or be used to restore effectiveness of certain existing cancer chemotherapies and radiation.

The lipophilic inositol glycan compounds of formula (I) and/or (II), and salts, solvates and physiological functional derivatives thereof, can be administered in any manner that achieves the requisite therapeutic or prophylactic effect. Therapeutically or prophylactically effective doses of the compounds of the invention can be determined from animal or human data for analogous compounds that are known to exhibit similar pharmacological activities. The applied doses can be adjusted based on the relative bioavailability, potency and *in vivo* half-life of the administered compounds as compared with these other agents.

Adjusting the dose to achieve maximal efficacy in humans based on the methods described above and other methods that are well-known is well within the capabilities of the ordinarily skilled artisan.

The compounds of the present invention are also useful in the treatment of one or more diseases afflicting mammals which are characterized by cellular proliferation including blood vessel proliferative disorders including arthritis and restenosis; fibrotic disorders including hepatic cirrhosis and atherosclerosis; mesangial cell proliferative disorders include glomerulonephritis, diabetic nephropathy, malignant nephrosclerosis, thrombotic microangiopathy syndromes, proliferative retinopathies, organ transplant rejection and glomerulopathies; and metabolic disorders, glucose-metabolism disorders, psoriasis, diabetes mellitus, chronic wound healing, inflammation and neurodegenerative diseases.

One skilled in the art will appreciate further features and advantages of the invention based on the above-described embodiments. Accordingly, the invention is not to be limited by what has been particularly shown and described, nor by the examples set forth below. All publications and references cited throughout this application, for example patent documents including issued or granted patents or equivalents; patent application publications; and non-patent literature documents or other source material; are hereby incorporated by reference herein in their entireties, as though individually incorporated by reference, to the extent each reference is at least partially not inconsistent with the disclosure in this application (for example, a reference that is partially inconsistent is incorporated by reference except for the partially inconsistent portion of the reference).

When a group of substituents is disclosed herein, it is understood that all individual members of those groups and all subgroups, including any isomers and enantiomers of the group members, and classes of compounds that can be formed using the substituents are disclosed separately. When a Markush group or other grouping is used herein, all individual members of the group and all combinations and subcombinations possible of the group are intended to be individually included in the disclosure. When a compound is described herein such that a particular isomer or enantiomer of the compound is not specified, for example, in a formula or in a chemical name, that description is intended to include each isomer and enantiomer of the compound described individually or in any combination. When an atom is described herein, including in a composition, any isotope of such atom is intended to be included. Specific names of compounds are intended to be exemplary, as it is known that one of ordinary skill in the art can name the same compounds differently. Every formulation or combination of components described or exemplified herein can be used to practice the invention, unless otherwise stated. Whenever a range is given in the specification, for example, a temperature range, a time range, or a composition range, all intermediate ranges and subranges, as well as all individual values included in the ranges given are intended to be included in the disclosure.

Where the terms "comprise", "comprises", "comprised", or "comprising" are used herein, they are to be interpreted as specifying the presence of the stated features, integers, steps, or components referred to, but not to preclude the presence or addition of one or more other feature, integer, step, component, or group thereof.

The invention has been described with reference to various specific and preferred embodiments and techniques. However, it should be understood that many variations and modifications may be made while remaining within the spirit and scope of the invention. It will be apparent to one of ordinary skill in the art that methods, devices, device elements, materials, procedures and techniques other than those specifically described herein can be applied to the practice of the invention as broadly disclosed herein without resort to undue experimentation. All art-known functional equivalents of methods, devices, device elements, materials, procedures and techniques described herein are intended to be encompassed by this invention. Whenever a range is disclosed, all subranges and individual values are intended to be encompassed. This invention is not to be limited by the embodiments disclosed, including any shown in the drawings or exemplified in the specification, which are given by way of example or illustration and not of limitation.

### EXAMPLES

As demonstrated by the Examples, the present invention provides novel synthesis methods for lipophilic inositol glycan compositions, and methods for use of these compounds in the treatment of cancer and glucose-metabolism disorders, including, but not limited to, diabetes, obesity and metabolic syndrome.

### I. Synthesis of Exemplary Lipophilic Inositol Glycan Compounds:

Glucosamine donor **65a** was prepared as described (Dietrich, H.; Espinosa, J. F.; Chiara, J. L.; Jimenez-Barbero, J.; Leon, Y.; Varela-Nieto, I.; Mato, J.-M.; Cano, F. H.; Foces-Foces, C.; Martín-Lomas, M. Chem. Eur. J. 1999, 320-336; Kinzy, W.; Schmidt, R. R. Liebigs Ann. Chem. 1985, 1537-1545.) The anomers ratio was 1:1, when neat CCI₃CN was used. It is possible to get about 1:3 (α/β) ration when 5-10 equiv of CCl₃CN in CH₂Cl₂ were utilized. Trimethyl ortho palmytate **80** was prepared according to literature procedure from palmitonitrile (Pressova, M.; Smrt, J. Collect. Czech. Chem. Commun. 1989, 54, 487-497). Palmitonitrile was synthesized from commercially available palmitoyl chloride (Pern, R. B.; Gribble, G. Org. Prep. Proced. Int. 1983, 15, 297-302.). Pyridinium p-toluenesulfonate was prepared as described (Miyashita, M.; Yoshikoshi, A.; Grieco, P. A. J. Org. Chem. 1977, 42, 3772-3774.). *O*,*O-*dibenzyl-*N,N*-diisopropylaminophosphoroamidite was prepared from phosphorous trichloride, benzyl alcohol and diisopropylamine via two step procedure (Tanaka, T.; Tamatsukuri, S.; Ikehara, M. Tetrahedron Lett. 1986, 27, 199-202; Uhlmann, E.; Engels, J. Tetrahedron Lett. 1986, 27, 1023-1026.). Reagents obtained from commercial sources were used without further purification with the following exceptions. THF, toluene and methylene chloride were purified using an alumina purification system (Pangborn, A. B.; Giardello, M. A.; Grubbs, R. H.; Rosen, R. K.; Timmers, F. J. Organometallics 1996, 15, 1518-1520.). Benzyl alcohol and diisipropylamine were distilled from CaH₂. Phosphorous trichloride was refluxed for 2 h and redistilled. BF₃·Et₂O was purified by distillation from calcium hydride *in vacou* as described (Zweifel, G.; Brown, H. C. Hydration of Olefines, Dienes, and Acetylenes via Hydroboration. In Organic Reactions; Cope, A. C, Ed.; Jonh Wiley & Sons: New York, 1963; Vol. 13, pp. 142-144.). HPLC grade chloroform was shaked with conc. H₂SO₄ (3 times), washed with water, dried over CaCl₂ and distilled from P₂O₅. Starting materials were dried by coevaporation with toluene. Rotary evaporation was accomplished on at 2-10 Torr and 23 °C. Reactions were monitored by TLC on Baker glass-backed silica gel plates with a 254-nm fluorescent indicator or stained using a solution of 2.5% p-anisaldehyde, 3.5 % H₂SO₄, 1 % AcOH in ethanol. NMR data were obtained for ¹H at 300 MHz, ¹³C at 75 MHz, ¹⁹F at 283 MHz, ³¹P at 121 MHz and processed in DISNMR or XWinNMR software environments. TMS (0.03%) was used as an internal standard in CDCl₃ solutions. Chemical shifts for the compounds dissolved in d4-MeOH and d3-MeCN were referenced to the corresponding residual solvent peak. LR MS were recorded on Thermo Finnigan LTQ instrument with ESI inlet in 50 % aqueous methanol containing 1 % acetic acid (positive ionization mode) or in 50 % aqueous methanol containing 0.2 % triethylamine (negative ionization mode).

### 2-Azido-3,4,6-tri-O-benzyl-2-deoxy-D-glucopyranosyl-(1→ 6)-3,4,5-tri-O-benzyl-D-myo-inos itol-1,2-cyclic carbonate (78 α/β) and N-(2-azido-3,4,6-tri-O-benzyl-1,2-dideoxy-α-D-glucopyranosyl)trichloroacetamide (79).

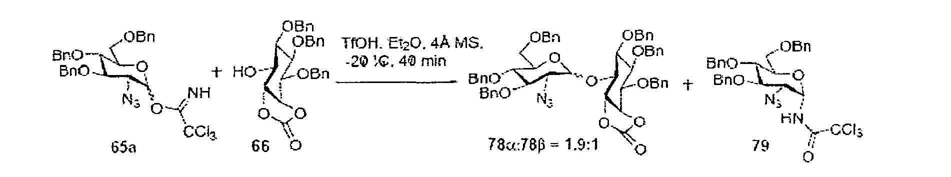

Anhydrous ether (2 mL) was added to a mixture of donor **65a** (34.5 mg, 55.8 µmol), acceptor **66** (25 mg, 5.25 µmol) and crushed, activated molecular sieves (55 mg) under Ar. The reaction mixture was chilled to -20 °C, TfOH (75 µL of 0.0704 M solution in Et₂O, 0.1 equiv) was introduced dropwise, and the reaction mixture was stirred at -20 °C for 1h and then allowed to warm up to 20 °C. Solid NaHCO₃ (100 mg) was added, the reaction mixture was stirred for 15 min and filtered. The filtrate was washed successively with cold 5% aq Na₂CO₃ (3 x 0.5 mL), water (2 x 0.5 mL) and with brine (2 x 0.5 mL), dried over Na₂SO₄ and evaporated. The crude reaction mixture was separated by flash chromatography (1:9 → 1:6 → 1:4.5 → 1:4 v/v EtOAc/Hex) giving **79** (6.5 mg, 20%), **78**β (11 mg, 22.4%) and **78**α (20.5 mg, 42%). For **79:** *R_{f}*=0.65 (1 : 3 v/v EtOAc/Hex); ¹H NMR (CDCl₃) δ 3.57-3.73 (m, 3H, **H**-3, H-5, **H**-6), 3.78-3.96 (m, 3H, **H**-6', **H**-4, H-2), 4.49 (d, J = 11.94, 1H, Ph**CH**₂O), 4.58 (d, J = 10.63, 1H, Ph**CH₂**O), 4.65 (d, J = 11.95, 1H, Ph**CH₂**O), 4.77 (d, J = 10.62, 1H, Ph**CH**₂O), 4.85 (d, J = 10.66, 1H, Ph**CH**₂O), 4.92 (d, J = 10.67, 1H, Ph**CH₂**O), 5.66 (dd, J = 6.55, 5.37 Hz, 1H, **H**-1), 7.00 (d, J = 6.48, 1H, NH), 7.1-7.2 (m, 2H, **Ph**CH₂O), 7.2-7.4 (m, 15H, **Ph**CH₂O (13H) + C**H**Cl₃). NMR data foranomers **78** was only briefly described (Jaworek, C. H. Synthesis of Inositol Phosphate Glycans. Ph. D. Thesis, Tufts University, Medford, MA, May 2000.). For **78**β: *R_{f}*=0.36 (1 : 3 v/v EtOAc/Hex); ¹H NMR (CDCl₃) δ 3.38-3.53 (m, 3H), 3.65-3.78 (m, 3H), 3.83 (ψt, J = 3.57 Hz, 1H, Ino-H), 3.9-3.95 (m, 1H), 4.35 (d, J = 11.68 Hz, 1H, Ph**CH₂**O), 4.4 (d, J = 11.64 Hz, 1H, Ph**CH₂**O), 4.55-4.74 (m, 8H, Ph**CH₂**O (6H), GluN-**H**-1, Ino-H), 4.74-4.95 (m, 6H, Ph**CH₂**O (4H), Ino-**H** (2H)), 7.18-7.45 (m, 36.3H, **Ph**CH₂O (30H) + C**H**CI₃). For **78**α *R_{f}*=0.31 (1 : 3 v/v EtOAc/Hex); ¹H NMR (CDCl₃) δ 3.33 (dd, J= 11.07, 1.82 Hz, 1H, GluN-H-6), 3.38-3.49 (m, 2H, GluN-**H**-2, GluN-**H**-6'), 3.58 (dd, J = 9.98, 3.64 Hz, 1H, Ino-H), 3.74 (dd, J = 9.87, 8.99 Hz, 1H, GluN-H-4), 3.8 (ψt, J = 3.39 Hz, 1H, Ino-H), 3.85-3.95 (m, 2H, GluN-H-6, Ino-H), 3.97-4.09 (m, 1H, GluN-**H**-5), 4.1 (d, J = 12.06 Hz, PhCH₂O), 4.07 (s, 2H, Ph**CH₂**O), 4.44-4.62 (m, 6H, Ph**CH**₂O (5H), Ino-**H** (1H)), 4.67 (d, J = 12.19 Hz, 1H, Ph**CH**₂O), 4.74-4.93 (m, 5H, Ph**CH₂**O (3H), Ino-**H** (2H)), 5.32 (d, J = 3.61 Hz, 1H, GluN-**H**-1), 7.18-7.45 (m, 34.2H, **Ph**CH₂O (30H) + C**H**Cl₃).

### 3,4,5,6-Tetra-O-benzyl-2-O-palmitoyl-myo-inositol (81) and 3,4,5,6-tetra-O-benzyl-1-O-palmitoyl-myo-inositol (82).

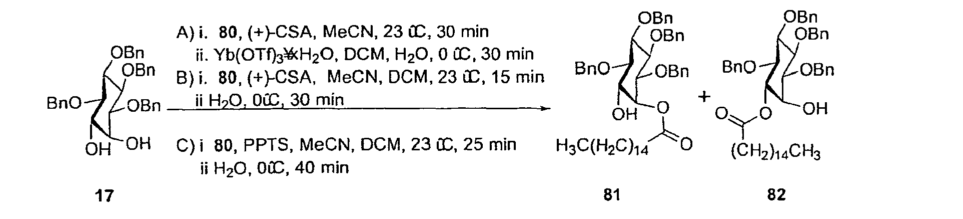

**Method A**. Trimethyl orthopalmitate (**80**, 137 µL, 117 mg, 0.37 mmol, 10 equiv) was added via syringe to a solution of diole **17** (20 mg, 37 µmol) in 3 mL of MeCN under Ar. The Ar inlet was quickly removed, solid (+)-CSA (9 mg, 37 µmol, 1 equiv) was quickly added, the Ar inlet was reattached to the reaction flask, and the reaction mixture was stirred at 23 °C for 30 min. TLC showed 4 spots with *R_{f}*=0.32, 0.42, 0.76, 0.95 (1 : 3 v/v EtOAc/Hex). Starting material 17 was absent in the reaction mixture. Volatile materials were evaporated *in vacuo,* the residue was redissolved in CH₂Cl₂ under Ar and chilled in an ice bath (0 °C). The Ar inlet was quickly removed, solid Yb(OTf)₃ (23 mg, 37 µmol, 1 equiv) was added in one portion, the Ar inlet was reconnected, and the reaction mixture was stirred at 0 °C for 1h. TLC showed essentially the same set of spots: *R_{f}*=0-3, 0.62, 0.8, 0.95 (1 : 5 v/v EtOAc/Hex). Water (1 drop) was added and the reaction mixture was stirred at 0 °C for 30 min. TLC showed 3 spots with *R_{f}*=0.3, 0.5 and 0.95. Triethylamine (0.5 mL) was added to the reaction mixture and volatile materials were evaporated *in vacuo.* Products were separated by flash chromatography (1:7 → 1:6 v/v EtOAc/Hex) providing 21 mg of pure **81**, 7 mg of a mixture of **81** with the methyl ester of camphorsulfonic acid (**MeCSA**) and 4 mg of almost pure **82** (14%). The mixture of **81** and **MeCSA** was separated via PTLC providing 4 mg of pure **81** (combined yield 86%). **Method B**. Trimethyl orthopalmitate (**80**, 314 µL of 0.2 M solution in MeCN, 1.5 equiv) was syringed to a flask with diole **17** (22.6 mg, 41.9 µmol) under Ar and the mixture was stirred for 5 min. A solution of (+)-CSA (1.9 mg, 8.18 µmol, 0.2 equiv in 1 mL of CH2C12) was added dropwise and the reaction mixture was stirred at 23 °C for 30 min. Water (1 drop) was added at 0 °C, reaction mixture was stirred for 30 min at this temperature and was worked up in the same way as in **Method A** providing 22 mg (73%) of **81** and 6 mg (20%) of **82**.

**Method C**. Trimethyl orthopalmitate (**80**, 600 µL of 0.2 M solution in MeCN, 3 equiv) was syringed to a flask with diole **17** (22.5 mg, 41.7 µmol) under Ar and the mixture was stirred for 5 min. The Ar inlet was quickly removed, solid PPTS (2 mg, 8.34 µmol, 0.2 equiv) was quickly added, the Ar inlet was reattached to the reaction flask, and the reaction mixture was stirred at 23 °C for 25 min. Water (100 µL of 2.1M solution in MeCN, 210 µmol, 5 equiv) was added at 0 °C, reaction mixture was stirred for 40 min at this temperature and was worked up in the same way as in **Method A** providing 27.3 mg (84%) of **81** and 3.3 mg (10%) of **82**. For **81** *R_{f}*=0.32 (1 : 3 v/v EtOAc/Hex), ¹H NMR spectrum was identical to that described in the literature (Schlueter, U.; Lu, J.; Fraser-Raid, B. Org. Lett. 2003, 5, 255-257.) For **MeCSA** *R_{f}*=0.42 (1 : 3 v/v EtOAc/Hex) ¹H NMR (CDCl₃) δ 0.88 (s, 3H), 1.11 (s, 3H), 1.45 (ddd, J = 12.67, 9.24, 3.77 Hz, 1H), 1.68 (ddd, J = 13.85, 9.28, 4.51 Hz, 1H), 1.96 (d, J = 18.53 Hz), 2.1-2.17 (m, 2H), 2.35-2.55 (m, 2H), 2.95 (d, J = 15.13 Hz, 1H), 3.62 (d, J = 15.13 Hz, 1H), 3.96 (s, 3H). For **82** *R_{f}*=0.6 (1 : 3 v/v EtOAc/Hex). ¹H NMR (CDCl₃) δ 0.88 (m, 3H, CH₂CH₂(CH₂)₁₂**CH₃**), 1.17-1.35 (m, 24H, CH₂CH₂(**CH₂**)**₁₂**CH₃), 1.53-1.65 (m, 8H, CH₂CH₂(**CH₂**)₁₂CH₃ (2H), "H₂O" (6H)), 2.2-2.41 (m, 2H, **CH₂**CH₂(CH₂)12CH₃), 2.42 (s, 1H, **OH**), 3.5-3.59 (m, 2H, **H**-3, **H**-5), 3.94 (ψt, J = 9.51 Hz, 1H, **H**-4), 4.07 (ψt, J = 9.67, 1H, **H**-6), 4.29 (ψt, J = 2.65 Hz, 1H, **H**-2), 4.67 (d, J = 11.61 Hz, 1H, Ph**CH₂**O), 4.71 (d, J = 11.61 Hz, 1H, Ph**CH₂**O), 4.72 (d, J = 11.21 Hz, 1H, Ph**CH₂**O), 4.8-4.92 (m, 6H, Ph**CH₂**O (5H), **H**-1), 7.2-7.4 (m, 23.4H, **Ph**CH₂O (20H) + **CH**Cl₃).

### 2-Azido-3,4,6-tri-O-benzyl-2-deoxy-αD-glucopyranosyl-(1→6)-3,4,5-tri-O-benzyl-2-palmito yl-D-myo-inositol (63) and 2-azido-3,4,6-tri-O-benzyl-2-deoxy-α-D-glucopyranosyl-(1→6)-3,4,5-tri-O-benzyl-1-palmitoyl-D-myo-inositol (83)

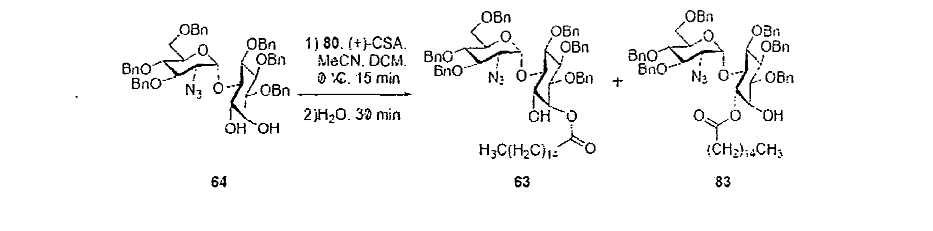

Diole **64** (15.5 mg, 17.1 µmol) was dissolved in CH₂Cl₂ (150 µL) under Ar. Trimethyl orthopalmitate (**80**, 450 µL of 0.114 M solution in MeCN, 3 equiv) was added with stirring. The reaction mixture was chilled to 0 °C and (+)-CSA (0.3 mL of 0.0144 M solution, 0.25 equiv) was added. The reaction mixture was stirred for 15 min at 0 °C and a solution of 2.5 mg of (+)-CSA in 500 µL MeCN, 100 µL CH₂Cl₂ and 30 µL H₂O (32 µL, corresponds to 5 equiv of H₂O) is added. The reaction mixture was stirred for 30 min at 0 °C and quenched with Et₃N (0.1 mL). Volatile materials were evaporated *in vacuo.* Products were separated by flash chromatography (1:14 v/v EtOAc/PhH) providing a mixture of **83** with **63** and a major fraction that had only **63**. The two isomers were separated by PTLC (1:3 v/v EtOAc/Hex) providing pure **83** (4 mg, 20.4%) and an additional amount of **63** (14 mg, 71.4% combined yield). For **63**: *R_{f}*=0.22 (1:14 v/v EtOAc/PhH); ¹H NMR spectrum was identical to that described in the literature (Liu X.; Seeberger, P. Chem. Commun. 2004, 1708-1709.) For **83** *R_{f}*=0.41 (1:14 v/v EtOAc/PhH); 1H NMR (CDCl₃) δ 0.88 (m, 3.6H, CH₂CH₂(CH₂)₁₂**CH₃** (3H)), 1.19- 1.4 (m, 29.2H, CH₂CH₂(**CH**₂)₁₂CH₃ (3H)), 1.6-1.75 (m, 2H, CH₂**CH₂**(CH₂)₁₂CH₃ (2H)), 2.32, (s, 1H, **OH**), 2.42 (t, J = 7.64 Hz, 2H, **CH**₂CH₂(CH₂)₁₂CH₃), 3.03 (dd, J = 11.2, 2.07 Hz, 1H, GluN-**H**-6), 3.11 (dd, J = 11.28, 1.72 Hz, 1H, GluN-**H**-6'), 3.29 (dd, J = 10.3, 3.73 Hz, 1H, GluN-**H**-2), 3.46 (ψt, J = 9.44 Hz, 1H, Ino-**H**-5), 3.6 (dd, J = 9.51, 2.63 Hz, 1H, Ino-**H**-3), 3.7 (dd, J = 10.06, 9.00, 1H, GluN-**H**-4), 3.87-4.05 (m, 3H, GluN-**H**-3, GluN-**H**-5, Ino-**H**-4), 4.18-4.41 (m, 4H, Ph**CH**₂O (2H), Ino-**H**-2, Ino-**H**-6), 4.5 (d, J = 12.05 Hz, Ph**CH**₂O), 4.6-4.74 (m, 4H, Ph**CH**₂O), 4.8-4.98 (m, 5H, Ph**CH**₂O (4H), Ino-**H**-1), 5.05 (d, J = 10.9 Hz, 1H, Ph**CH**₂O), 5.34 (d, J = 3.69 Hz, 1H, GluN-**H**-1), 7.05-7.15 (m, 4H), **Ph**CH₂O), 7.15-7.4 (m, 32H, **Ph**CH₂O (30H) + **CH**Cl₃); {¹H}¹³C NMR (CDCl₃) δ 173.5, 138.8, 18-7,138.4, 138.38, 138.3, 137.8, 129.0, 128.9, 128.8, 128.7, 128.6, 128.55, 128.5, 128.45, 128.4, 128.3, 128.1, 128.06, 128.0, 127.9, 127.8, 98.6, 82.0, 81.0, 80.4, 80.2, 78.6, 76.4, 76.1, 75.7, 75.1, 74.7, 74.68, 73.8, 73.2, 70.9, 68.1, 67.8, 63.5, 34.8, 32.4, 30.15, 30.1, 30.05, 29.9, 29.8, 29.7, 29.6, 25.1, 23.1, 14.6.

### 3,4,5,6-Tetra-O-benzyl-1-O-(dibenzylphosphono)-2-O-palmitoyl-myo-inositol (85).

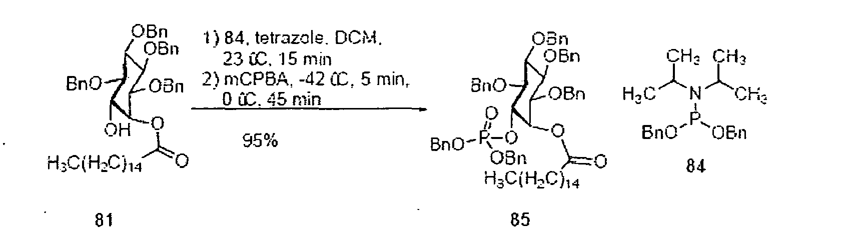

Alcohol **81** (8.3 mg, 10.6 µmol) and tetrazole (4.5 mg, 64 µmol, 6 equiv) were coevaporated together with toluene *in vacuo.* CH₂Cl₂ (0.7 mL) was added to the reactants under Ar followed by amidite **84** (160 µL of 0.2 M solution in CH₂Cl₂, (3 equiv). The reaction mixture was stirred for 30 min at 23 °C. TLC analysis (1:3 v/v EtOAc/Hex) indicated the absence of starting material (*R_{f}*=0.22). The reaction mixture was chilled to -42 °C and mCPBA (0.5 mL of 0.095 M solution in CH₂Cl₂, 47.7 µmol, 4.5 equiv) was added. The reaction mixture was stirred for 5 min at -42 °C and 30 min at 0 °C. Dichloromethane (1 mL) was added and the reaction mixture was quenched with Na₂S₂O₃ (1 mL of 1 M solution) at 0 °C. The organic layer was separated and washed successively with 1M Na₂S₂O₃ (3 x 0.5 mL), water (1 x 0.5 mL), 5% NaHCO₃ (3 x 0.5 mL), water (1 x 0.5 mL) and brine (2 x 1 mL). The organic phase was dried over Na₂SO₄ and evaporated. Residue was purified by PTLC (1:3 v/v EtOAc/Hex) providing 10.4 mg (95%) of pure 85. For **85** *R_{f}*=0.46 (1:3 v/v EtOAc/Hex); ¹H NMR (CDCl₃) δ 0.88 (m, 3H, CH₂CH₂(CH₂)₁₂**CH**₃), 1.15-1.38 (m, 25.7H, CH₂CH₂(**CH**₂)₁₂CH₃ (24H)), 1.55-1.67 (m, 4.16H, CH₂**CH**₂(CH₂)₁₂CH₃ (2H), "H₂O"), 2.38 (m, 2H, **CH**₂CH₂(CH2)₁₂ CH₃), 3.47-3.57 (m, 2H, **H**-3, H-5), 3.83 (ψt, J = 9.58 Hz, 1H, **H**-4), 3.83 (ψt, J = 9.65 Hz, 1H, **H**-6), 4.36-4.45 (m, 2H, **H**-1 (1H), Ph**CH**₂O (1H)), 4.7 (d, J = 10.88 Hz, 1H, Ph**CH**₂O), 4.73-5.08 (m, 10H, Ph**CH**₂O), 5.94 (ψt, J = 2.68 Hz, 1H, **H**-2), 7.15-7.4 (m, 33.26H, **Ph**CH₂O (30H) + C**H**Cl₃); {¹H} ¹³C NMR (CDCl₃) δ 173.0, 138.7, 138.45, 138.4, 137.7, 136.1, 136.0, 135.95, 135.9, 128.75, 128.7, 128.6, 128.55, 128.5, 128.3, 128.2, 128.0, 127.9, 127.7, 82.8, 81.4, 80.3, 80.2, 78.4, 76.6, 76.5, 76.2, 75.8, 72.4, 69.7, 69.6, 69.4, 69.3, 68.5, 34.6, 32.1, 29.9, 29.7, 29.6, 29.2, 25.5, 22.9, 14.4; {¹H}³¹P NMR (CDCl₃) 6 0.14.

### 2-Azido-3,4,6-tri-O-benzyl-2-deoxy-α-D-glucopyranosyl-(1→6)-3,4,5-tri-O-benzyl-1-O-(dibe nzylphosphono)-2-palmitoyl-D-myo-inositol (68).

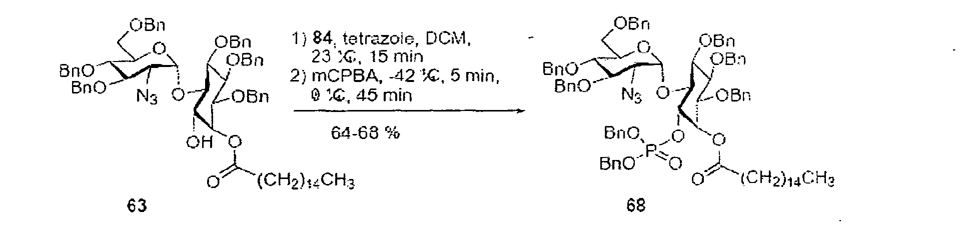

Alcohol **63** (2.7 mg, 2.36 µmol) and tetrazole (2.6 mg, 37.7 µmol, 16 equiv) were coevaporated together with toluene *in vacuo.* CH₂Cl₂ (0.5 mL) was added to the reactants under Ar followed by amidite **84** (95 µL of 0.2 M solution in CH₂Cl₂, 8 equiv). The reaction mixture was stirred for 20 min at 23 °C. TLC analysis (1:3 v/v EtOAc/Hex) indicated the absence of starting material (*R_{f}*=0.35). The reaction mixture was chilled to -42 °C and mCPBA (0.5 mL of 0.057 M solution in CH₂Cl₂, 28.3 µmol, 12 equiv) was added. The reaction mixture was stirred for 5 min at -42 °C and 40 min at 0 °C. Dichloromethane (1 mL) was added and the reaction mixture was quenched with Na₂S₂O₃ (1 mL of 1 M solution) at 0 °C. Organic layer was separated and washed successively with 1M Na₂S₂O₃ (3 x 0.5 mL), water (1 x 0.5 mL), 5% NaHCO₃ (3 x 0.5 mL), water (1 x 0.5 mL) and brine (2 x 1 mL). The organic phase was dried over Na₂SO₄ and evaporated. Residue was purified by flash chromatography (1:5 v/v EtOAc/Hex) providing 2.4 mg of material contaminated with 25 mol % (by NMR) of (BnO)₂P(O)N(*ⁱ*Pr)₂. This material was combined with the products obtained after phosphorylation of a second batch of **63** with 10 equiv of tetrazole, 5 equiv of **84** and 7.5 equiv mCPBA. Combined mixture was purified by PTLC (150:100:2 v/v/v Et2O/Hex/EtOH) to produce 5.4 mg (2.25 mg (68%) from the first batch and 3.15 mg (64%) from the second) of **68**. For **68**: *R_{f}*=0.53 (150:100:2 v/v/v Et2O/Hex/EtOH) ¹H NMR (CDCl₃) δ 0.88 (m, 3H, CH₂CH₂(CH₂)₁₂**CH**₃), 1.15-1.35 (m, 26H, CH₂CH₂(**CH2**)**₁₂**CH₃ (24H)), 1.53-1.65 (m, 7.5H, CH₂**CH₂**(CH₂)₁₂CH₃ (2H), "H₂O"), 2.25-2.45 (m, 2H, **CH₂**CH₂(CH₂)₁₂CH₃, 3.2 (dd, J = 10.33, 3.77 Hz, 1H, GluN-**H**-2), 2.25 (dd, J = 11.1, 2.14 Hz, 1H, GluN-**H**-6), 3.32 (m, 1H, GluN-**H**-6'), 3.44 (ψt, J = 9.44 Hz, 1H, Ino-**H**-5), 3.51 (dd, J = 9.8, 2.65 Hz, 1H, Ino-**H**-3), 3.68 (ψt, J = 9.92 Hz, 1H, GluN**H**-4), 3.81 (ψt, J = 9.56 Hz, 1H, Ino-**H**-4), 3.98 (ψt, J = 10.18 Hz, 1H, GluN-**H**-3), 4.08 (m, 1H, GluN-**H**-5), 4.21 (ψt, J = 9.63 Hz, 1H, Ino-**H**-6), 4.25 (d, J = 11.96 Hz, 1H, PhC**H**₂O), 4.37 (d, J = 11.01 Hz, 1H, Ph**CH**₂O), 4.40 (d, J = 10.74 Hz, 1H, Ph**CH**₂O), 4.52 (m, 1H, Ino-**H**-1), 4.53 (d, J = 12.06 Hz, 1H, Ph**CH₂**O), 4.65-4.78 (m, 4H, Ph**CH**₂O), 4.85 (s, 2H, Ph**CH**₂O), 4.90 (d, J = 10.59 Hz, 1H, Ph**CH**₂O), 4.96 (d, J = 10.63 Hz, 1H, Ph**CH**₂O), 5.0 (d, ³J_{P-H} = 8.43 Hz, 2H, Ph**CH₂**O), 5.05 (d, ³J_{P-H} = 8.47 Hz, 2H, Ph**CH**₂O), 5.52 (d, J = 3.72 Hz, GluN-**H**-1), 5.98 (ψt, J = 2.54 Hz, 1H, Ino-**H**-2, 7.0-7.1 (m, 4H, **Ph**CH₂O), 7.1-7.4 (m, 43.5H, **Ph**CH₂O (40H) +**CH**Cl₃)); {¹H}¹³C NMR (CDCl₃) δ 172.7, 138.6, 138.2, 137.9, 137.7, 136.1, 136, 135.9, 135.8, 128.8, 128.7, 128.64, 128.6, 128.52, 128.5, 128.4, 128.3, 128.2, 128.1, 128.0, 127.9, 127.8, 127.7, 97.6, 81.7, 80.8, 80.0, 78.6, 78.4, 77.8 (hidden by CHCl₃, extracted from HMQC), 76.5, 75.9, 75.4, 75.05, 74.5, 74.4, 73.6, 72.4, 70.7, 69.96, 69.88, 69.7, 69.64, 68.6, 68.1, 63.4, 34.5, 34.2, 29.9, 29.8, 29.6, 29.58, 29.3, 25.4, 22.9, 14.3; {¹H}³¹P NMR (CDCl₃) δ -0.23; LRMS m/z 1406.24 (M+H⁺), 1428.7 (M+Na⁺) calcd for C₈₄H₁₀₀N₃O₁₄P 1405.69; HRMS m/z 1428.6834 (M+Na⁺), calcd for C₈₄H₁₀₀N₃NaO₁₄P⁺ 1428.6835; error (ppm) -0.1.

### 2-Amino-3,4,6-tri-O-benzyl-2-deoxy-α-D-glucopyranosyl-(1→6)-3,4,5-tri-O-benzyl-1-O-(ph osphono)-2-palmitoyl-D-myo-inositol, lactam (86) and 2-azido-3,4,6-tri-O-benzyl-2-deoxy-α-D-glucopyranosyl-(1→6)-3,4,5-tri-O-benzyl-1-O-(dibe nzylphos-phono)-2-palmitoyl-D-myo-inositol (68).

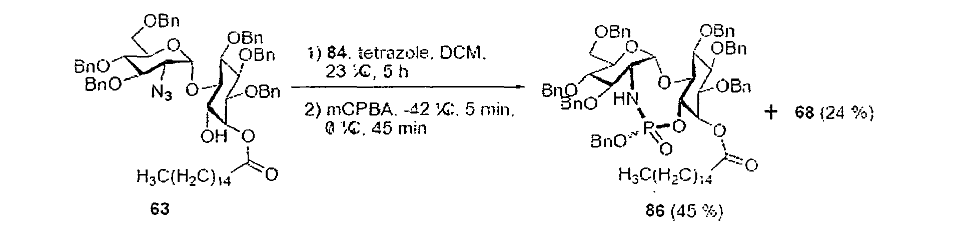

Compound **63** (10 mg, 8.72 µmol) and tetrazole (2.4 mg, 34.9 µmol, 4 equiv) were coevaporated together with toluene *in vacuo.* CH₂Cl₂ (0.5 mL) was added to the reactants under Ar followed by amidite **84** (87 µL of 0.2 M solution in CH₂Cl₂, 2 equiv). The reaction mixture was stirred at 23 °C for 1 h. TLC analysis (1:4 v/v EtOAc/Hex) showed the presents of starting material (*R_{f}*=0.24). Additional amount of phosphorylating reagent **84** (87 µL of 0.2 M solution in CH₂Cl₂, 2 equiv) was added and the reaction mixture was stirred for 1 h. TLC analysis (1:4 v/v EtOAc/Hex) showed that starting material is still present in the reaction mixture. Tetrazole (4 equiv) and **84** (4 equiv) were added, reaction mixture was stirred for another 30 min and TLC indicated absence of starting material. After another 1.5 h, the reaction mixture was chilled to -42 °C and mCPBA (1 mL. of 0.087 M solution in CH₂Cl₂, 12 equiv) was added. The reaction mixture was stirred for 5 min at -42 °C and 45 min at 0 °C. Dichloromethane (1 mL) was added and the reaction mixture was quenched with Na₂S₂O₃ (1 mL of 1 M solution) at 0 °C. Organic layer was separated and washed successively with 1M Na₂S₂O₃ (3 x 0.5 mL), water (1 x 0.5 mL), 5% NaHCO₃ (3 x 0.5 mL), water (1 x 0.5 mL) and brine (2 x 1 mL). The organic phase was dried over Na₂SO₄ and evaporated. The residue was separated by PTLC giving 5 mg (45%) of lactam **86** and 3 mg (24%) of **68**. For **86**: *R_{f}*=0.66 (2 : 3 v/v EtOAc/Hex); ¹H NMR (CDCl₃) δ 0.88 (m, 3.4H, CH₂CH₂(CH₂)₁₂**CH**₃ (3H)), 1.12-1.35 (m, 27.4H, CH₂CH₂(**CH₂**)**₁₂**CH₃ (24H)), 1.5-1.7 (m, 14.62H, CH₂**CH₂**(CH₂)₁₂CH₃ (2H), "H₂O"), 2.34 (m, 2H, **CH₂**CH₂(CH₂)₁₂CH₃, 3.2 (m, 1H, GluN-**H**-2), 3.22 (dd, J = 10.74, 1.76 Hz, 1H, GluN-**H**-6), 3.33 (dd, ²J_{H-P} = 7.7 Hz, ³J_{NH-GluN-**H**-2} = 2.89 Hz, 1H, NH), 3.49 (ψt, J = 9.15, 1H, Ino-**H**-5), 3.53-3.61 (m, 2H, Ino-**H**-3, GluN-**H**-6'), 3.72 (ψt, J = 9.1 Hz, 1H, GluN-**H**-4), 3.79-3.87 (m, 2H, GluN-**H**-5, Ino-**H**-4), 3.97 (ψt, J = 9.86 Hz, 1H, GluN-**H**-3), 4.03 (ψt, J = 9.51 Hz, 1H, Ino-**H**-6), 4.3 (d, J = 12.11 Hz, 1H, Ph**CH₂**O), 4.36 (ψtd, ³J_{H-P} = 9.73 Hz, ³J_{Ino-H-6-Ino-H-1} = 9.73 Hz, ³J_{Ino-H-2-Ino-H-1} = 2.67 Hz), 4.45-4.55 (m, 3H, Ph**CH₂**O), 4.67-4.88 (m, 7H, Ph**CH₂**O), 4.96 (dd, ³J_{H-P} = 7.69 Hz, ²J_{H-H} = 11.74 Hz, 1H, Ph**CH**₂O - P), 4.98 (d, J = 9.3 Hz, 1H, Ph**CH**₂O), 5.06 (dd, 3JH-P = 6.78 Hz, 2JH-H = 11.78 Hz, 1H, Ph**CH**₂O - P), 5.33 (d, J = 3.71 Hz, 1H, GluN-**H**-1), 5.87 (ψt, J = 2.57 Hz, 1H, Ino-**H**-2), 7.07-7.17 (m, 2H, **Ph**CH₂O), 7.2 -7.4 (m, 41H, **Ph**CH₂O (33H) + C**H**Cl₃); {¹H}³¹P NMR (CDCl₃) δ 7.91; LRMS m/z 1272.68 (M+H⁺), 1294.72 (M+Na⁺) calcd for C₇₇H₉₄NO₁₃P 1271.65.

### Synthesis of IG-1 (13):

### 2-Amino-2-deoxy-α-D-glucopyranosyl-(1→6)-2-palmitoyl-D-myo-inositol (13).

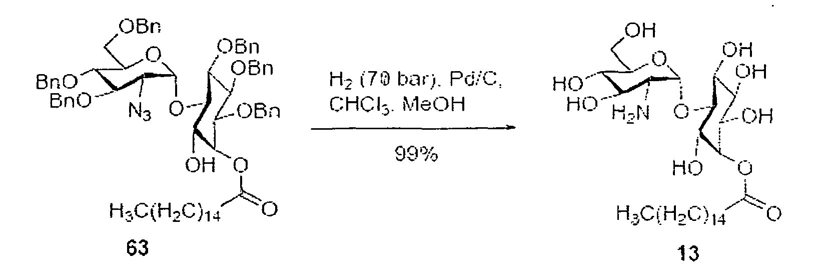

Compound **63** (6 mg, 5.24 µmol) was dissolved in 1 mL of chloroform and 1 mL of methanol. 6 Mg of 10% Pd/C were added. The reaction mixture was stirred for 4 h at 70 bar of H₂, after that 3 mg of 10 % Pd/C were added and the reaction mixture was stirred under the same hydrogen pressure for 4 h. The slurry was filtered through Celite pad (5 mm height), the pad was rinsed (4 x 0.5 mL of methanol-chloroform mixture, 5:1, v/v) and the solution was evaporated giving 3.5 mg of pure **13** (quant.). 1H NMR (*d-*4-MeOH) δ 0.9 (m, 3H, CH₂CH₂(CH₂)₁₂**CH**₃), 1.21-1.45 (m, 26H, CH₂CH₂(**CH**₂)₁₂CH₃ (24H), grease), 1.58- 1.7 (m, 2H, CH₂**CH**₂(CH₂)₁₂CH₃), 2.35-2.5 (m, 2H, **CH**₂CH₂(CH₂)₁₂CH₃, 3.1 (dd, J = 10.58, 3.67 Hz, 1H, GluN-**H**-2), 3.27-3.37 (m, 3.6H, Ino-**H** (1H), C**H**D₂OD), 4.43 (ψt, J = 9.43, 1H, GluN-**H**-4), 3.48-3.61 (m, 2H, Tno-**H** (2H)), 3.63-3.86 (m, 5H, Ino-**H** (2H), GluN-**H**-3, GluN-**H**-6, GluN-**H**-6'), 4.08 (ddd, J = 2.48, 4.21, 9.71 Hz, 1H, GluN-**H**-5), 5.33 (d, J = 3.67 Hz, 1H, GluN-**H**-1), 5.42 (ψt, J = 2.48 Hz, 1H, Ino-**H**-2); {1H}13C NMR (*d-*4-MeOH) δ 175.3, 98.1, 82.3, 75.8, 75.0, 74.7, 74.1, 72.1, 71.6, 71.5, 62.0, 56.5, 35.3, 33.2, 31.0, 30.8, 30.71, 30.7, 30.4, 26.1, 24.0, 14.6; LRMS m/z 580.36 (M+H⁺), 602.45 (M+Na⁺) calcd for C₂₈H₅₃NO₁₁ 579.36.

### Synthesis of IG-2 (14):

### 2-Amino-2-deoxy-α-D-glucopyranosyl-(1→6)-2-palmitoyl-D-myo-inositol-1-phosphate (14).

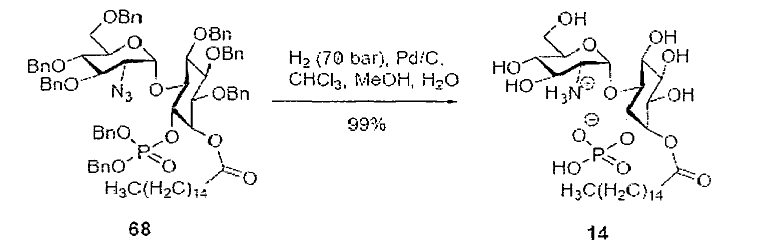

Compound **68** (5.4 mg, 3.84 µmol) was dissolved in 1 mL of chloroform, 1 mL of methanol and 0.3 mL of H2O. 6 Mg of 10% Pd/C were added and the reaction mixture was stirred for 5 h at 70 bar of H₂. The slurry was filtered through Celite pad (5 mm height), the pad was rinsed (4 x 0.5 mL of methanol-chloroform-water mixture, 3:3:1, v/v) and the solution was evaporated giving 2.7 mg of **14** (quant.). ¹H NMR (*d-*4-MeOH) δ 0.9 (m, 4H, CH₂CH₂(CH₂)₁₂**CH**₃ (3H)), 1.21-1.45 (m, 31H, CH₂CH₂(**CH₂**)**₁₂**CH₃ (24H)), 1.58-1.2 (m, 2H, CH₂**CH**₂(CH₂)₁₂CH₃), 3.4 (ψt, J = 6.76 Hz, 2H, **CH₂**CH₂(CH₂)₁₂CH₃, 3.11 (dd, J = 10.31, 3.51 Hz, 1H, GluN-**H**-2), 3.27-3.47 (m, 5.59H, Ino-**H**-6, GluN-**H**-4, CHD2OD), 3.51-3.66 (m, 2H, Ino-**H**-3, Ino-**H**-4), 3.7-3.88 (m, 3H, GluN-**H**-6, GluN**H**-6', GluN-**H**-3), 3.94 (ψt, **J** = 9.23, 1H, Ino-**H**-6), 4.16 (m, 1**H**, GluN-**H**-5), 4.13 (ψt, J = 8.97 Hz, 1H, Ino-**H**-1), 5.53 (d, J = 3.81 Hz, 1H, GluN-**H**-1), 5.6 (br. s., 1H, Ino-**H**-2); {¹H} ¹³C NMR (*d-*4-MeOH) δ 174.8, 96.6, 79.2, 76.7, 74.9, 74.8, 74.6, 74.0, 72.4, 72.1, 71.6, 71.2, 62.1, 55.8, 35.4, 33.2, 30.9, 30.8, 30.6, 30.4, 30.0, 26.2, 23.9, 14.6; {¹H}³¹P NMR (*d-*4-MeOH) δ 1.26; LRMS m/z 658.50 (M-H⁺), calcd for C₂₈H₅₄NO₁₄P 659.3284.

### 2-Azido-3,4,6-tri-O-benzyl-2-deoxy-D-glucopyranosyl-(1→6)-3,4,5-tri-O-benzyl-1,2-didehy dro-1,2-dideoxy-D-myo-inositol (73 α/β) and 2-azido-3,4,6-tri-O-benzyl-1-fluoro-1,2-dideoxy-α-D-glucopyranose (87).

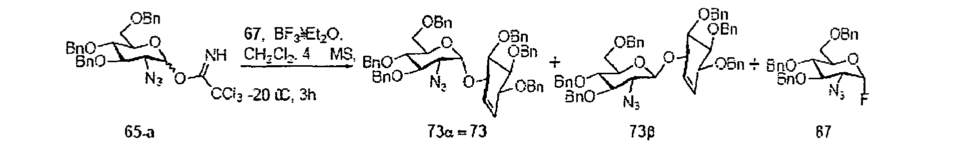

Anhydrous CH₂Cl₂ (1 mL) was added to a mixture of donor **65a** (16.5 mg, 26.7 µmol), acceptor **67** (13 mg, 32 µmol) and crushed activated molecular sieves (150 mg) under Ar. The reaction mixture was chilled to -20 °C, BF₃ · Et₂O (75 µL of 0.05 M solution in CH₂Cl₂, 0.15 equiv) was introduced dropwise and the reaction mixture was stirred at -20 °C for 2h. Additional amount BF₃·Et₂O (300 µL of 0.05 M solution in CH₂Cl₂, 0.5 equiv) was introduced dropwise and the reaction mixture was stirred at -20 °C for additional 1h and then allowed to warm up to 20 °C. Solid NaHCO₃ (100 mg) was added, the reaction mixture was stirred for 15 minutes and filtered. The filtrate was washed successively with cold 5% aq Na₂CO₃ (3 x 0.5 mL), water (2 x 0.5 mL) and with brine (2 x 0.5 mL), dried over Na₂SO₄ and evaporated. Crude reaction mixture was separated by flash chromatography (1:6 → 1:4.5 → 1:3 v/v EtOAc/Hex) giving **87** (3 mg 24%), **73**β (8 mg 34%) and 73α(4 mg 17%). For **87**: *R_{f}*=0.66 (1:4 v/v EtOAc/Hex); ¹H NMR (CDCl₃) δ 3.49 (ddd, ³J_{H2-F} = 26 Hz, ³J_{H2-H3} = 10.08 Hz, ³**J_{H2-H1}** = 2.6 Hz), 1H, **H**-2), 3.68 (dd, J = 10.98, 1.85 Hz, 1H, **H**-6), 3.75-3.87 (m, 2H, **H**-4, **H**-6'), 3.91-4.01 (m, 2H, **H**-3, **H**-5), 4.5 (d, J = 12.47 Hz, 1H, PhCH₂O), 4.55 (d, J = 10.9 Hz, 1H, Ph**CH₂**O), 4.63 (d, J = 12.08 Hz, 1H, Ph**CH₂**O), 4.82 (d, J = 12.47 Hz, 1H, Ph**CH₂**O), 4.85 (d, J = 10.84 Hz, 1H, Ph**CH₂**O), 4.91 (d, J = 10.69 Hz, 1H, Ph**CH**₂O), 5.67 (dd, ²J_{H1-F}= 52.8 Hz, ³J_{H2-H1} = 2.67 Hz, 1H, **H**-1), 7.15-7.21 (m, 2H, **Ph**CH₂O), 7.28-7.4 (m, 13H, **Ph**CH₂O); {¹H}¹³C NMR (CDCl₃) δ 138, 137.9, 137.8, 128.7, 128.69, 128.3, 128.2, 128.1, 128.08, 128.0, 107.9, 104.9, 78.4, 75.9, 75.4, 73.8, 73.5, 73.4, 67.9, 63.9, 63.6; ¹⁹F NMR (CDCl₃) δ -146.4 (dd, J = 52.7, 25.8 Hz). For 73β: *R_{f}*=0.61 (1:4 v/v EtOAc/Hex); ¹H NMR (CDCl₃) δ 3.3 - 3.49 (m, 3H, GluN-H-2, GluN-H-3, GluN-H-5), 3.59-3.82 (m, 5H, GluN-H-4, GluN-H-6, GluN-H-6', Ino-H (2H)), 4.25 (m, 1H, Ino-H), 4.45 (m, 1H, Ino-H), 4.47-4.62 (m, 3H, PhCH₂O), 4.64 (d, J = 7.6 Hz, 1H, GluN-**H**-1), 4.65 - 4.75 (m, 2H, Ph**CH₂**O), 4.75-4.92 (m, 6H, Ph**CH₂**O), 4.97 (d, **J** = 10.16 Hz, 1H, Ph**CH₂**O), 5.68-5.8 (m, 2H, Ino-**H** (2H)), 7.15-7.21 (m, 2H, **Ph**CH₂O), 7.21-7.41 (m, 33.3H, **Ph**CH₂O (28H) + **CH**Cl₃); {¹H}¹³C NMR (CDCl₃) δ 139.0, 138.9, 138.6, 138.4, 138.3, 138.2, 129.3, 128.9, 128.9, 128.8, 128.8, 128.6, 128.5, 128.4, 128.3, 128.3, 128.3, 128.2, 128.0, 102.9, 84.3, 84.1, 83.6, 80.5, 80.2, 78.2, 76.0, 75.5, 75.3, 73.9, 72.9, 68.8, 67.0. For 73α: *R_{f}*=0.53 (1:4 v/v EtOAc/Hex); 1H NMR (CDCl₃) δ 3.34 (dd, J = 10.9, 1.9 Hz, 1H, GluN-**H**-6), 3.4 (d, J = 11.1, 2.7, 1H, GluN-**H**-6'), 3.45 (dd, J = 10.5, 3.5 Hz, 1H, GluN-**H**-2), 3.65-3.79 (m, 3H, GluN-**H**-4, Ino-**H** (2H)), 3.96 (dd, J = 10.1, 9 Hz, I H, GluN-**H**-3), 4.02 (m, 1H, GluN-**H**-5), 4.25 (m, 1H, Ino-**H**), 4.3 (d, J = 11.9 Hz, 1H, Ph**CH₂**O), 4.4 (m, 1H, Ino-**H**), 4.45 (d, J = 11 Hz, 1H, Ph**CH₂**O), 4.55 (d, J = 12 Hz, 1H, PhCH₂O), 4.63-4.75 (m, 3H, Ph**CH₂**O), 4.77 (d, J = 11 Hz, 1H, PhCH₂O), 4.82-5.0 (m, 5H, Ph**CH₂**O), 5.1 (d, J = 3.6 Hz, 1H, GluN-**H**-1), 5.7-5.83 (m, 2H, Ino-**H** (2H)), 7.1-7.45 (m, 33.3H, **Ph**CH₂O (30H) + **CH**Cl₃); {¹H} ¹³C NMR (CDCl₃) 138.7, 138.4, 138.3, 138.3, 138.0, 137.9, 128.9, 128.7, 128.6, 128.5, 128.5, 128.3, 128.2, 128.1, 128.1, 128.0, 127.9, 127.8, 127.8, 127.7, 125.3, 95.2, 84.1, 82.3, 80.6, 80.4, 78.3, 76.1, 76.0, 78.5, 75.6, 75.1, 73.6, 72.7, 70.8, 67.9, 63.4.

### 2-Azido-3,4,6-tri-O-benzyl-2-deoxy-D-glucopyranosyl-(1→6)-3,4,5-tri-O-benzyl-1,2-didehy dro-1,2-dideoxy-D-myo-inositol (73 α/β)

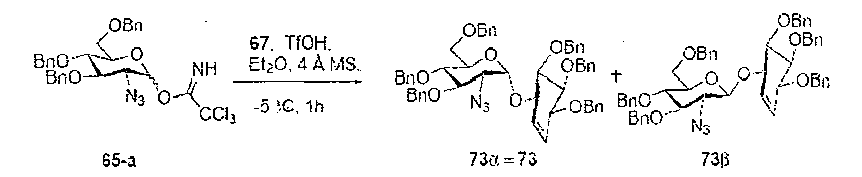

Anhydrous ether (6 mL) was added to a mixture of donor **65a** (90 mg, 145 µmol), acceptor **67** (70 mg, 168 µmol) and 4Å crushed activated molecular sieves (100 mg) under Ar. The reaction mixture was chilled to -5 °C, TfOH (232 µL of 0.0626 M solution in Et₂O, 0.1 equiv) was introduced dropwise and the reaction mixture was stirred at -5 °C for 1h and then allowed to warm up to 20 °C. Solid NaHCO₃ (100 mg) was added, the reaction mixture was stirred for 15 min and filtered. The filtrate was washed successively with cold 5% aq Na₂CO₃ (3 0.5 mL), water (2 0.5 mL) and with brine (2 0.5 mL), dried over NaSO₄ and evaporated. The crude reaction mixture was separated by flash chromatography (1:10 → 1:7 → 1:6 → 1:5 → 1:3 v/v EtOAc/Heptane) giving fractions with pure coupling products. Fractions with impure **73**α and **73**β were combined separately and evaporated. The products were separated by PTLC (1:3 v/v EtOAc/Heptane) and combined with previous fractions providing 74 mg (58%) of **73**α and 26.5 mg (21 %) of **73**β.

**2-Azido-3,4,6-tri-*O-*benzyl-2-deoxy-α-D-glucopyranosyl-(1→6)-3,4,5-tri-*O-*benzyl-2-palmito yl-D-*myo*-inositol (63), 2-azido-3,4,6-tri-*O-*benzyl-2-deoxy-α-D-glucopyrano-syl-(1→6)-3,4,5-tri-*O-*benzyl-1-palmit oyl-D-*myo*-inositol (83), 2-azido-3,4,6-tri-*O-*ben-zyl-2-deoxy-α-D-glucopyranosyl-(1→3)-3,4,5-tri-*O-*benzyl-2-palmit oyl-D-*myo*-inosi-tol (89) and 2-azido-3,4,6-tri-*O-*benyl-2-deoxy-α-D-glucopyranosyl-(1→6)-3,4,5-tri-*O-*benzyl-1-palmitoy 1-D-*myo*-inositol (90).**

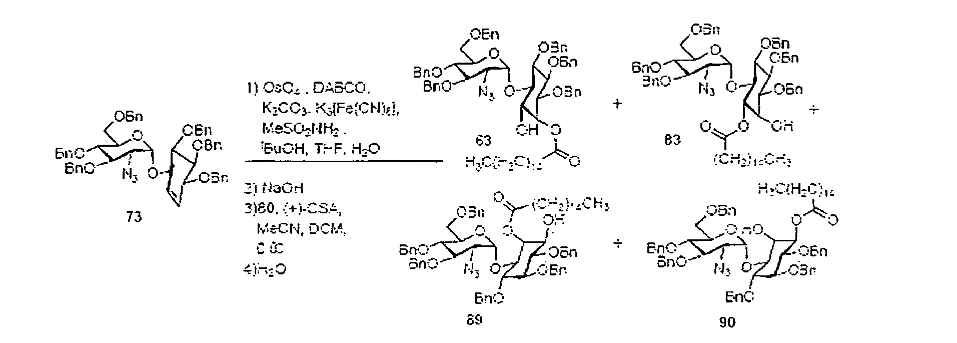

Alkene **73** (44.4 mg, 50.8 µmol) was dissolved in a mixture of THF (0.7 mL) and *^{t}*BuOH (0.8 mL) and the solution was transfered to a 25 mL pear shaped flask. Water (500 µL) was added followed by K₃[Fe(CN)₆] (50 mg, 152 *µ*mol, 3 equiv), K₂CO₃ (152 µL of 1 M aq solution, 3 equiv), DABCO (170 µL of 0.089 M aq solution, 15.2 µmol, 0.3 equiv), OsO₄ (90 µL of 0.056 M aq solution, 0.1 equiv) at vigorous stirring. Finally, a solution of MeSO₂NM₂ (9.6 mg, 102 µmol in 0.5 mL of H₂O) was added. The reaction mixture was vigorously stirred at 23 °C and monitored by TLC (3 : 4 v/v EtOAc/Heptane, for **73** R*_{f}* =0.76, for **64** and **88** R*_{f}* =0.36-0.42). TLC indicated ca. 60-70 % conversion after for 12 h. NaOH (76 µL of 1m aq solution) was added with stirring. The reaction mixture was stirred for another 8 h and the reaction was quenched by addition of Na₂SO₃. A color change from yellow to colorless (organic phase) and black (aq phase) was observed. The reaction mixture was vigorously stirred for 15 min, and the organic layer was separated. The aqueous layer was extracted with toluene (3 x 0.5 mL) and the combined organic liquids were washed successively with 1M NaOH (2 x 1mL, water (2 x 0.5 mL), IM Na₂S₂O₃ (3 x 0.5 mL), water (1 x 0.5 mL) and brine (2 x 1 mL). The organic phase was dried over Na₂SO₄ and evaporated to give 44 mg of a mixture of dioles **64** and **88.** The mixture of dioles (68 mg, 74.9 µmol) was dissolved in CH₂Cl₂ (400 µL) under Ar. Trimethyl orthopalmitate **(80,** 1.6 mL of 0.114 M solution in MeCN, 2.5 equiv) was added with stirring. The reaction mixture was chilled to 0 °C and (+)-CSA (443 µL of 0.0422 M solution in MeCN, 0.25 equiv) was added. The reaction mixture was stirred for 25 min at 0 °C and a solution of 2 mg of (+)-CSA in a mixture of 500 µL MeCN, 100 µL CH₂Cl₂ and 30 µL H₂O (142 µL, corresponds to 5 equiv of H₂O) is added. The reaction mixture is stirred for 30 min at 0 °C and quenched with Et₃N (0.1 mL). Volatile materials were evaporated *in vacuo.* Products were separated by flash chromatography (96:4 → 14:1 → 8:1 → 7:1 v/v PhH/EtOAc) providing three fractions. Fraction A: a mixture of **90, 83** with 63, fraction B: pure 63, fraction C: pure **89** (30 mg). Isomers from fraction A were separated by PTLC (1:3 v!v EtOAc/Hex) providing **90** (6 mg), **83** (8 mg) and 63 (28 mg). Combined yield of palmitates was 85%, ratios **90/89** = 1/4.9, **83/63** = 1/3.5. For 90: R*_{f}*=0.57 (1 : 14 v/v EtOAc/PhH); ¹H NMR (CDCl₃) δ 0.88 (m, 3.3H, CH₂CH₂(CH₂)₁₂C**H**₃), 1.15-1.37 (m, 28.3H, CH₂CH₂(CH₂)₁₂CH₃ (24H)), 1.52-1.65 (m, 7.6H, CH₂CH₂(CH₂)₁₂CH₃ (2H), "**H**₂O" (5.6H)), 2.2-2.43 (m, 2H, **CH**₂CH₂(CH₂)₁₂CH₃, 2.96 (br. s 1**H**, O**H**), 2.9 (dd, J = 11.26, 1.77 Hz, 1H, GluN-**H**-6), 3.37 (dd, J = 11.22, 2.61 Hz, 1H, GluN-**H**-6'), 3.53 (ψt, J= 9.43 Hz, 1H, Ino-**H**-5), 3.58 (dd, 1H, J=9.99 Hz, 1H, GluN-**H**-2), 3.7 (dd, J = 9.42, 2.58 Hz, 1H, Ino-**H**-3), 3.75 (ψt, J = 9.25 Hz, GluN-**H**-4), 3.86-3.99 (m, 3H, Ino-**H**-4, GluN-**H**-3, GluN-**H**-5), 4.1 (ψt, J = 9.87 Hz, 1H, Ino-**H**-6), 4.24 (m, 1H, Ino-**H**-2), 4.31 (d, J = 12.01 Hz, 1H, PhC**H**₂O), 4.44-4.54 (m, 2H, PhC**H**₂O), 4.67 (d, J = 10.62 Hz, 1H, PhC**H**₂O), 4.72-4.79 (m, 2**H**, PhC**H**₂O), 4.8-4.97 (m, 8H, PHC**H**₂O (6H), Ino-**H**-1, GluN-**H**-1), 7.08-7.2 (m, 5H, **Ph**CH₂O), 7.2-7.4 (m, 33H, **Ph**CH₂O (25H) + C**H**Cl₃); {¹H} ¹³C NMR (CDCl₃) δ 173.5, 138.7, 138.6, 138.5, 138.3, 138.1, 137.9, 128.7, 128.6, 128.55, 128.4, 128.1, 128.12, 128.0, 127.9, 127.8, 127.7, 94.4, 83.5, 81.1, 79.8, 79.3, 78.4, 78.0, 76.3, 76.1, 75.8, 75.75, 75.0, 73.6, 72.8, 71.4, 67.9, 66.5, 64.0, 34.5, 32.1, 29.91, 29.9, 29.7, 29.6, 29.5, 29.4, 25.0, 22.9, 14.3. For **89**: R*_{f}*=0.08 (1 : 14 v/v EtOAc/PhH); ¹H NMR (CDC₃) δ 0.87 (m, 3.2H, CH₂CH₂(CH₂)₁₂**CH**₃ (3**H**)), 1.17-1.43 (m, 27H, CH₂CH₂(CH₂)₁₂CH₃ (24H)), 1.62-1.73 (m, 2H, CH₂C**H**₂(CH₂)CH₃), 2.17 (d, J = 2.89 Hz, 1H, OR), 2.35-2.52 (m, 2H, CH₂CH₂(CH₂)₁₂CH₃, 3.32 (dd, J = 10.24, 3.6 Hz, 1H, GluN-**H**-2), 3.44-3.83 (m, 7H, Ino-**H**-1, Ino-**H**-3, Ino-**H**-5, Ino-**H**-6, GluN-**H**-4, GluN-**H**-6, GluN-**H**-6'), 3.87-4.08 (m, 3H, Ino-**H**-4, GluN-**H**-3, GluN-**H**-5), 4.38 (d, J = 12.06 Hz, 1H, PhC**H**₂O), 4.47 (d, J = 10.99 Hz, 1H, PhC**H**₂O), 4.58 (d, J = 12.05 Hz, 1H, PhC**H**₂O), 4.7-4.9 (m, 8H, PhC**H**₂O), 4.95 (d, 1H, PhC**H**₂O), 5.22 (d, J = 3.72 Hz, 1H, GIuN-**H**-1), 5.71 1 (ψt, J = 2.64 Hz, 1H, Ino-**H**-2), 7.0-7.17 (m, 5H, **Ph**CH₂O), 7.2-7.37 (m, 30H, **PH**CH₂O (25H) + C**H**Cl₃); {¹H} ¹³C NMR (CDCl₃) δ 173.6, 138.5, 138.4, 138.0, 137.9, 128.9, 128.7, 128.65, 128.6, 128.5, 128.48, 128.3, 128.25, 128.2, 128.1, 127.94, 127.9, 127.8, 93.9, 83.6, 81.61, 81.23, 80.1, 78.3, 76.1, 75.8, 75.4, 75.1, 73.4, 72.7, 70.7, 70.4, 68.2, 67.1, 63.0, 34.36, 32.14, 29.9, 29.87, 29.81, 29.62, 29.6, 29.36, 25.1, 22.9, 14.4.

### Synthesis of IG-13:

### 2-Amino-2-deoxy-α-D-glucopyranosyl-(1→6)-1-palmitoyl-D-myo-inositol (91).

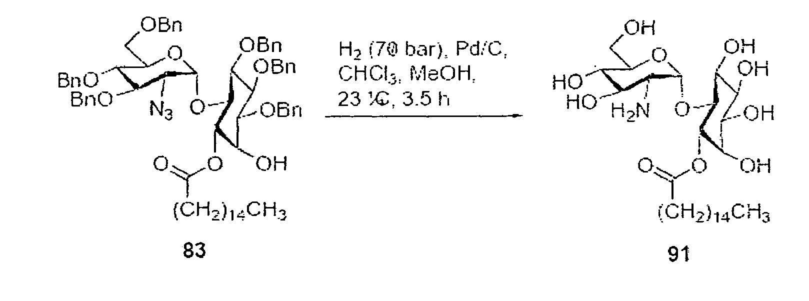

Compound **83** (3 mg, 2.62 µmol) was dissolved in 0.7 mL of chloroform and 0.7 mL of methanol. 4 Mg of 10% Pd/C were added. The reaction mixture was stirred for 3.5 h at 70 bar of H₂. The slurry was filtered through Celite pad (5 mm height), the pad was rinsed (4 x 0.5 mL of methanol-chloroform mixture, 5:1, v/v) and the solution was evaporated giving 1.5 mg of pure 91 (quant.). ¹H NMR (*d-*4-MeOH) δ 0.9 (m, 3.8H, CH₂CH₂(CH₂)₁₂**CH**₃ (3H), grease), 1.21-1.45 (m, 31H, CH₂CH₂(C**H**₂)₁₂CH₃ (24H), **grease**), 1.58-1.72 (m, 2H, CH₂CH₂(CH₂)₁₂CH₃), 2.3-2.52 (m, 2H, **CH**₂CH₂(CH₂)₁₂CH₃, 3.13 (dd, J = 10.71, 3.84 Hz, GluN-**H**-2), 3.23-3.49 (m, 3H, Ino-**H**-3, Ino-**H**-5, GluN-**H**-4), 3.63-3.85 (m, 4H, Ino-**4**, GluN-**H**-3, GluN-**H**-6, GluN-**H**-6'), 4.0-4.1 (m, 2H, Ino-**H**-6, GluN-**H**-5), 4.19 (ψt, J = 2.55 Hz, 1H, Ino-**H**-2), 4.71 (dd, J = 10.22, 2.58 Hz, 1H, Ino-**H**-1), 5.26 (d, J = 3.76 Hz, 1H, GluN-**H**-1); {¹H}¹³C NMR (*d-*4-MeOH) δ 175.0, 97.0, 79.5, 76.4, 74.8, 74.5, 74.3, 72.6, 71.6, 71.2, 71.1, 61.9, 56.3, 35.3, 33.2, 30.9, 30.8, 30.7, 30.6, 30.5, 25.9, 23.9, 14.6; LRMS m/z 580.34 (M+H⁺), 602.44 (M+Na⁺) calcd for C₂₈H₅₃NO₁₁ 579.36.

### Synthesis of IG-14:

### 2-Amino- 2-deoxy-α-D-glucopyranosyl-(1→3)-2-palmitoyl-D-myo-inositol (92).

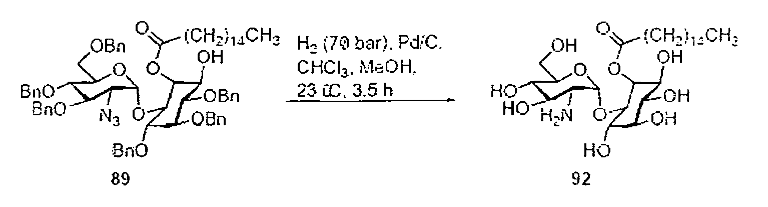

Compound **89** (3 mg, 2.62 µmol) was dissolved in 0.7 mL of chloroform and 0.7 mL of methanol. 4 Mg of 10% Pd/C were added. The reaction mixture was stirred for 3.5 h at 70 bar of H₂. The slurry was filtered through Celite pad (5 mm height), the pad was rinsed (4 x 0.5 mL of methanol-chloroform mixture, 5:1, v/v) and the solution was evaporated giving 1.5 mg of pure **92** (quant.). ¹H NMR (*d-*4-MeOH) δ 0.9 (m, 3.9H, CH₂CH₂(CH₂)₁₂**CH**₃ (3H), grease), 1.21-1.44 (m, 31H, CH₂CH₂(C**H**₂)₁₂CH₃ (24H), grease), 1.58-1.72 (m, 2H, CH₂C**H**₂(C**H**₂)₁₂CH₃), 2.35-2.52 (m, 2H, **CH**₂CH₂(CH₂)₁₂CH₃, 3.13 (dd, J = 10.67, 3.58 Hz, 1H, GluN-**H**-2), 3.25 (ψt, J = 9.01 Hz, 1H, Ino-**H**), 3.38 (m, 1H, Ino-**H**), 3.54 (dd, J = 9.91, 2.86 Hz, 1H, Ino-**H**), 3.58-3.72 (m, 3H, Ino-**H** (2H), GluN-**H**-6), 3.75, 3.87 (m, 3H, Ino-**H**, GluN-H-3, GluN-**H**-6'), 4.2 (ddd, J = 2.2, 5.13, 10.16 Hz, 1H, GluN-**H**-5), 5.38 (d, J = 3.55 Hz, 1H, GluN-**H**-1), 5.45 (ψt, J=2.32 Hz, 1H, Ino-**H**-2); {¹H}¹³C NMR (*d-*4-MeOH) δ 177, 94.6, 76.8, 76.7, 74.7, 74.3, 73.1, 72.9, 71.8, 71.4, 71.2, 62.2, 55.9, 35.5, 33.2, 30.9, 30.8, 30.7, 30.6, 30.5, 26.1, 23.9, 14.6; LRMS m/z 580.30 (M+H⁺), 602.34 (M+Na⁺) calcd for C₂₈H₅₃NO₁₁ 579.36.

### Synthesis of IG-15:

### 2-Amino-2-deoxy-α-D-glucopyranosyl-(1→3)-1-palmitoyl-D-myo-inositol (93).

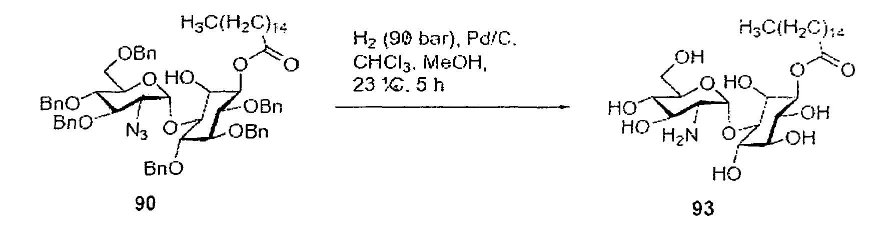

Compound **90** (3 mg, 2.62 µmol) was dissolved in 0.7 mL of chloroform and 0.7 mL of methanol. 4 Mg of 10% Pd/C were added. The reaction mixture was stirred for 5.5 h at 90 bar of H₂. The slurry was filtered through Celite pad (5 mm height), the pad was rinsed (4 x 0.5 mL of methanol-chloroform mixture, 5:1, v/v) and the solution was evaporated giving 1.5 mg of pure **92** (quant.). ¹H NMR (*d-*4-MeOH) δ 0.9 (m, 3.5H, CH₂CH₂(CH₂)₁₂**CH₃** (3H), grease), 1.21-1.4 (m, 30H, CH₂CH₂(C**H**₂)₁₂CH₃ (24H), grease), 1.58-1.7 (m, 2H, CH₂**CH**₂(CH₂)₁₂CH₃), 2.38-2.5 (m, 2H, **CH**₂CH₂(CH₂)₁₂CH₃, 3.16 (dd, J = 10.52, 3.62 Hz, 1H, GluN-**H**-2), 3.28 (ψt; J = 9.06, 1H, Ino-**H**-5), 3.4 (dd, J = 9.98, 8.94 Hz, 1H, GluN-**H**-4), 3.56-3.89 (m, 6H, Ino-**H**-3, Ino-**H-4,** Ino-**H**-6, GluN-**H**-3, GluN-**H**-6, GluN-**H**-6'), 4.04 (ddd, J = 9.59, 5.21, 2.5 Hz, 1H, GluN-**H**-5), 4.18 (ψt, J = 2.45 Hz, 1H, Ino-**H**-2), 4.65 (dd, J = 10.25,2.5 Hz, 1H, Ino-**H**-1), 5.18 (d. J = 3.62 Hz, 1H, GluN-**H**-1); {¹H}¹³*C* NMR (*d-*4-MeOH) δ 175.2, 93.8, 77.1, 76.6, 75.4, 74.4, 72.5, 71.8, 71.6, 71.4, 68.0, 62.1, 55.9, 35.1, 33.2, 31.0, 30.9, 30-8, 30.6, 30.4, 26.1, 23.9, 14.58; LRMS m/z 580.45 (M+H+), 602.45 (M+Na+) calcd for C₂₈H₅₃NO₁₁ 579.36.

### II. Lipophilic Inositol Glycan Compounds Reduce Cancer Cell Proliferation and Lengthen the Survival Times of Cancer Subjects

The following Example demonstrates that the lipophilic inositol glycan compounds of the present invention can induce cancer cell death and improve the survival of mice bearing cancer.

### Cell lines

Human pancreatic cancer cell lines (Panc-1, MiaPaca2, BXPC3, Aspc1), human hepatoma cell line (HuH7), human gastric cancer cell lines (AGS, MKN45), human colon cancer cell line (CoCM-1), human mammary cancer cell line (MCF-7), human neuroblastoma (SH-SY5Y), human prostate cancer cells (PC3), and murine Ehrlich ascites cancer cells and mouse 3T3-LI fibroblasts were obtained from American Type Culture Collection (Rockville, MD, USA). The cancer cells were cultured in RPMI 1640 containing 10% fetal calf serum. Cultures were maintained at 37 °C in a humidified atmosphere of 5% CO₂.

Human primary culture cells, dermal fibroblasts, epidermal keratinocytes and umbilical vein endothelial cells were purchased from Cell Applications (San Diego, CA, USA) and cultured according to the manufacturer's instructions.

### Cell proliferation analysis

Cell proliferation assays were performed with the CellTiter 96 AQueous One Solution Cell Proliferation Assay kit according to the manufacturer's instructions (Promega, Madison, WI, USA). Briefly, 5 × 10³ cells/well in medium were placed onto 96-well plates. IGs were added to cellular cultures at final concentrations of 0, 1, 10, and 100 µM. Cultures were incubated for 3 days or 7 days and then added to 20 µL/well of CellTiter 96 AQueous One Solution Reagent, which contains a tetrazolium compound, 3-(4,5-dimethylthiazol-2-yl)-5-(3-carboxymethoxyphenyl)-2-(4-sulfophenyl)-2H- tetrazolium, an inner salt; MTS was added to the culture medium. After incubating the plate for 1 h in a CO₂ incubator, absorbance at 490 nm was recorded with a 96-well plate reader.

### Animal survival analysis

Male ddy mice weighing 20-30g obtained from and maintained on a standard diet and water *ad libitum.* Ehrlich ascites cancer cells (5X10⁶ cells) were injected intraperitoneal to the mice. After 7 days later, the mice were subjected to daily intraperitoneal injection of IG1 or IG1 plus alkyl-acyl-phosphatidic acids (AAPAs) for additional 7 days. The endpoint of experiment was determined by spontaneous death of animals.

### Cancer Study Results

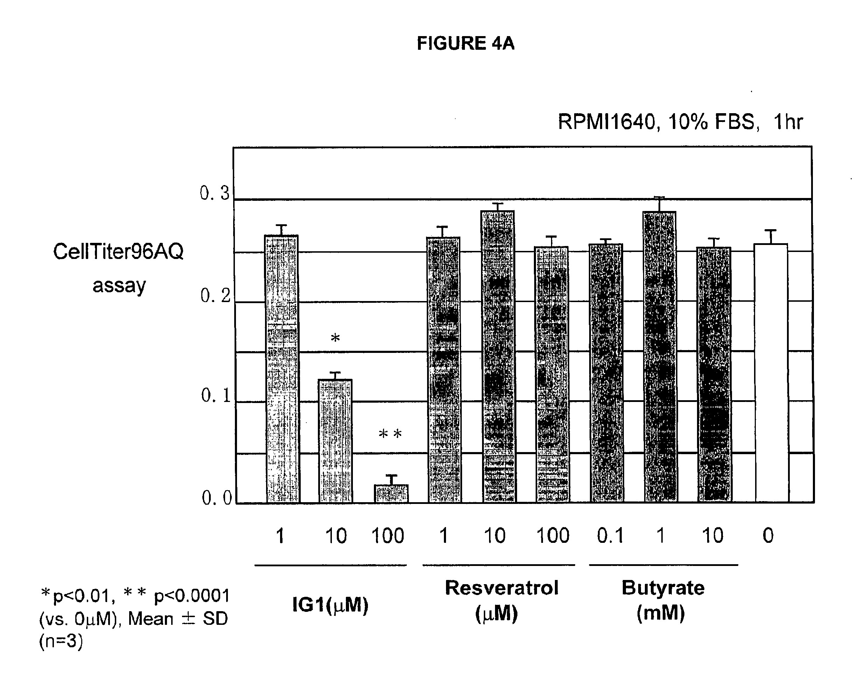

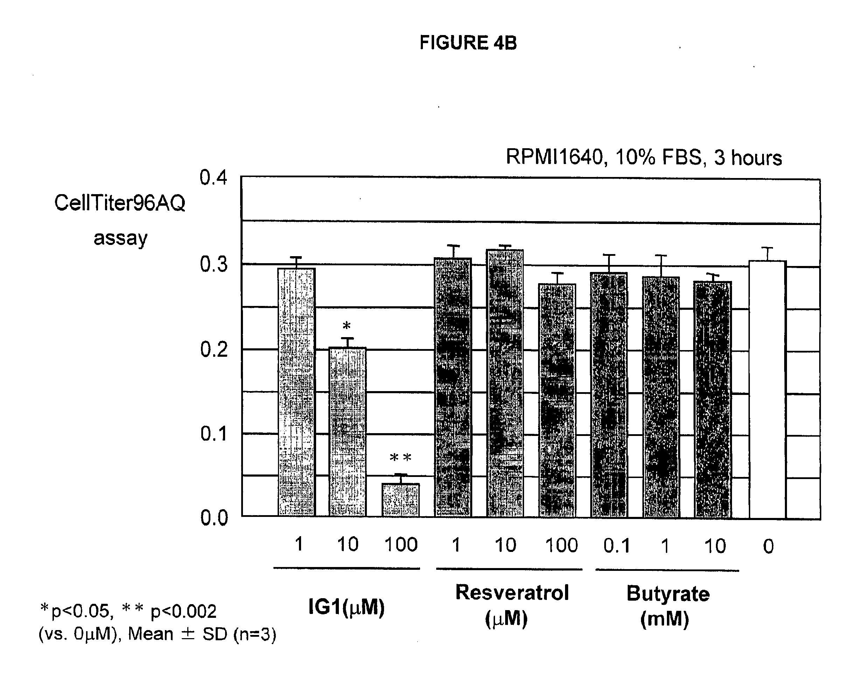

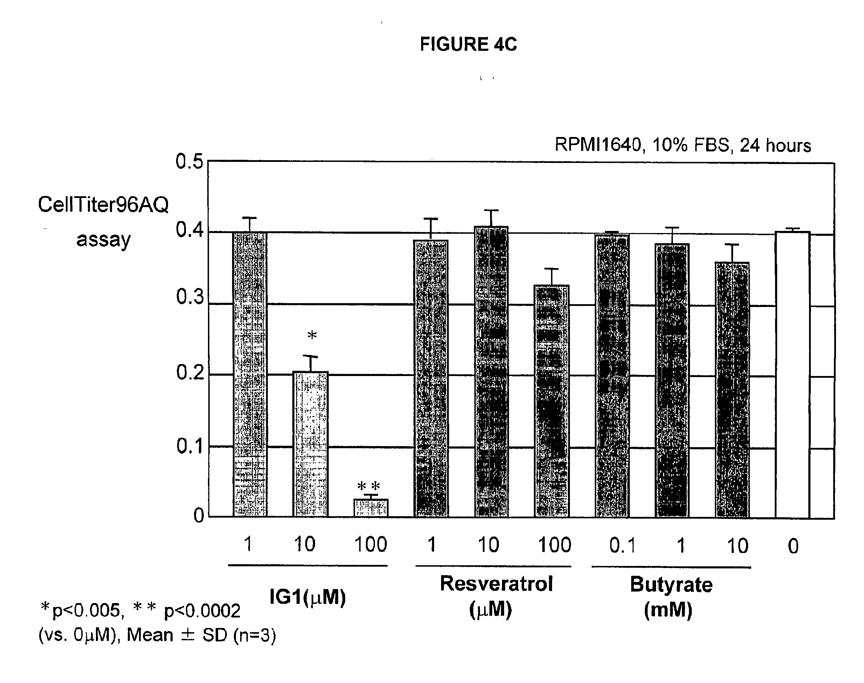

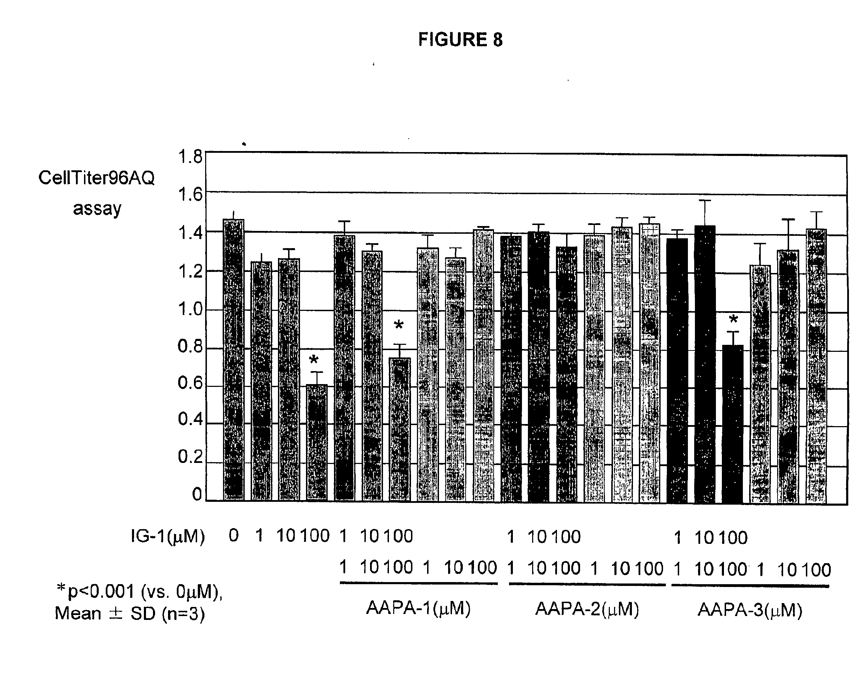

The exemplary lipophilic inositol glycan compounds used in this examples were prepared by novel chemical synthesis methods described above. The synthetic compounds are found to be cytotoxic to several human tumor cell lines, including, but not limited to, human pancreatic cancer cells (Panc-1, MiaPaca2, BXPC3, Aspc1), human hepatoma cells (HuH7), human gastric cancer cells (AGS, MKN45), human colon cancer cells (CoCM-1), human mammary cancer cells (MCF-7), human neuroblastoma (SH-SY5Y), human prostate cancer cells (PC3), and murine Ehrlich ascites cancer cells. However, these compounds are not cytotoxic to human normal cells, including, but not limited to, human dermal fibroblasts, human epidermal keratinocytes, with exception of human umbilical vein endothelial cells (Fig. 8), discussed below. The data, shown in the Figures 4-6, demonstrates that these compounds are cytotoxic by activating intrinsic cell death mechanism in cancer cells.

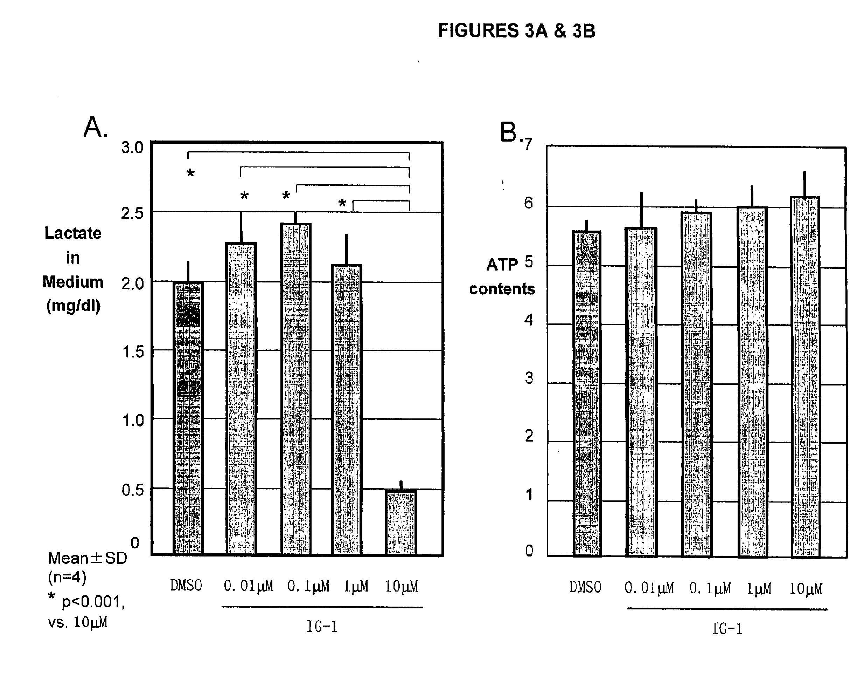

As shown in Figure 3, the lipophilic inositol glycans can normalize aerobic glycolysis without effects on ATP contents in Panc-1 cells at 10µM concentrations in a serum-free medium. (1 hr incubation). IG-1 at the concentrations of 10-100 µM, induced very rapid cell death in Panc-1, human pancreatic cancer cells in a 1 hour after IG 1 addition to the medium (Fig. 4A, 4B, and 4C). This rapid cancer cell death was demonstrated in the all tested cancer cell lines, such as human pancreatic cancer cells (MiaPaca2, BXPC3, Aspc1), human hepatoma (HuH7), human gastric cancer cells (AGS, MKN45), human colon cancer cells (CoCM-1), human mammary cancer cells (MCF-7), human neuroblastoma (SH-SYSY), human prostate cancer cells (PC3), and murine Ehrlich ascites cancer cells at the same IG1 concentrations of 10-100 µM. However, IG-1 at the concentrations of 1-100 µM did not affect the proliferation of human normal cultured cells (data not shown), with the exception of HUVEC (Fig. 8).

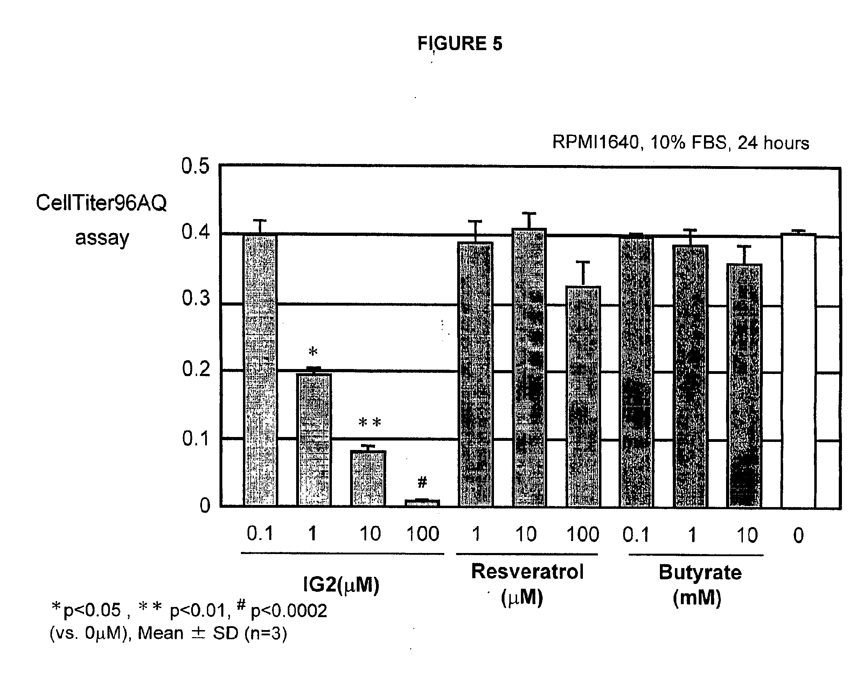

In contrast, IG-2 induced cancer cell death in human prostate cancer cells (PC3) (Fig. 5), human neuroblastoma (SH-SY5Y), and human hepatoma (HuH7) in 24 hours after IG-2 treatments. IG-2 at the concentrations of 1-100 µM also showed no effect on the proliferation of human normal cultured cells (data not shown).

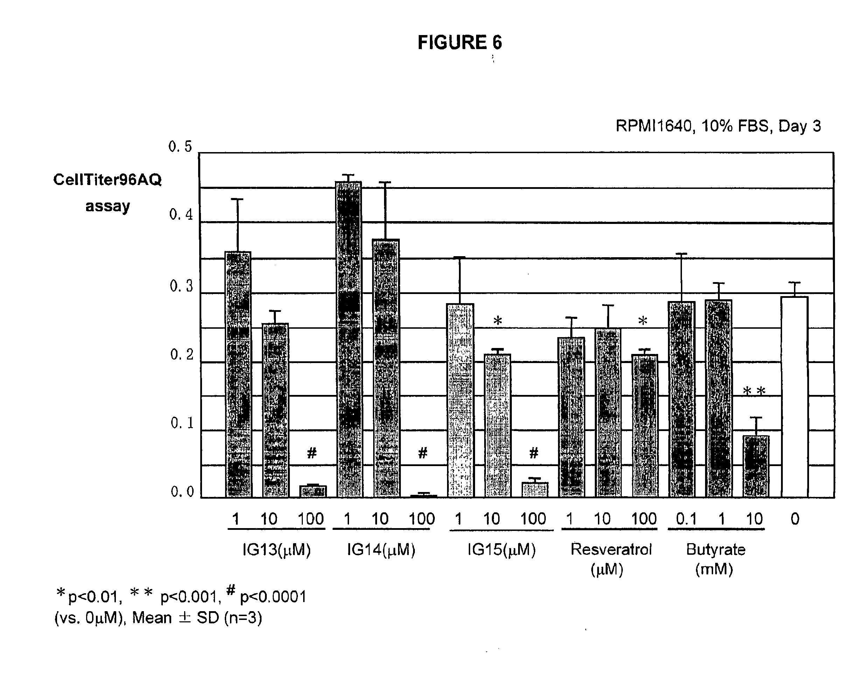

IG13, IG14, and IG15, three IG1 analogues also induced cancer cell death in human pancreatic cancer cells (Panc-1) in 72 hours after the treatment with IGs (Fig. 6). IG13, IG 14, andIG15 at the concentrations of 1-100µM also did not affect the proliferation of human normal cultured cells (data not shown).

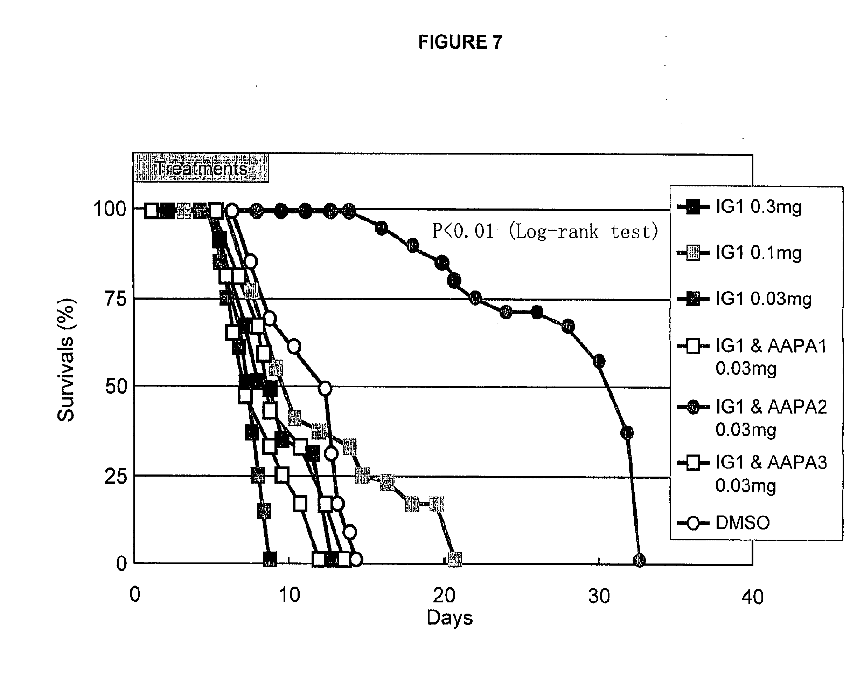

To evaluate *in vivo* anti-cancer bioactivities of IG1, we added IG-1 intraperotoneally to the ddy mice bearing bearing Ehrlich Ascites cancer cells for 7 days, and investigate the survival times of the mice. IG-1 treatment did not affect the survival times as compared with DMSO only. Alkyl, acyl-phosphatidic acid (AAPA) is a putative product from glycosylphospatidylinositol (GPI) precursor of IG1 by the hydrolysis with GPI-specific phospholipase D (GPI-PLD). Three species of AAPA, AAPA1 (1-0-alkyl-(18:0)-2-0-acyl-(18:0)-sn-glycerol-phosphate), AAPA2 (1-0-alkyl (18:0)-2-0-acyl-(20:4)-sn-glycerol-phosphate), and AAPA3 (1-0-alkyl-(18:0)-2-0-acyl (22:4)-sn-glycerol-phosphate), were prepared by chemical synthesis methods described above and the survival times of cancer-bearing mice by the treatment with IG1 plus AAPA compounds were investigated. IG1 plus AAPA2 significantly increased survival times of the cancer-bearing mice, as compared with IG1 only or combination of IG1 plus other AAPAs (Fig.7). Fig. 7 shows a Kaplan-Meier survival curve of the ddy mice bearing Ehrlich ascites cancer cells after daily administration of IG1 and alkyl-acyl-phosphatidic acid for 7 days. IG1 induces rapid cell death in of human umbilical vein endothelial cells (HUVEC) in 1 hour, however AAPA2 inhibits this rapid cell death of HUVEC by IG-1 (Fig. 8). Thus, AAPA, a putative product from GPI precursor of IG1 by the hydrolysis with GPI-PLD, can interact with lipophilic inositol glycan as a co-partner on cellular signaling.

### II. Lipophilic Inositol Glycan Compounds Have Insulin-Mimicking Bioactivities, Can Improve Glucose Tolerance, and Can Treat or Reduce Glucose-Metabolism Disorders

In another aspect, the compositions and methods of the present invention can be used to modulate blood glucose. The lipophilic inositol glycan compounds of formula (I) and (II), or salts, solvates and physiological functional derivatives thereof, can be used to modulate blood glucose. Thus, the compositions and methods of the present invention can be used to prevent, delay onset or treat glucose-metabolism disorders. The lipophilic inositol glycan compounds can be used for the therapeutic and prophylactic treatment of glucose-metabolism disorders. In one embodiment, administration of lipophilic inositol glycan compounds or functional analogs can modulate blood glucose. The lipophilic inositol glycan compounds or functional analogs can modulate the secretion of insulin leading to the modulation of blood glucose. Glucose-metabolism disorder is intended to refer to any disorder relating to glucose uptake or release, as well as, insulin expression, production, secretion, or usage. The glucose-metabolism disorder can be selected from, but not limited to, the group consisting of obesity, diabetes, insulin resistance, hyperglycemia, glucose intolerance, hyerinsulinemia, Syndrome X, hypercholesterolemia, hyperlipoproteinemia, hypertriglyceridemia, atherosclerosis, and diabetic renal disease.

The following Example demonstrates that the lipophilic inositol glycan compounds of the present invention have insulin-mimicking bioactivities and can improve glucose tolerance in art-recognized mice models with type 1, type 2 diabetes, obesity and metabolic syndrome.

### Materials and Methods:

### Glycogen synthesis and lipogenesis in 3T3-L1 adipocytes

3T3-L1 fibroblasts were maintained in Dulbecco's modified Eagle's medium (DMEM) with 25mM glucose plus 10% fetal bovine serum (FBS). Following 2 days at confluence, differentiation was initiated by the addition of DMEM containing 10% FBS, 167 nM insulin, 0.25 µ mol/liter dexamethasone, and 0.5 mM isobutylmethylxanthine. Three days later, the medium was replaced with DMEM plus 10% FBS and 167 nM insulin. After 2 more days, the medium was switched to DMEM plus 10% fetal bovine serum. Adipocytes were used 6-14 days after completion of the differentiation protocol, when >90% of the cells expressed the adipocyte phenotype. Prior to experiments, cells were washed two times with DMEM medium containing 5mM glucose, 0.2% bovine serum albumin, 25 mM Hepes (pH 7.4), 100 units/ml penicillin, 100 units/ml streptomycin and 0.29 mg/ml glutamine, and were incubated in the same medium for 2 h. Glycogen and lipid synthesis were measured in 24-well dishes. Following pretreatment with various concentrations of IGs in DMSO for 30 min, cells were stimulated for 30 min in the absence and presence of 1-100 nM insulin. Then 1 µCi of [¹⁴C]-glucose (approximately 220 cpm/nmol) was added to all wells. After a 60-min incubation at 37°C, cells were washed three times on ice with PBS, and adipocytes were collected in 1 ml of distilled water. 400 µl of the cell suspension was added to 400 µl of PBS. The lipids were then extracted overnight with 5 ml of Betafluor (National Diagnostics), and glucose incorporation into lipid was measured by scintillation counting. 400 µl of the cell suspension was added to 600 µl of 50% KOH, and glycogen was precipitated and radioactivity was counted by a liquid scintillation counter

### Measurement of lipogenesis in native rat adipocytes.

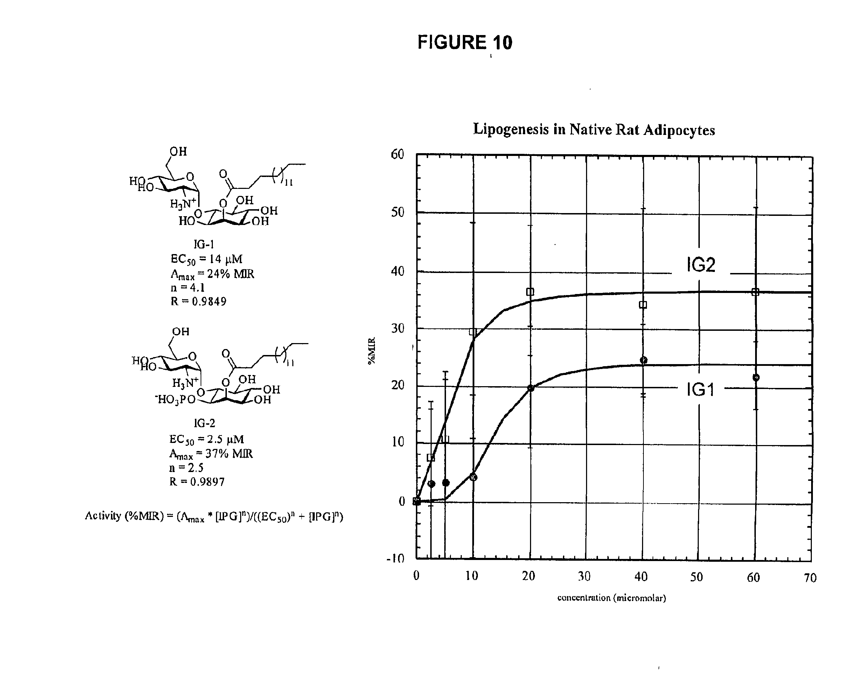

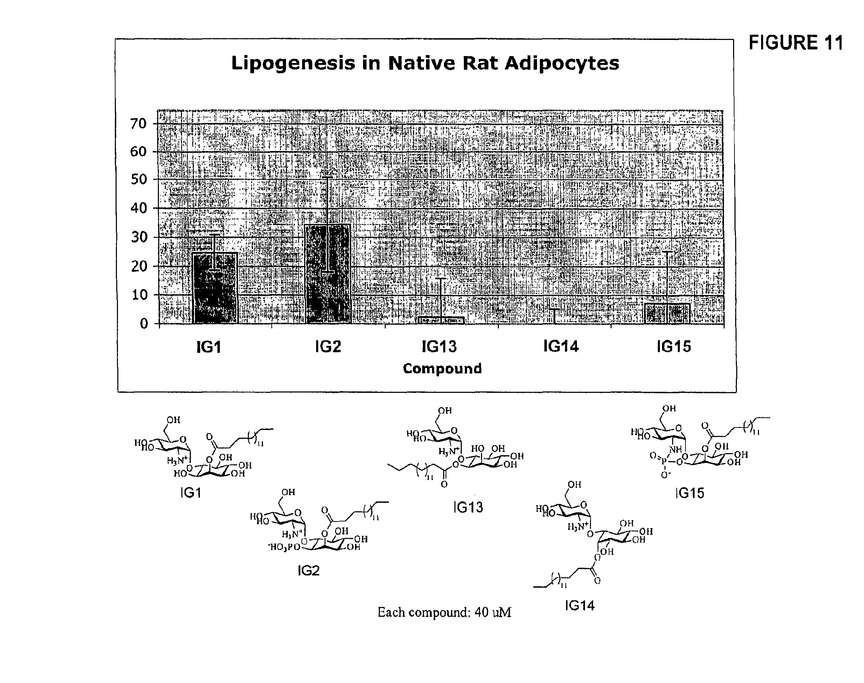

Adipocytes were isolated from epididymal fat pads dissected from freshly sacrificed male Long-Evans rats and incorporation of [6-³H]-glucose into triglycerides in response to treatment with inositol glycans or insulin was measured as previously described (Chakraborty, N. and M. d'Alarcao, Bioorganic & Medicinal Chemistry 13 (2005) 6732-6741). In this assay both IG-1 and IG-2 stimulated lipogenesis as shown in Figure 10. Fitting these data to a receptor binding model (Chakraborty, N. and M. d'Alarcao, Bioorganic & Medicinal Chemistry 13 (2005) 6732-6741) results in an EC₅₀ value for stimulation of lipogenesis of 14 µM and 2.5 µM for IG-1 and IG-2, respectively. By contrast, 40 µM IG-13, IG-14, or IG-15 stimulated little or no lipogenesis in this assay (Figure 11).

### Animal studies

Male C57BL/6 mice were housed individually and given free access to a standard diet (65% carbohydrate, 4% fat, and 24% protein) or a high-fat diet (Quick Fat; 60.2% carbohydrate, 15.3% fat, and 24.5% protein; Clea Japan, Tokyo, Japan), starting at 5 weeks of age, for 5 weeks. Db/db mice also were housed individually and given free access to a standard diet. C57BL/6 mice at 5 weeks of age, were received a single dose of 75µg/g body weight streptozotoxin (STZ). 7 days after injections, the mice, whose fasting plasma glucose concentrations were over 400mg/dl, were used as STZ-diabetic mice. Animal study protocols were in accordance with the institutional guidelines for animal experiments at Tohoku University.

### Triglyceride and glycogen contents of the liver

Frozen livers were homogenized, and triglycerides were extracted with CHCl₃:CH₃OH (2:1, vol: vol), dried and resuspended in 2-propanol (17). Triglyceride contents were measured using Lipidos liquid (TOYOBO, Osaka, Japan). Glycogen was isolated from 30-50 mg of frozen liver by dissolving the tissue in 30% potassium hydroxide saturated with Na₂SO₄ for 30 min at 100°C, followed by ethanol precipitation. Glycogen content was determined by the phenol-sulfuric acid spectrophotometric method at 490 nm (18) and expressed as micrograms of glycogen per milligram of liver.

### Blood analysis

Blood glucose was assayed with Glucose Ace (Sanwa Kagaku Kenkyusho, Nagoya, Japan). Serum insulin and leptin were determined with ELISA kits (Morinaga Institute of Biological Science, Yokohama, Japan). Serum adiponectin and tumor necrosis factor-α (TNF- α) concentrations were measured with an ELISA kit (Ohtsuka Pharmaceutical, Tokyo, Japan) and a TNF-α assay kit (Amersham Biosciences, Uppsala, Sweden), respectively. Serum total cholesterol, triglyceride, and FFA concentrations were determined with a Cholescolor liquid, Lipidos liquid (TOYOBO, Osaka, Japan), and NEFA C (Wako Pure Chemical, Osaka, Japan) kits, respectively.

### Glucose tolerance tests

Glucose tolerance was assessed with intraperitoneal glucose tolerance. Glucose tolerance tests were performed on fasted (16 h) mice. Mice were given intraperitoneal glucose (2 g/kg body wt), and blood glucose was assayed immediately before and at 15, 30, 60, and 120 min after administration.

### Intravenous administration of IG1

Intravenous administration of IG-1 was performed on mice fed ad libitum. Mice received an injection of IG1 or human regular insulin (1 units/kg body wt for normal diets; 2 units/Kg for high fat diets, db/db, and STZ-diabetic mice; Eli Lilly, Kobe, Japan) from tail veins, and blood glucose was assayed immediately before and at 30, 60, 90, 120, 180, 240 and 300 min after injection.

### Statistical Analysis

The statistical significance of differences was assessed by the unpaired Student's t -test. Ap value of < 0.05 was considered significant.

### Results of Study on Animal Models with Glucose-Metabolism Disorders

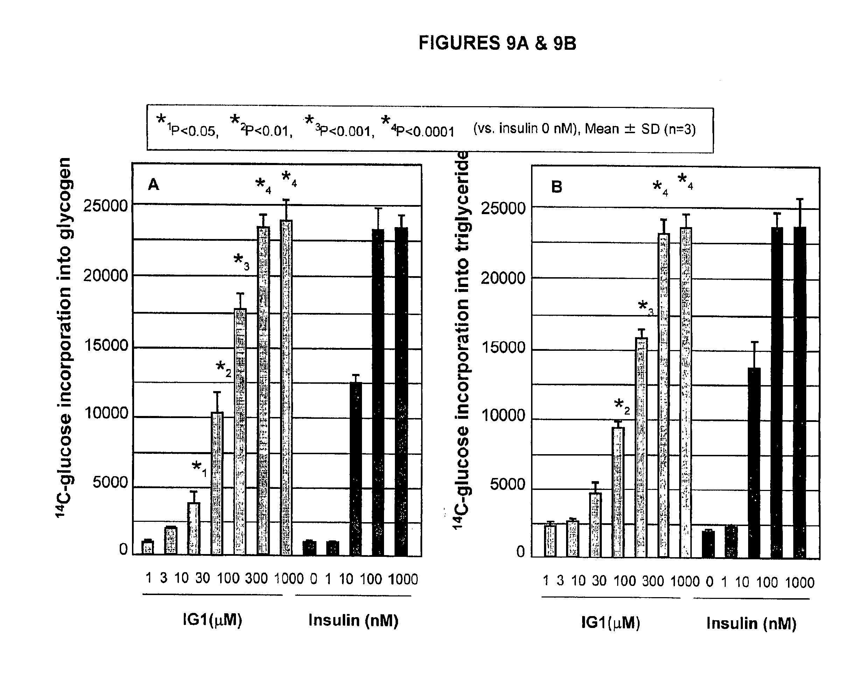

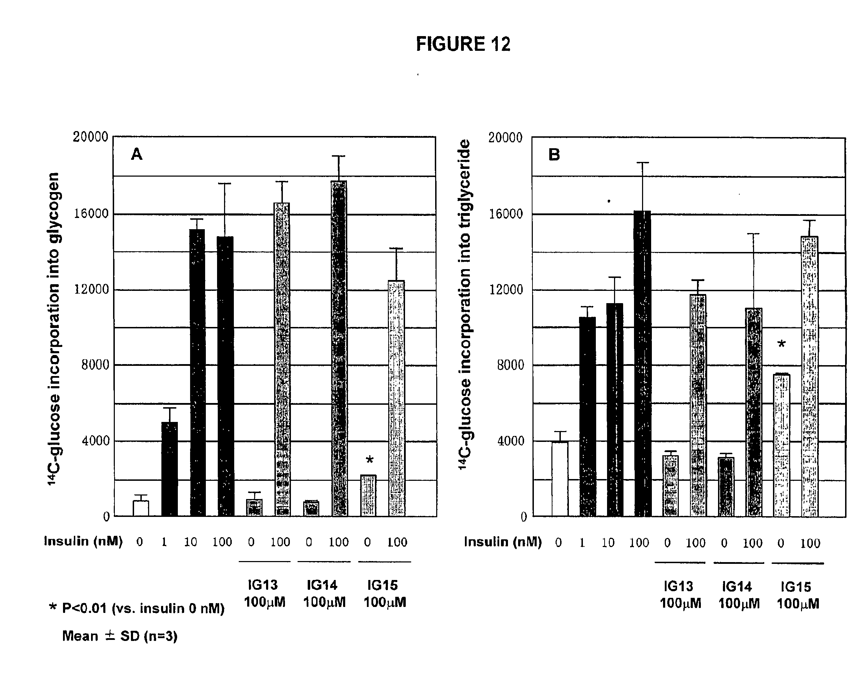

The lipophilic inositol glycan compounds of formula (I) and (II), or salts, solvates and physiological functional derivatives thereof of the present invention can stimulate glycogen synthesis and lipogenesis. As shown, IG1 at 10-1000µM concentrations stimulates glycogen synthesis and lipogenesis in 3T3-L1 adipocytes (Fig. 9A, 9B). IG1 and IG2 stimulate lipogenesis in rat isolated adipocytes (Fig.10, 11). IG15 also stimulates glycogen synthesis and lipogenesis in 3T3-L1 adipocytes at the concentration of 100µM (Fig.12A, 12B).

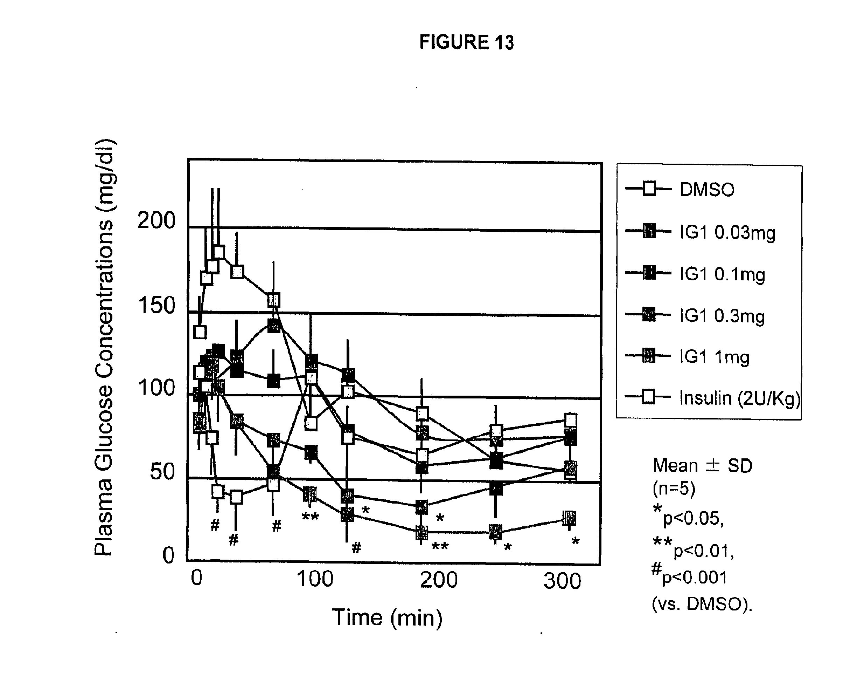

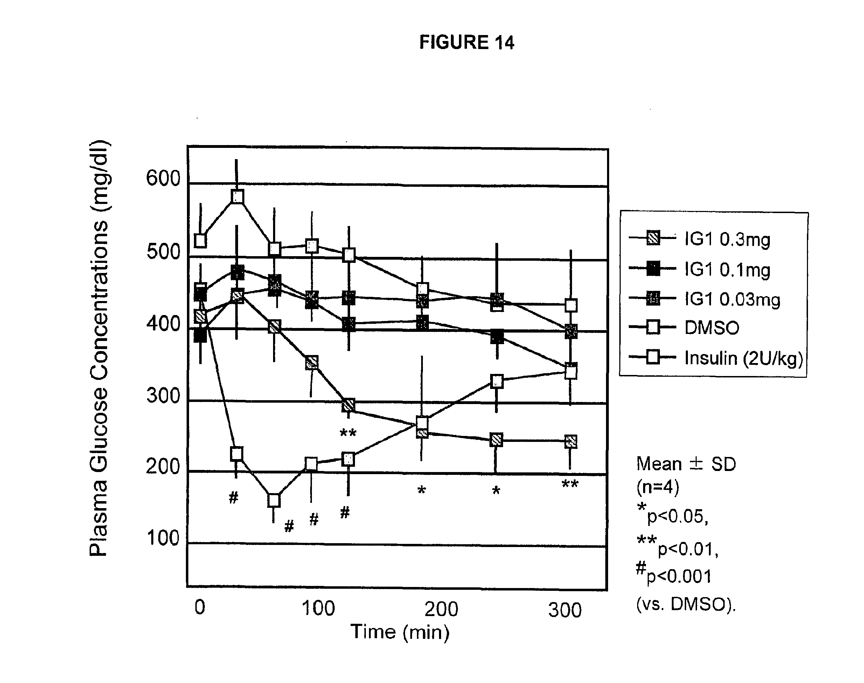

The lipophilic inositol glycan compounds of formula (I) and (II), or salts, solvates and physiological functional derivatives thereof, of the present invention can reduce plasma glucose concentrations in art recognized models of glucose metabolism disorders. Intravenous administration of IG1 (0.3-1mg per mice) significantly reduced plasma glucose concentrations in the C57B/6 mice on high-fat diets, animal model of metabolic syndrome in 90-300 min after administration (Fig. 13). IG1 significantly increases hepatic and muscular glycogen contents in the C57B/6 mice on high-fat diets (data not shown). Intravenous administration of IG1 (0.3mg per mice) significantly reduced plasma glucose concentrations in the mice with streptozotocin (STZ)-diabetic mice, animal model of insulin-deficient type 1 diabetes in 120-300 min after administration (Fig. 14). IG1 also increases hepatic and muscular glycogen synthesis in the STZ-diabetic mice (data not shown). Intravenous administration of IG I reduced plasma glucose concentrations in the C57B/6 mice on normal diets (normal mice) and db/db, obese-diabetic mice (type 2 diabetes model). IG1 increases hepatic and muscular glycogen synthesis in the C57B/6 mice on normal diets and db/db mice (data not shown).

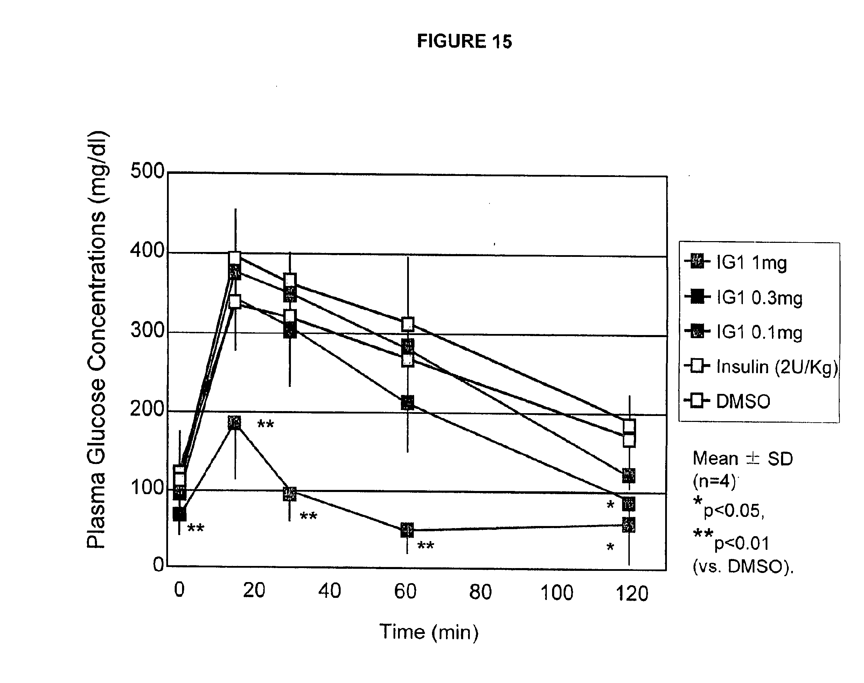

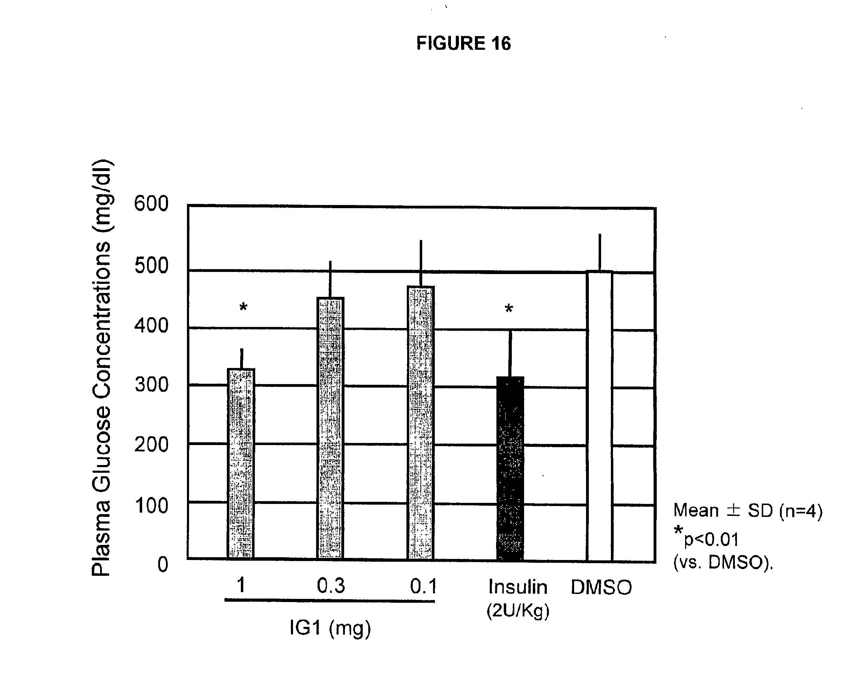

The lipophilic inositol glycan compounds of formula (I) and (II), or salts, solvates and physiological functional derivatives thereof, of the present invention can significantly improve glucose tolerance, reduce fasting plasma glucose concentrations, and increase hepatic and muscular glycogen synthesis, reduce daily food intake, reduce body weight, and reduce fasting plasma concentrations of insulin, triglyceride, free fatty acids and leptin in art recognized models of glucose metabolism disorders. Intraperitoneal administration of IG1 (1mg per mice) for every 7 days significantly improved glucose tolerance in the C57B/6 mice on high-fat diets (Fig. 15). Fig. 13 depicts time courses showing reduction of plasma glucose concentrations in the C57B/6 mice on high-fat diets after the intravenous administration of IG1. Fig. 16 shows improved fasting plasma glucose concentrations in the STZ-diabetic mice after daily intraperitoneal administration of IG1 for 7 days. After 7 days, IG-1 treatment increases hepatic and muscular glycogen contents and decreases hepatic triglyceride contents in the C57B/6 mice on high-fat diets (data not shown). Intraperitoneal administration of IG1 (0.3mg per mice) for every 7 days significantly reduced fasting plasma glucose concentrations in the STZ-diabetic mice (Fig. 14). IG-1 also increases hepatic and muscular glycogen synthesis in the STZ-diabetic mice (data not shown). Intraperitoneal administration of IG1 for every 7 days improves glucose tolerance and increases hepatic and muscular glycogen synthesis in the C57B/6 mice on normal diets and db/db mice (data not shown). Fig. 16 shows improved fasting plasma glucose concentrations in the STZ-diabetic mice after daily intraperitoneal administration of IG1 for 7 days. The treatment with IG1 for 7 days significantly reduces daily food intakes, body weight, and fasting plasma concentrations of insulin, triglyceride, free fatty acids and leptin in the C57B/6 mice on high-fat diets, animal models for metabolic syndrome (Table 2).

**Table 2. Metabolic parameters in the mice on high fat-diets after the IG1 treatment for 7 days. (mean ± SD (n=6))**

| | **DMSO** | **IG1 (1 mg)** | **p value** |
|---|---|---|---|
| Body weight (g) | 34.7 ± 1.1 | 32.1 ± 1.8 | p<0.05 |
| Food intake (g/day) | 4.04 ± 0.43 | 2.93 ± 0.37 | p<0.01 |
| Fasting plasma glucose (mg/dl) | 104.2 ± 16.1 | 62.8 ± 6.3 | p<0.01 |
| Fasting plasma insulin (ng/ml) | 1.13 ± 0.32 | 0.44 ± 0.19 | p<0.01 |
| Total cholesterol (mg/dl) | 201.3 ± 12.1 | 177.9 ± 24.6 | NS |
| Triglyceride (mg/dl) | 34.7 ± 4.1 | 26.2 ± 4.4 | p<0.05 |
| NEFA(mEq/l) | 1.12±0.2 | 0.78±0.18 | p<0.05 |
| Adiponectin (µg/ml) | 49.3 ± 3 .9 | 50.4 ± 4.8 | NS |
| TNF-1α (pg/ml) | 32.7 ± 2.9 | 33.7 ± 4.4 | NS |
| Leptin (ng/ml) | 33.7 ± 7.1 | 17.3 ± 5.4 | p<0.05 |

Thus, IG1 improves glucose tolerance, insulin sensitivity and lipid metabolism in the C57B/6 mice on normal, or high-fat diets, db/db, or STZ-diabetic mice. The treatment with IG for 28 days significantly reduces daily food intakes, body weight, and fasting plasma concentrations of insulin, triglyceride, free fatty acids and leptin in the ob/ob obese mice (see Table 3) and the C57B/6 mice on high-fat diets (data not shown).

The Examples demonstrates that the lipophilic inositol glycan compounds of formula (I) and (II), or salts, solvates and physiological functional derivatives thereof, of the present invention have insulin-mimetic bioactivities in 3T3-L1 adipocytes (Fig. 6-9). For example, IG1 has hypoglycemic bioactivities in normal mice and in mice models of type 1, type 2 diabetes, obesity and metabolic syndrome (Fig. 10-11). As shown in concentration-effect relationship curve of Fig. 10, IG-1 has an EC₅₀ (concentration of half maximum effect) of 14µM, Aₘₐₓ (maximal effect) of 24%, and IG-2 has an EC₅₀ of 2.5µM, Aₘₐₓ of 37%. Long-term treatment with lipophilic inositol glycan compounds improves glucose tolerance in normal mice and in mice models of glucose-metabolism disorders, such as type 1 and type 2 diabetes, obesity and metabolic syndrome) (Figs. 12-16). For example, as demonstrated, the treatment with IG1 for 7 days and 28 days significantly reduces daily food intakes, body weights, and fasting plasma concentrations of glucose, insulin, triglyceride and free fatty acids, and hepatic triglyceride contents in art recognized mice models of glucose-metabolism disorders (Table 2 and 3).

**Table 3. Metabolic parameters in the ob/ob obese mice after the IG-1 treatment for 28 days. (Mean ± SD (n=6))**

| | **DMSO** | **IG1 (1 mg)** | **p value** |
|---|---|---|---|
| Bodv weight (g) | 47.9 ± 4.4 | 40.1 ± 3.2 | p<0.01 |
| Food intake (g/day) | 5.32 ± 0.94 | 4.53 ± 0.74 | p<0.05 |
| Fasting plasma glucose (mg/dl) | 89.8 ± 11.1 | 66.1 ± 8.3 | n<0.01 |
| Fasting plasma insulin (ng/ml) | 27.6 ± 2.2 | 16.3 ± 4.3 | p<0.01 |
| Total cholesterol (mg/dl) | 185.6 ± 19.5 | 172.4 ± 13.6 | NS |
| Triglyceride (mg/dl) | 42.2 ± 4.1 | 31.2 ± 5.3 | p<0.05 |
| NEFA(mEq/l) | 1.45 ± 0.41 | 0.88 ± 0.34 | p<0.05 |
| Adiponectin (µg/mll | 34.3 ± 3.5 | 47.6 ± 4.6 | P<0.05 |
| TNF-1α (pg/ml) | 49.7 ± 4,3 | 34.7 ± 6.7 | NS |

## Claims

1. A myo-lipophilic inositol glycan compound for the treatment of diabetes or obesity.

2. The compound of claim 1, wherein the lipophilic myo-inositol glycan compound is selected from the compounds of formula:
A-O₁-B,
wherein A is a hexosamine selected from the group consisting of glucosamine and galactosamine;
O₁ (an oxygen linkage) is selected from the group consisting of alpha 1,6, alpha 1,3,
and alpha 1,2; and
B is myo-inositol, wherein the B moiety is optionally mono- or di-substituted, independently of one another, by phosphate, thiophosphate, alkyl group (C₁₋₂₂ or acyl residues (O=C-C₀₋₂₁).

3. The compound of claim 1, wherein the lipophilic myo-inositol glycan compound is selected from the compounds of formula II: 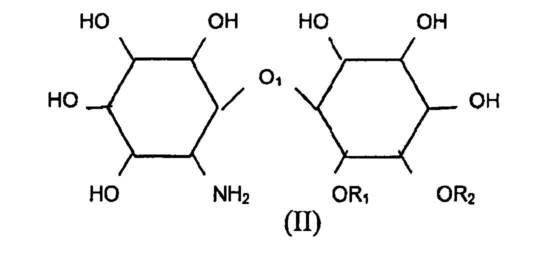 wherein O₁ is selected from the group consisting of alpha 1,6 and alpha 1,3 linkage;
R₁ is selected from the group consisting of phosphate, alkyl group (C₁₋₂₂) and acyl residues (O=C-C₀₋₂₁); and
R₂ is selected from the group consisting of alkyl group (C₁₋₂₂) and acyl residues (O=C-C₀₋₂₁).

4. The compound of claim 3, wherein at least one of R₁ or R₂ is palmitoyl.

5. The compound of claim 3 or 4, wherein R₁ is phosphate.

6. The compound of claim 1, wherein the compound is selected from the group consisting of
2-amino-2-deoxy-α-D-glucopyranosyl-(1->6)-2-palmitoyl-D-*myo*-inositol (**13**),
2-amino-2-deoxy-α-D-glucopyranosyl-(1->6)-2-palmitoyl-D-*myo*-inositol-1-phosphate (**14**), 2-amino-2-deoxy-α-D-glucopyranosyl-(1->6)-1-palmitoyl-D-*myo*-inositol (**91**), 2-amino-2-deoxy-α-D-glucopyranosyl-(1->3)-2-palmitoyl-D-*myo*-inositol (**92**), and 2-amino-2-deoxy-α-D-glucopyranosyl-(1->3)-1-palmitoyl-D-*myo*-inositol (**93**).

7. A substantially purified lipophilic myo-inositol glycan compound of formula:
A-O₁-B,
wherein A is a hexosamine selected from the group consisting of glucosamine and galactosamine;
O₁ is selected from the group consisting of alpha 1,6, alpha 1,3, and alpha 1,2; and
B is myo-inositol, wherein the B moiety is optionally mono- or di-substituted, independently of one another, by phosphate, thiophosphate, alkyl group (C₁-₂₂) or acyl residues (O=C-C₀₋₂₁).

8. The substantially purified lipophilic myo-inositol glycan compound of claim 7, wherein the compound is selected from the group consisting of 2-amino-2-deoxy-α-D-glucopyranosyl-(1->6)-2-palmitoyl-D-*myo*-inositol (**13**), 2-amino-2-deoxy-α-D-glucopyranosyl-(1->6)-2-palmitoyl-D-*myo*-inositol-1-phosphate (**14**), 2-amino-2-deoxy-α-D-glucopyranosyl-(1->6)-1-palmitoyl-D-*myo*-inositol (**91**), 2-amino- 2-deoxy-α-D-glucopyranosyl-(1->3)-2-palmitoyl-D-*myo*-inositol (**92**), and 2-amino-2-deoxy-α-D-glucopyranosyl-(1->3)-1-pahnitoyl-D-*myo*-inositol (**93**).

9. A therapeutic composition comprising a compound of claim 7 or 8, wherein said compound is configured for administration to a subject having or suspected of having diabetes or obesity.

10. A pharmaceutical composition, comprising: a therapeutically effective amount of one or more myo-inositol glycan compounds of formula: 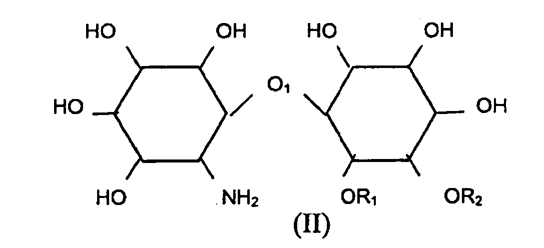 wherein O₁ is selected from the group consisting of alpha 1,6 and alpha 1,3 linkage;
R₁ is selected from the group consisting of phosphate, alkyl group (C₁₋₂₂) and acyl residues (O=C-C₀₋₂₁); and
R₂ is selected from the group consisting of alkyl group (C₁₋₂₂) and acyl residues (O=C-C₀₋₂₁); or a salt thereof and one or more of pharmaceutically acceptable carriers, diluents and excipients.

11. The pharmaceutical composition of claim 10, further comprising at least one compound of formula: 1-Alkyl(C₁₋₂₂)-2-Acyl(O=C-C₀₋₂₁)-sn-glycerol-Phosphate.

12. The pharmaceutical composition of claim 10, further comprising at least one additional agent that is an anti-neoplastic agent.

13. The pharmaceutical composition of claim 10, which is used for the treatment of diseases associated with abnormal glucose metabolism such as diabetes and obesity.
